# EUROPEAN PATENT APPLICATION

(11) **EP 4 534 652 A1**
(43) Date of publication of application: **09.04.2025**
(21) Application number: 23811155.3
(22) Date of filing: 25.05.2023
(51) Int. Cl.: C12N 5/0783, C12N 13/00, C07K 16/00, C07K 16/28, A61K 39/00, A61P 35/00

(54) **SPECIFIC T CELL FOR PREVENTING OR TREATING CANCER, AND PREPARATION METHOD THEREFOR**

(30) Priority: 26.05.2022 CN 202210586911
(71) Applicant: Suzhou Ersheng Biopharmaceutical Co., Ltd., Suzhou, Jiangsu 215125 (CN)
(72) Inventor: LIU, Mi, Suzhou, Jiangsu 215125 (CN)
(74) Representative: Winter, Brandl - Partnerschaft mbB
(86) International application number: PCT/CN2023/096419
(87) International publication number: WO 2023/227085

(57) **Abstract**

The present disclosure relates to a method for preparing a cancer-specific T cell based on peripheral blood or a peripheral immune organ for preventing or treating cancer, specifically including steps of: first, isolating an immune cell from peripheral blood or the peripheral immune organ; then, co-incubating with a nanoparticle and/or a microparticle loaded with a tumor whole-cell antigen for a period of time to activate the cancer-specific T cell; then, isolating a cancer-specific T cell activated by the tumor antigen; and, reinfusing the cancer-specific T cell into the body to exert an anti-cancer effect after *in vitro* expansion. According to the nanoparticle or microparticle prepared by the present disclosure, a tumor antigen component is loaded onto the microparticle and/or the nanoparticle to activate the cancer-specific T cell, and then the cancer-specific T cell is expanded and reinfused into a patient for treating cancer or preventing recurrence or metastasis. The cancer-specific T cell isolated by a sorting method has high specificity, and may prevent or treat cancer by killing cancer cells after expansion.

## Description

### TECHNICAL FIELD

The present disclosure relates to the technical field of immunotherapy, and in particular to a specific T cell for preventing or treating cancer, a preparation method thereof, and an application thereof.

### BACKGROUND

Immune cells refer to cells involved in or related to immune responses, including congenital lymphocytes, various phagocytes, and lymphocytes that can recognize antigens and produce specific immune responses, such as T cells, B cells, NK cells, DC cells, macrophages, granulocytes, mast cells, etc. Autologous immune cell therapeutic agents are used to fight tumors by isolating and culturing immune cells from one's own blood. By supplementing highly active immune cells, the number of immune cells in the body is increased, and the original immune cells in the body are activated at the same time, thus greatly improving the ability of immune cells to kill tumor cells, bacteria, viruses, etc., and achieving the purpose of preventing cancer and fighting cancer.

In the prior application CN202011027741.0 filed by the invention team of the present disclosure, a detection method is disclosed that can detect T cells activated with nanoparticles loaded with patient tumor components. However, this application is a detection method rather than a treatment method. It does not sort or expand the activated T cells, nor does it involve a reinfusion step. In the detection process, T cells need to be fixed and stained, and corresponding active T cells cannot be provided. Therefore, it has not been reported in the prior art that nanoparticles loading tumor antigens are used to activate and enrich specific types of T cells to exert tumor therapeutic effects.

Due to the small number and low activity of cancer-specific T cells in cancer patients, especially those with poor physical function, the cancer-specific T cells have problems that affect anti-tumor immunotherapy, such as difficulty in activation, slow expansion, long culture cycle, and low cell viability. In the present disclosure, the cancer-specific T cells with the function of recognizing cancer cells and killing cancer cells are further stimulated by *in vitro* tumor antigen, then sorted, isolated and expanded on a large scale and then reinfused into patients for use, thus realizing an effective method for preventing cancer occurrence, preventing cancer metastasis and treating cancer by reinfusing the cancer-specific T cells.

Technical content such as nanoparticle activation of tumor-specific T cells disclosed in Chinese Patent Application (202011027741.0) is an essential component of the present disclosure.

### SUMMARY

A purpose of the present disclosure is to provide a method for preparing a cancer-specific T cell derived from an autologous or allogeneic source for preventing or treating cancer, specifically including steps of: first, isolating an immune cell from peripheral blood or a peripheral immune organ; then, co-incubating with antigen-presenting cell (APC) and a nanoparticle and/or a microparticle loaded with a tumor whole-cell component or containing a partial antigen component of whole-cell component for a period of time to activate a cancer-specific T cell; then, isolating the cancer-specific T cell activated by a tumor antigen; and, reinfusing the cancer-specific T cell into the body to exert an anti-cancer effect after *in vitro* expansion.

In a preferred technical solution of the present disclosure, the preparation method specifically includes steps of:
(1) first, isolating an immune cell from peripheral blood or a peripheral immune organ, or sorting a T cell from the immune cell;
(2) co-incubating a microparticle and/or a nanoparticle loaded with a tumor antigen component with an antigen-presenting cell and the T cell after mixing, and sorting out a T cell activated and expressing a specific cell marker; and
(3) co-incubating the T cell expressing the specific cell marker sorted in the step (2) with a cytokine and/or an antibody to obtain an expanded specific T cell;
and preferably, in the step (2), the tumor antigen component may be a whole-cell lysate component of tumor tissue/a cancer cell, or the antigen component may be a portion of a whole-cell lysate component of tumor tissue/a cancer cell.

The whole-cell lysate component includes a water-soluble component and a water-insoluble component, and the water-insoluble component is dissolved (or solubilized) using a dissolving solution (or solubilizing solution) containing a dissolving agent (or solubilizing agent).

Preferably, in the step (2), the tumor antigen component is obtained by whole-cell lysis of one or more cancer cells and/or tumor tissue, or is obtained by treatment after whole-cell lysis of one or more cancer cells and/or tumor tissue, or is obtained by lysis after whole-cell treatment of one or more cancer cells and/or tumor tissue, and preferably, at least one of the cancer cells or tumor tissue is the same as a target disease type; or the antigen component consists of a portion of a component in one or more cancer cells and/or tumor tissue, and the portion of the component contains a protein/peptide component and/or an mRNA component in a lysate; and
in a preferred technical solution of the present disclosure, when the whole-cell antigen component is a portion of a whole-cell lysate component, the antigen component contains protein and peptide components and/or an mRNA component in the whole-cell lysate component.

Preferably, the nanoparticle and/or the microparticle particle loaded with the tumor antigen component may be co-incubated together with the antigen-presenting cell and the T cell to activate the cancer-specific T cell; or the nanoparticle and/or the microparticle loaded with the tumor antigen component may be first co-incubated with the antigen-presenting cell to activate the antigen-presenting cell, and then the activated antigen-presenting cell is alone co-incubated with the T cell to activate the cancer-specific T cell; and after the nanoparticle and/or the microparticle loaded with the tumor antigen component is first co-incubated with the antigen-presenting cell to activate the antigen-presenting cell, the antigen-presenting cell may be co-incubated with the T cell to activate the specific T cell without special treatment, or the antigen-presenting cell may be treated with fixation, radiation, irradiation, modification, inactivation, mineralization, etc., and then co-incubated with the T cell to activate the specific T cell.

In a preferred technical solution of the present disclosure, in the step (1), the autologous or allogeneic source with the immune cell isolated from peripheral blood or a peripheral immune system may not be treated, or may be treated with radiotherapy, immunotherapy, chemotherapy, particle therapy, and vaccine therapy when the above cell is isolated and extracted.

In a preferred technical solution of the present disclosure, the T cell sorted in the step (1) is any one of a CD3⁺ T cell, a CD3⁺ CD8⁺ T cell and a CD3⁺ CD4⁺ T cell, or a combination thereof.

In a preferred technical solution of the present disclosure, a method of the sorting in the step (1) and the step (2) is any one of flow cytometry and a magnetic bead method, or a combination thereof. During sorting of the activated cancer-specific T cell, one activation marker for T cell activation may be used, or a combination of more than one different marker may be used as an activation marker.

In a preferred technical solution of the present disclosure, in the step (2), the tumor antigen component is obtained by whole-cell lysis of one or more cancer cells and/or tumor tissue, or is obtained by treatment after whole-cell lysis of one or more cancer cells and/or tumor tissue, or is obtained by lysis after whole-cell treatment of one or more cancer cells and/or tumor tissue, and preferably, at least one of the cancer cells or tumor tissue is the same as a target disease type; or the antigen component consists of a portion of a component in one or more cancer cells and/or tumor tissue, and the portion of the component contains a protein/peptide component and/or an mRNA component in a lysate.

In a preferred technical solution of the present disclosure, the antigen component may be: (1) a whole-cell lysate component of a cancer cell/tumor tissue; (2) or a whole-cell component consisting of a group of proteins and peptides in a whole-cell lysate component of a cancer cell/tumor tissue; (3) or protein and peptide components plus an mRNA component in a whole-cell component of a cancer cell/tumor tissue.

In a preferred technical solution of the present disclosure, the tumor antigen component is a cell lysis component of tumor tissue and/or a cancer cell, including one or both of a water-soluble component and a water-insoluble component generated after cell lysis of the tumor tissue and/or the cancer cell, and the water-soluble component and the water-insoluble component are collected separately and the nanoparticle or the microparticle is prepared separately; or the cancer cell or the tumor tissue may be lysed directly and the whole-cell component may be solubilized by directly utilizing the solubilizing solution containing the solubilizing agent, and the nanoparticle or the microparticle is prepared, and the water-insoluble component is solubilized by the solubilizing (dissolving) solution containing the solubilizing (dissolving) agent. Alternatively, the tumor antigen component may be protein and peptide components obtained after appropriate treatment of the above lysate component, or protein and peptide components plus an mRNA component.

In a preferred technical solution of the present disclosure, when the antigen component is a whole-cell lysate component, a preparation method thereof is: (1) first, lysing the cancer cell/the tumor tissue; then, preparing a water-soluble component and a water-insoluble component separately; and then, solubilizing the water-insoluble component using a specific solubilizing agent containing the solubilizing solution for use; or (2) lysing the cell using the solubilizing solution containing the solubilizing agent, and then solubilizing a lysed whole-cell component using the solubilizing solution containing the solubilizing agent.

In a preferred technical solution of the present disclosure, the antigen component is a portion of the whole-cell lysate component (containing protein and peptide components in the whole-cell component of the cancer cell), a preparation method thereof is: (1) first, lysing the cancer cell/the tumor tissue; then, preparing the water-soluble component and the water-insoluble component separately; then, solubilizing the water-insoluble component using the specific solubilizing agent containing the solubilizing solution for use; then, isolating and extracting the protein and peptide components in the water-soluble component from the water-soluble component using an appropriate method; and then, using the protein and peptide components isolated and extracted from the water-soluble component together with all the water-insoluble component as the antigen component; (2) first, lysing the cancer cell/the tumor tissue; then, preparing the water-soluble component and the water-insoluble component separately; then, solubilizing the water-insoluble component using the specific solubilizing agent containing the solubilizing solution for use; then, isolating and extracting the protein and peptide components in the water-soluble component from the water-insoluble component using an appropriate method; and then, using the protein and peptide components isolated and extracted from the water-insoluble component together with all the water-soluble component as the antigen component; (3) first, lysing the cancer cell/the tumor tissue; then, preparing the water-soluble component and the water-insoluble component separately; then, solubilizing the water-insoluble component using the specific solubilizing agent containing the solubilizing solution; then, isolating and extracting the protein and peptide components in the water-soluble component from the water-soluble component and the water-insoluble component using an appropriate method, respectively; and then, using the protein and peptide components isolated and extracted from the water-soluble component and the water-insoluble component together as the antigen component; or (4) lysing the cell using a solubilizing solution containing a solubilizing agent; then, solubilizing a lysed whole-cell component using the solubilizing solution containing the solubilizing agent; and then, isolating and extracting protein and peptide components therein using an appropriate method. In the above preparation method, a step of isolating and extracting whole-cell mRNA may be added, and the whole-cell mRNA may be used as a portion of the antigen component.

The appropriate method for isolating and extracting the above protein and peptide components includes but is not limited to salting-out, heating, enzymatic hydrolysis, etc.

The protein and peptide components are isolated and extracted, and then re-dissolved in the solubilizing solution containing the solubilizing agent.

In a preferred technical solution of the present disclosure, a concentration of the nanoparticle and/or the microparticle is 2.5 ng/mL to 50 mg/mL when the nanoparticle and/or the microparticle is co-incubated with the antigen-presenting cell alone or when the nanoparticle and/or the microparticle is co-incubated with the antigen-presenting cell and the T cell simultaneously; and time of the co-incubation is 1 hour to 168 hours.

In a preferred technical solution of the present disclosure, the whole-cell component may be treated by inactivation or (and) denaturation, solidification, biomineralization, ionization, chemical modification, nuclease treatment, etc. before or (and) after lysis before preparing the nanoparticle or the microparticle; or the nanoparticle or the microparticle may also be prepared directly without any inactivation or (and) denaturation, solidification, biomineralization, ionization, chemical modification, and nuclease treatment before or (and) after the cell lysis.

In a preferred technical solution of the present disclosure, the tumor tissue cell is treated by inactivation or (and) denaturation before the lysis, the tumor tissue cell may also be treated by inactivation or (and) denaturation after the cell lysis, or the tumor tissue cell may also be treated by inactivation or (and) denaturation both before and after the cell lysis.

In a preferred technical solution of the present disclosure, a method of the inactivation or (and) denaturation treatment before or (and) after the cell lysis includes any one of ultraviolet irradiation, high-temperature heating, radiation irradiation, high pressure, curing, biomineralization, ionization, chemical modification, nuclease treatment, collagenase treatment, and freeze-drying, or a combination thereof.

In a preferred technical solution of the present disclosure, in the step (2), a number ratio of the antigen-presenting cell to the T cell used is greater than 1:1; and a pre-existing cancer-specific T cell in a peripheral immune cell is activated *in vitro* after presentation by the antigen-presenting cell using the nanoparticle or the microparticle, and the nanoparticle or the microparticle is selected from a nanoparticle having a particle size of 1 nm to 1000 nm or a microparticle having a particle size of 1 µm to 1000 µm.

In a preferred technical solution of the present disclosure, the antigen-presenting cell co-incubated with the T cell and the nanoparticle and/or the microparticle is derived from an autologous source, an allogeneic source, a cell line, a stem cell, or any mixture thereof; and the co-incubated antigen-presenting cell is a B cell, a dendritic cell, a macrophage, or any mixture of the three.

In a preferred technical solution of the present disclosure, the antigen-presenting cell is derived from an autologous antigen-presenting cell, an allogeneic antigen-presenting cell, an antigen-presenting cell line, or an antigen-presenting cell differentiated from the stem cell, preferably, any one of the dendritic cell (DC), the B cell and the macrophage, or a combination thereof; and more preferably, a combination of more than one antigen-presenting cell is used.

In a preferred technical solution of the present disclosure, the mixing and co-incubating is selected from any one of three ways of: (a) mixing and co-incubating the three directly for a certain period of time; (b) co-incubating the microparticle and/or the nanoparticle with the antigen-presenting cell for a period of time, and then adding the T cell for co-incubating; and (c) first co-incubating the microparticle and/or the nanoparticle with the antigen-presenting cell for a period of time, and sorting an incubated antigen-presenting cell and then co-incubating both the antigen-presenting cell and the T cell.

Before the T cell is co-incubated with the nanoparticle and/or the microparticle and the antigen-presenting cell, the T cell may be cultured separately at rest for a period of time or appropriately sorted; or before the T cell is co-incubated with the activated antigen-presenting cell, the T cell may be cultured separately at rest for a period of time or appropriately sorted.

In a preferred technical solution of the present disclosure, a culture condition of the mixing and co-incubating is co-incubating for 1 h to 168 h under conditions of 30-38°C and 1-10% CO₂.

In a preferred technical solution of the present disclosure, a cytokine may be added in the mixing and co-incubating; and the cytokine added includes but is not limited to an interleukin, a tumor necrosis factor, an interferon, and a growth factor; and preferably, the cytokine added includes interleukin 7 (IL-7) and interleukin 15 (IL-15).

In a preferred technical solution of the present disclosure, in the step (2), the method of the sorting is isolating a cell expressing the specific cell marker from a cell population by flow cytometry or the magnetic bead method, etc. after binding an antibody with fluorescence or magnetism or a specific ligand to the specific cell marker on a surface of the T cell.

In a preferred technical solution of the present disclosure, in the step (2), the sorted T cell expressing the specific cell marker includes but is not limited to any one of CD69, CD137, CD25, CD134, CD80, CD86, OX40L, OX40, CD28, FAS-L, IL-2R, HLA- DR, CD127 (IL-7R), CD150, CD107A, CD83, CD166, CD39, CD178, CD212, CD229, CD100, CD107b, CD108, CD109, CD113, CD122, CD126, CD253, CD197, PD-1, TIM3, LAG-3, TIGIT, CD62L, CD70, CTLA-4 (CD152), CD27, CD26, CD30, TNFRSF9, CD74, PD-L1 (CD274), CD258, CD261, 4-1BB, CD154, ICAM-1, LFA-1, LFA-2, VLA-4, CD160, CD71, CXCR3, TNFRSF14, TNFRSF18, TNFSF4, TNFSF9, TNFSF14, CD11a, CD101, CD48, CD244, CD49a, CD95, CD44, CXCR 1, CD103, CD45RO, ICOS (CD278), VTCN1, HHLA2, LGAL59, CCR7, CD357, BCL6, TCF-1, CD38, CD27, etc., or a combination thereof.

In a preferred technical solution of the present disclosure, in the step (3), a concentration of the cytokine is 1 ng/mL to 6000 ng/mL, preferably 5 ng/mL to 200 ng/mL, and more preferably 10 ng/mL to 30 ng/mL.

In a preferred technical solution of the present disclosure, in the step (3), the cytokine includes but is not limited to an interleukin, an interferon and a tumor necrosis factor.

In a preferred technical solution of the present disclosure, the interleukin includes but is not limited to interleukin 2 (IL-2), interleukin 7 (IL-7), interleukin 12 (IL-12), interleukin 15 (IL-15), interleukin 17 (IL-17), and interleukin 21 (IL-21).

In a preferred technical solution of the present disclosure, a concentration of the antibody is 1 ng/mL to 6000 ng/mL, preferably 5 ng/mL to 100 ng/mL, and more preferably 10 ng/mL to 30 ng/mL.

In a preferred technical solution of the present disclosure, the antibody includes but is not limited to any one of an αCD3 antibody, an αCD28 antibody, an αCD80 antibody, an αCD86 antibody, and an αOX40 antibody, or a combination thereof.

In a preferred technical solution of the present disclosure, time of the co-incubation of the nanoparticle and/or the microparticle with a mixture of the antigen-presenting cell and the T cell is at least 4 hour, and preferably 6 hours to 96 hours.

In a preferred technical solution of the present disclosure, time of the co-incubation of the nanoparticle and/or the microparticle with the antigen-presenting cell alone is at least 1 hour, and preferably 6 hours to 96 hours.

In a preferred technical solution of the present disclosure, time of the co-incubation of a mixture of the activated antigen-presenting cell and the T cell is at least 1 hour, and preferably 6 hours to 96 hours.

In a preferred technical solution of the present disclosure, time of the expansion culture is at least 1 day, and preferably 4 days to 36 days.

In a preferred technical solution of the present disclosure, when the nanoparticle and/or the microparticle is co-incubated with the antigen-presenting cell alone, a concentration of the nanoparticle and/or the microparticle is 2.5 ng/mL to 50 mg/mL.

In a preferred technical solution of the present disclosure, when the nanoparticle and/or the microparticle is co-incubated with the antigen-presenting cell and the T cell, a concentration of the nanoparticle and/or the microparticle is 2.5 ng/mL to 50 mg/mL.

In a preferred technical solution of the present disclosure, a content of the protein and peptide components in the antigen component loaded by the nanoparticle and/or the microparticle is higher than 10 ng/mL. In a preferred technical solution of the present disclosure, the specific T cell obtained after *in vitro* expansion obtained in the step (3) is reinfused into the body to exert an anticancer effect.

In a preferred technical solution of the present disclosure, the tumor antigen loaded by the nanoparticle and/or the microparticle is the whole-cell component of the tumor tissue and/or the cancer cell containing the water-soluble component and/or the water-insoluble component of the tumor tissue and/or the cancer cell.

In a preferred technical solution of the present disclosure, the nanoparticle and/or the microparticle for activating the cancer-specific T cell is loaded with a component of one and/or more tumor tissue and/or cancer cells in such a way that the water-soluble component and the water-insoluble component of a whole cell are separately or together encapsulated in an interior of the particle and/or separately or together loaded onto a surface of the particle.

In a preferred technical solution of the present disclosure, an original water-insoluble portion of the whole-cell component derived from the tumor tissue or the cancer cell loaded by the nanoparticle and/or the microparticle for activating the cancer-specific T cell is changed from insoluble in pure water to soluble in an aqueous solution containing a solubilizing agent/a dissolving agent or in an organic solvent by an appropriate solubilizing method; and the solubilizing agent/dissolving agent adopted is selected from one or more of a compound with a structure of structural formula 1, a deoxycholate, a lauryl sulfate, glycerol, a protein-degrading enzyme, albumin, lecithin, a peptide, an amino acid, a glycoside, and a choline,
where structural formula 1 is as follows: the structure of structural formula 1 is as follows: 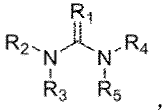
R₁ is C, N, S or O, and R₂-R₅ are independently selected from at least one of hydrogen, alkyl, amino, carboxyl, and substituted or unsubstituted guanidino.

The compound with the structural formula 1 includes but is not limited to metformin hydrochloride, metformin sulfate, metformin sulfonate, a metformin salt, metformin, polyhexamethyleneguanidine hydrochloride, agmatine sulfate, methylguanidine hydrochloride, tetramethylguanidine hydrochloride, urea, guanidine hydrochloride, guanidine sulfate, guanidine sulfonate, a guanidine salt, other compounds containing guanidino or urea, guanidine carbonate, arginine, guanidinoacetic acid, guanidinophosphoric acid, guanidine sulfamate, guanidinosuccinic acid, semicarbazide hydrochloride, carbamoylurea, acetylurea, a sulfonylurea compound (glibenclamide, gliclazide, gliquidone, glimepiride, etc.), a thiourea compound (thiouracil, imidazole, etc.), nitrosoureas, and other compounds with the structure of structural formula 1.

In a preferred technical solution of the present disclosure, the surface of the nanoparticle and/or the microparticle for activating the cancer-specific T cell is linked with a target head actively targeting the antigen-presenting cell.

In a preferred technical solution of the present disclosure, the way in which the water-soluble component and/or the water-insoluble component is loaded onto the surface of the cancer vaccine includes at least one of adsorption, covalent linkage, charge interaction, hydrophobic interaction, one-step or multi-step solidification, mineralization, and encapsulation.

In a preferred technical solution of the present disclosure, the nanoparticle has a particle size of 1 nm to 1000 nm; and the microparticle has a particle size of 1 µm to 1000 µm.

In a preferred technical solution of the present disclosure, the surface of the nanoparticle or the microparticle is electrically neutral, negatively charged or positively charged.

In a preferred technical solution of the present disclosure, a preparation material of the nanovaccine and/or the microvaccine is an organic synthetic polymer material, a natural polymer material or an inorganic material.

In a preferred technical solution of the present disclosure, the organic synthetic polymer material is PLGA, PLA, PGA, PEG, PCL, Poloxamer, PVA, PVP, PEI, PTMC, polyanhydride, PDON, PPDO, PMMA, a poly(amino acid), or a synthetic peptide; the natural polymer material is lecithin, cholesterol, sodium alginate, albumin, collagen, gelatin, a cell membrane component, starch, saccharides, or a polypeptide; and the inorganic material is ferric oxide, ferroferric oxide, calcium carbonate, or calcium phosphate.

In a preferred technical solution of the present disclosure, the component of one and/or more tumor tissue and/or cancer cells may be co-loaded onto the nanoparticle and/or the microparticle for activating the cancer-specific T cell together with an immune adjuvant.

In a preferred technical solution of the present disclosure, both the water-soluble portion and the water-insoluble portion may be solubilized by an aqueous solubilizing solution containing the solubilizing agent or the organic solvent. The solubilizing agent is at least one of solubilizing agents that may increase solubility of the protein or the peptide in the aqueous solution; and the organic solvent is an organic solvent that may dissolve the protein or the peptide.

In a preferred technical solution of the present disclosure, the original water-insoluble portion is changed from being insoluble in pure water to being soluble in an aqueous solution containing a solubilizing agent/a dissolving agent or in an organic solvent by an appropriate solubilizing method; and the solubilizing agent/dissolving agent adopted is selected from one or more of a compound with a structure of structural formula 1, a deoxycholate, a lauryl sulfate, glycerol, a protein-degrading enzyme, albumin, lecithin, a peptide, an amino acid, a glycoside, and a choline,
Where structural formula 1 is as follows: the structure of structural formula 1 is as follows:

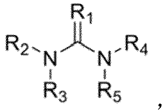

R₁ is C, N, S or O, and R₂-R₅ are independently selected from at least one of hydrogen, alkyl, amino, carboxyl, and substituted or unsubstituted guanidino.

The compound with the structural formula 1 includes but is not limited to metformin hydrochloride, metformin sulfate, metformin sulfonate, a metformin salt, metformin, polyhexamethyleneguanidine hydrochloride, agmatine sulfate, methylguanidine hydrochloride, tetramethylguanidine hydrochloride, urea, guanidine hydrochloride, guanidine sulfate, guanidine sulfonate, a guanidine salt, other compounds containing guanidino or urea, guanidine carbonate, arginine, guanidinoacetic acid, guanidinophosphoric acid, guanidine sulfamate, guanidinosuccinic acid, semicarbazide hydrochloride, carbamoylurea, acetylurea, a sulfonylurea compound (glibenclamide, gliclazide, gliquidone, glimepiride, etc.), a thiourea compound (thiouracil, imidazole, etc.), nitrosoureas, and other compounds with the structure of structural formula 1.

In a preferred technical solution of the present disclosure, a cell component loaded by the nanoparticle or the microparticle for activating the cancer-specific T cell is derived from a component obtained from one or more cancer cells and/or one or more whole cells of tumor tissue, the water-insoluble component is loaded onto a delivery particle, so that the nano-system or micron-system contains more antigens, and more preferably, the water-soluble component and the water-insoluble component are loaded onto the delivery particle simultaneously, so that the delivery particle is loaded with a whole-cell antigen component.

In a preferred technical solution of the present disclosure, for the cell component loaded by the nanoparticle and/or the microparticle for activating the cancer-specific T cell or a mixture thereof, the mixture includes but is not limited to water-soluble components mixed with each other, or water-insoluble components mixed with each other, or all or a portion of water-soluble components and all or a portion of water-soluble components mixed with each other.

In a preferred technical solution of the present disclosure, in the nanoparticle (NP) and/or the microparticle (MP), the cell component or the mixture thereof is loaded into an interior and/or onto the surface of the nanoparticle and/or the microparticle, specifically, a way of the loading is that the water-soluble component and the water-insoluble component of the cell are separately or together encapsulated in the interior of the particle and/or separately or together loaded onto the surface of the particle, including but not limited to the water-soluble component being loaded into the particle and loaded onto the surface of the particle, the water-insoluble component being together loaded into the particle and loaded onto the surface of the particle, the water-soluble component being loaded into the particle but the water-insoluble component being loaded onto the surface of the particle, the water-insoluble component being loaded into the particle and the water-soluble component being loaded onto the surface of the particle, the water-soluble component and the water-insoluble component being loaded into the particle but only the water-insoluble component being loaded onto the surface of the particle, the water-soluble component and the water-insoluble component being loaded into the particle but only the water-soluble component being loaded onto the surface of the particle, the water-soluble component being loaded into the particle but the water-soluble component and the water-insoluble component being together loaded onto the surface of the particle, the water-insoluble component being loaded into the particle but the water-soluble component and the water-insoluble component being together loaded onto the surface of the particle, and the water-soluble component and the water-insoluble component being together loaded into the particle and the water-soluble component and the water-insoluble component being together loaded onto the surface of the particle.

In a preferred technical solution of the present disclosure, the interior and/or the surface of the nanoparticle or the microparticle for activating the cancer-specific T cell may further include an immune-enhancing adjuvant, and the immune-enhancing adjuvant includes but is not limited to at least one of a microorganism-derived immune enhancer, a product of the human or animal immune system, an intrinsic immune agonist, an adaptive immune agonist, a chemically synthesized drug, fungal polysaccharides, traditional Chinese medicine, and others; and the immune-enhancing adjuvant includes but is not limited to at least one of the active ingredients of a pattern recognition receptor agonist, Bacillus Calmette-Guerin (BCG), a manganese-related adjuvant, a BCG cell wall backbone, a BCG methanol extract residue, BCG muramyl dipeptide, mycobacterium phlei, polyactin, mineral oil, a virus-like particle, an immune-enhanced reconstituted influenza virosome, a cholera enterotoxin, a saponin and a derivative thereof, resiquimod, thymosin, a neonatal bovine liver active peptide, miquimod, a polysaccharide, curcumin, immune adjuvant CpG, immune adjuvant poly(I:C), immune adjuvant poly ICLC, corynebacterium pumilus vaccine, a hemolytic streptococcus preparation, coenzyme Q10, levamisole, polycytidylic acid, a manganese adjuvant, an aluminum adjuvant, a calcium adjuvant, various cytokines, an interleukin, an interferon, polyinosinic acid, polyadenylic acid, alum, aluminum phosphate, lanolin, squalene, a cytokine, vegetable oil, endotoxin, a liposome adjuvant, MF59, double-stranded RNA, double-stranded DNA, an aluminum-related adjuvant, CAF01, ginseng, and astragalus membranaceus. It may be understood by those skilled in the art that the enumerated herein is not exhaustive, and other substances that may enhance the immune response may also be adopted as the immune-enhancing adjuvant.

In a preferred technical solution of the present disclosure, the immune-enhancing adjuvant is preferably a Toll-like receptor agonist.

In a preferred technical solution of the present disclosure, the immune-enhancing adjuvant is preferably two or more Toll-like receptor agonists used in combination.

In a preferred technical solution of the present disclosure, the immune adjuvant is co-loaded with the cell component in the nanoparticle or the microparticle, and may better the cancer-specific T cell after the nanoparticle or the microparticle is phagocytosed by the antigen-presenting cell.

In a preferred technical solution of the present disclosure, the microparticle or the nanoparticle encapsulate a substance that increases escape of the nanoparticle and/or the microparticle or loaded antigens thereof from a lysosome to cytoplasm. The substance that increases lysosome escape includes an amino acid, a peptide, saccharides, lipids, and an inorganic salt that may generate a proton sponge effect, preferably, the amino acid in the substance that increases lysosome escape includes a positively charged amino acid, and preferably, the peptide species that increases lysosome escape includes a positively charged amino acid.

In a preferred technical solution of the present disclosure, the surface of the nanoparticle or the microparticle may not be linked to a target head having an active targeting function, or may be linked to a target head having an active targeting function, and
the active targeting target head may be a commonly used target head such as mannose, a CD19 antibody, a CD20 antibody, a BCMA antibody, a CD32 antibody, a CD11c antibody, a CD103 antibody, and a CD44 antibody, and leads the particle system to the antigen-presenting cell *via* targeting delivery.

In a preferred technical solution of the present disclosure, the surface of the nanoparticle and/or the microparticle is linked with a target head actively targeting the antigen-presenting cell.

In a preferred technical solution of the present disclosure, the nanoparticle or the microparticle may not be modified during a preparation process, or an appropriate modification technique may be adopted to increase the antigen-loading capacity of the nanoparticle or the microparticle. The modification technique includes but is not limited to biomineralization (e.g., silicification, calcification or magnesiation), gelation, crosslinking, chemical modification, and addition of charged species.

In a preferred technical solution of the present disclosure, the form in which the cell component or the mixture thereof is loaded into the interior of the nanoparticle or the microparticle may be any way in which the cell component or the mixture thereof may be loaded into the interior of the nanoparticle or the microparticle.

In a preferred technical solution of the present disclosure, the way in which the cell component or the mixture thereof is loaded onto the surface of the nanoparticle or the microparticle includes but is not limited to adsorption, covalent linkage, charge interaction (e.g., addition of a positively charged species and addition of a negatively charged species), hydrophobic interaction, one-step or multi-step solidification, mineralization, encapsulation, etc.

In a preferred technical solution of the present disclosure, the water-soluble component and/or the water-insoluble component loaded onto the surface of the nanoparticle or the microparticle is one or more layers after being loaded, and when the vaccine surface is loaded with multiple layers of the water-soluble component and/or the water-insoluble component, there is a modifier between the layers.

In a preferred technical solution of the present disclosure, the nanoparticle has a particle size of 1 nm to 1000 nm, more preferably a particle size of 30 nm to 1000 nm, and most preferably a particle size of 100 nm to 600 nm.

In a preferred technical solution of the present disclosure, the microparticle has a particle size of 1 µm to 1000 µm, more preferably a particle size of 1 µm to 100 µm, more preferably a particle size of 1 µm to 10 µm, and most preferably a particle size of 1 µm to 5 µm.

In a preferred technical solution of the present disclosure, a shape of the nanoparticle or the microparticle includes any one of a sphere, an ellipsoidal shape, a barrel shape, a polygon, a rod shape, a sheet shape, a linear shape, a worm shape, a square, a triangle, a butterfly shape, or a disc shape.

In a preferred technical solution of the present disclosure, the way in which the water-soluble component and/or the water-insoluble component is loaded onto the surface of the cancer vaccine includes at least one of adsorption, covalent linkage, charge interaction, hydrophobic interaction, one-step or multi-step solidification, mineralization, and encapsulation.

In a preferred technical solution of the present disclosure, a preparation material of the nanovaccine and/or the microvaccine is an organic synthetic polymer material, a natural polymer material or an inorganic material.

In a preferred technical solution of the present disclosure, the organic synthetic polymeric material is a biocompatible or degradable polymeric material, including any one of PLGA, PLA, PGA, PLGA-PEG, PLA-PEG, PGA-PEG, PEG, PCL, Poloxamer, PVA, PVP, PEI, PTMC, a polyanhydride, PDON, PPDO, PMMA, a polyamino acid, a synthetic peptide, and a synthetic lipid, or a combination thereof.

In a preferred technical solution of the present disclosure, the natural polymer material is a biocompatible or degradable polymer material, including any one of lecithin, cholesterol, sodium alginate, albumin, collagen, gelatin, a cell membrane component, starch, saccharides, and a peptide, or a combination thereof.

In a preferred technical solution of the present disclosure, the inorganic material is a material without significant biological toxicity, including but not limited to ferric oxide, ferroferric oxide, calcium carbonate, calcium phosphate, etc.

Another purpose of the present disclosure is to provide a specific T cell prepared by the method of the present disclosure.

Another purpose of the present disclosure is to provide a cancer-specific T cell derived from an autologous or allogeneic source for preventing or treating cancer, the specific T cell including but not limited to any one of T cells such as CD3⁺ CD69⁺, CD3⁺ CD8⁺ CD69⁺, CD3⁺ CD4⁺ CD69+, CD3⁺ CD137⁺, CD3⁺ CD4⁺ CD137⁺, CD3⁺ CD8⁺ CD137⁺, CD3⁺ CD25⁺, CD3⁺CD8⁺ CD25⁺, CD3⁺CD4⁺CD25⁺, CD3⁺ CD134⁺, CD3⁺ CD8⁺ CD134⁺, CD3⁺ CD4⁺ CD134⁺, CD3⁺ IL-2R⁺, CD3⁺ CD8⁺ IL-2R⁺, CD3⁺ CD4⁺ IL-2R⁺, CD3⁺ HLA-DR⁺, CD3⁺ CD8⁺ HLA-DR⁺, CD3⁺ CD4⁺ HLA-DR⁺, CD3⁺ FASL⁺ CD3⁺ CD8⁺ FASL⁺, CD3⁺ CD4⁺ FASL⁺, CD3⁺ OX40⁺, CD3⁺ CD8⁺ OX40⁺, CD3⁺ CD4⁺ OX40⁺, CD3⁺ TCF-1⁺, CD3⁺ CD8⁺ TCF-1⁺, CD3⁺ CD4⁺ TCF-1⁺, CD3⁺ PD-1⁺, CD3⁺ CD8⁺ PD-1⁺, CD3⁺ CD4⁺PD-1⁺, CD3⁺ CD39⁺, CD3⁺ CD8⁺ CD39⁺, CD3⁺ CD4⁺ CD39⁺, CD3⁺ CD38⁺, CD3⁺ CD8⁺ CD38⁺, CD3⁺ CD4⁺ CD38⁺, CD3⁺ CD28⁺, CD3⁺ CD8⁺ CD28⁺, CD3⁺ CD4⁺ CD28⁺, CD3⁺ CD71⁺, CD3⁺ CD8⁺ CD71⁺, CD3⁺ CD4⁺ CD71⁺, CD3⁺ CD44⁺, CD3⁺ CD8⁺ CD44⁺, CD3⁺ CD4⁺ CD44⁺, CD3⁺ CXCR3⁺, CD3⁺ CD8⁺ CXCR3⁺, CD3⁺ CD4⁺ CXCR3⁺, CD3⁺ CXCR1⁺, CD3⁺ CD8⁺ CXCR1⁺, CD3⁺ CD4⁺ CXCR1⁺, CD3⁺ ICAM-1⁺, CD3⁺ CD8⁺ ICAM-1⁺, CD3⁺ CD4⁺ ICAM-1⁺, CD3⁺CD70⁺, CD3⁺CD8⁺ CD70⁺, CD3⁺ CD4⁺ CD70⁺, CD3⁺ CD154⁺, CD3⁺ CD8⁺ CD154⁺, CD3⁺ CD4⁺ CD154⁺, CD3⁺ CD62L⁺, CD3⁺ CD8⁺ CD62L⁺, CD3⁺ CD4⁺ CD62L⁺, CD3⁺ CD154⁺, CD3⁺ CD8⁺ CD154⁺, CD3⁺ CD4⁺ CD154⁺, CD3⁺ CD160⁺, CD3⁺CD8⁺ CD160⁺, CD3⁺ CD4⁺ CD160⁺, CD3⁺ CD160⁺, CD3⁺ CD8⁺ CD160⁺, CD3⁺ CD4⁺ CD160⁺, CD3⁺ ICOS⁺, CD3⁺ CD8⁺ ICOS ⁺, CD3⁺ CD4⁺ ICOS⁺, CD3⁺ CD27⁺, CD3⁺ CD8⁺ CD27⁺, CD3⁺ CD4⁺ CD27⁺, CD3⁺ CD107A⁺, CD3⁺ CD8⁺ CD107A⁺, CD3⁺ CD4⁺ CD107A⁺, etc., or a combination thereof, where viability of the specific T cell is greater than 60%, preferably greater than 70%, and more preferably greater than 80%; and
the specific T cell is obtained by co-incubating a nanoparticle and/or a microparticle loaded with a tumor antigen component with a peripheral blood mononuclear cell (PBMC) isolated from an immune cell of peripheral blood or a peripheral immune organ.

In some embodiments, the PBMC cell is sorted and then co-incubated with the nanoparticle and/or the microparticle loaded with the tumor antigen component.

In some embodiments, the cell co-incubated with the nanoparticle and/or the microparticle loaded with the tumor antigen component is sorted again to obtain the specific T cell.

In some embodiments, the specific T cell may also be subjected to a step of *in vitro* expansion, the step may be performed before the above sorting step or after the above sorting step, and preferably after the sorting step.

In some embodiments, when the PBMC cell is co-incubated with the nanoparticle and/or the microparticle, an antigen-presenting cell (APC) is also present.

In some preferred embodiments, the specific T cell is a CD3⁺ CD8⁺ CD69⁺ cell; in some preferred embodiments, the specific T cell is a CD3⁺ CD137⁺ cell; in some preferred embodiments, the specific T cell is a CD3⁺ CD8⁺ CD25⁺ cell and/or a CD3⁺ CD8⁺ CD69⁺ cell; in some preferred embodiments, the specific T cell is a CD3⁺ CD69⁺ cell; in some preferred embodiments, the specific T cell is a CD3⁺ CD69⁺ cell; in some preferred embodiments, the specific T cell is a CD3⁺ CD8⁺ CD69⁺ cell; in some preferred embodiments, the specific T cell is a CD3⁺ CD8⁺ CD25⁺ cell and/or a CD3⁺ CD4⁺ CD69⁺ cell; in some preferred embodiments, the specific T cell is a CD8⁺ CD69⁺ cell; in some preferred embodiments, the specific T cell is a CD8⁺ CD137⁺ cell; in some preferred embodiments, the specific T cell is a CD8⁺ CD69⁺ cell and/or a CD4⁺ CD69⁺ cell; in some preferred embodiments, the specific T cell is a CD3⁺ CD25⁺ cell; in some preferred embodiments, the specific T cell is CD3⁺ HLA-DR⁺; and in some preferred embodiments, the specific T cell is CD3⁺ FASL⁺.

The various types of T cells may be used alone or in combination according to a need of a patient.

A purpose of the present disclosure is to provide a method for preparing a cancer-specific T cell derived from an autologous or allogeneic source for preventing or treating cancer, specifically including steps of:
(1) isolating a mononuclear cell PBMC from peripheral blood or the immune cell of the peripheral immune organ; preferably, the PBMC is subjected to a first step of sorting to obtain at least one of the following effector T cells: a CD3⁺ CD8⁺ T cell, a CD19⁺ B cell, a CD3⁺ T cell, a CD8⁺ T cell, a CD4⁺ T cell, a B220⁺ B cell, a CD69⁻ PBMC cell, a CD25⁻ PBMC cell, a CD3⁺ CD69⁺ T cell, and a CD11c⁺ DC cell; before the T cell is co-incubated with the nanoparticle and/or the microparticle and the antigen-presenting cell, the T cell may be cultured separately at rest for a period of time or appropriately sorted; or before the T cell is co-incubated with the activated antigen-presenting cell, the T cell may be cultured separately at rest for a period of time or appropriately sorted.
(2) preparing the nanoparticle and/or the microparticle loaded with a tumor antigen component,
   where preferably, the tumor antigen component is prepared by first isolating a tumor cell or tissue, and lysing the tumor cell or the tissue to obtain any one of a water-soluble component, a water-insoluble component and a whole component, or a combination thereof; and
   preferably, the nanoparticle and/or the microparticle loaded with the tumor antigen component is prepared by a double emulsion method;
(3) adding the nanoparticle and/or the microparticle loaded with the tumor antigen component into a medium, co-incubating with the PBMC cell or the T cell in the step (1) to obtain a cell culture, and further sorting a specific T cell from the cell culture, where the specific T cell includes but is not limited to any one of T cells such as CD3⁺ CD69⁺, CD3⁺ CD8⁺ CD69⁺, CD3⁺ CD4⁺ CD69+, CD3⁺ CD137⁺, CD3⁺ CD4⁺ CD137⁺, CD3⁺ CD8⁺ CD137⁺, CD3⁺ CD25⁺, CD3⁺ CD8⁺ CD25⁺, CD3⁺ CD4⁺ CD25⁺, CD3⁺CD134⁺, CD3⁺ CD8⁺ CD134⁺, CD3⁺ CD4⁺ CD134⁺, CD3⁺ IL-2R⁺, CD3⁺ CD8⁺ IL-2R⁺, CD3⁺ CD4⁺ IL-2R⁺, CD3⁺ HLA-DR⁺, CD3⁺ CD8⁺ HLA-DR⁺, CD3⁺ CD4⁺ HLA-DR⁺, CD3⁺ FASL⁺ CD3⁺ CD8⁺ FASL⁺, CD3⁺ CD4⁺ FASL⁺, CD3⁺ OX40⁺, CD3⁺ CD8⁺ OX40⁺, CD3⁺ CD4⁺ OX40⁺, CD3⁺ TCF-1⁺, CD3⁺ CD8⁺ TCF-1⁺, CD3⁺ CD4⁺ TCF-1⁺, CD3⁺ PD-1⁺, CD3⁺ CD8⁺ PD-1⁺, CD3⁺ CD4⁺ PD-1⁺, CD3⁺ CD39⁺, CD3⁺ CD8⁺ CD39⁺, CD3⁺ CD4⁺ CD39⁺, CD3⁺ CD38⁺, CD3⁺ CD8⁺ CD38⁺, CD3⁺ CD4⁺ CD38⁺, CD3⁺ CD28⁺, CD3⁺ CD8⁺ CD28⁺, CD3⁺ CD4⁺ CD28⁺, CD3⁺ CD71⁺, CD3⁺ CD8⁺ CD71⁺, CD3⁺ CD4⁺ CD71⁺, CD3⁺ CD44⁺, CD3⁺ CD8⁺ CD44⁺, CD3⁺ CD4⁺ CD44⁺, CD3⁺ CXCR3⁺, CD3⁺ CD8⁺ CXCR3⁺, CD3⁺ CD4⁺ CXCR3⁺, CD3⁺ CXCR1⁺, CD3⁺ CD8⁺ CXCR1⁺, CD3⁺ CD4⁺ CXCR1⁺, CD3⁺ ICAM-1⁺, CD3⁺ CD8⁺ ICAM-1⁺, CD3⁺ CD4⁺ ICAM-1⁺, CD3⁺ CD70⁺, CD3⁺ CD8⁺ CD70⁺, CD3⁺ CD4⁺ CD70⁺, CD3⁺ CD154⁺, CD3⁺ CD8⁺ CD154⁺, CD3⁺ CD4⁺ CD154⁺, CD3⁺ CD62L⁺, CD3⁺ CD8⁺ CD62L⁺, CD3⁺ CD4⁺ CD62L⁺, CD3⁺ CD154⁺, CD3⁺ CD8⁺ CD154⁺, CD3⁺ CD4⁺ CD154⁺, CD3⁺CD160⁺, CD3⁺ CD8⁺ CD160⁺, CD3⁺ CD4⁺ CD160⁺, CD3⁺ CD160⁺, CD3⁺ CD8⁺ CD160⁺, CD3⁺ CD4⁺ CD160⁺, CD3⁺ ICOS⁺, CD3⁺ CD8⁺ ICOS ⁺, CD3⁺ CD4⁺ ICOS⁺, CD3⁺ CD27⁺, CD3⁺ CD8⁺ CD27⁺, CD3⁺ CD4⁺ CD27⁺, CD3⁺ CD107A⁺, CD3⁺ CD8⁺ CD107A⁺, CD3⁺ CD4⁺ CD107A⁺, etc., or a combination thereof; and one or more components of a surface activation marker may be used as the surface activation marker.
   Preferably, in the co-incubation process, 0.1 million/mL -50 million/mL of antigen-presenting cells are further added, and the antigen-presenting cells are any one of B cells, DC cells and macrophages, or a combination thereof;
   preferably, the nanoparticle and/or the microparticle loaded with the tumor antigen component may be co-incubated simultaneously with the antigen-presenting cell and the T cell to activate the cancer-specific T cell; or the nanoparticle and/or the microparticle loaded with the tumor antigen component may be first co-incubated with the antigen-presenting cell to activate the antigen-presenting cell, and then the activated antigen-presenting cell is co-incubated separately with the T cell to activate the cancer-specific T cell; and after the nanoparticle and/or the microparticle loaded with the tumor antigen component is first co-incubated with the antigen-presenting cell to activate the antigen-presenting cell, the antigen-presenting cell may be co-incubated with the T cell to activate the specific T cell without special treatment, or the antigen-presenting cell may be treated with fixation, radiation, irradiation, modification, inactivation, mineralization, etc., and then co-incubated with the T cell to activate the specific T cell;
   preferably, 2.5 ng/mL -50 mg/mL of the nanoparticle and/or the microparticle loading the tumor antigen component is added into the medium, and incubated with 0.01 million/mL -50 million/mL of the PBMC cells or the sorted cells for 4 h to 96 h under conditions of 30-38°C and 1-5% CO₂ to obtain a cell culture;
   preferably, in the co-incubation process, 10 ng/mL to 500 ng/mL of an interleukin is further added, and the interleukin is any one of IL-2, IL-7, IL-12, IL-15, IL-17, and IL-21, or a combination thereof;
   the medium is any one of DMEM high glucose complete medium, RPM 1640 medium and AIM V serum-free medium;
   the co-incubation is performed under conditions of 30-38°C for 1 h to 168 h, preferably 4 h to 96 h, and more preferably 6 h to 72 h; and
   viability of the specific T cell is greater than 60%, preferably greater than 70%, and more preferably greater than 80%; and
(4) expanding the specific T cell obtained in the step (3),
   where preferably, steps of the expanding are adding 0.01-50 million cells/mL of the specific T cell sorted in the step (2) to an expansion medium, co-incubating under conditions of 30-38°C and 1-5% CO2, changing the medium using an expansion medium every 2 days to 3 days, and co-incubating for 5 days to 30 days to obtain an expanded specific T;
   preferably, the expansion medium is performed in the expansion medium, and the expansion medium is any one of DMEM high glucose complete medium and RPM 1640 medium;
   preferably, a condition of expansion culture is culturing at 30-38°C for 4 days to 72 days, and preferably 5 days to 30 days;
   preferably, the expansion medium further includes 200 U/mL -1000 U/mL of an interleukin, 10 ng/mL - 200 ng/mL of an antibody and/or 1 ng/mL - 10 ng/mL of a granulocyte-macrophage colony-stimulating factor (GM-CSF);
   preferably, the interleukin is any one of IL-2, IL-7, IL-12, IL-15, IL-17, and IL-21, or a combination thereof; and
   preferably, the antibody is any one of an αCD3 antibody and an αCD28 antibody, or a combination thereof.

In a preferred technical solution of the present disclosure, in the step (1), the cell is selected from any part of an individual suffering from a neoplastic disease, and preferably a splenocyte and a lymphocyte.

Viability of the expanded specific T cell is greater than 60%, preferably greater than 75%, and more preferably greater than 80%.

In a preferred technical solution of the present disclosure, in the step (2), the method of the sorting is isolating a cell expressing the specific cell marker from a cell population by flow cytometry or the magnetic bead method, etc. after binding an antibody with fluorescence or magnetism or a specific ligand to the specific cell marker on a surface of the T cell.

In a preferred technical solution of the present disclosure, a cytokine may be added in the mixing and co-incubating; and the cytokine added includes but is not limited to an interleukin, a tumor necrosis factor, an interferon, and a growth factor; and preferably, the cytokine added includes interleukin 7 (IL-7) and interleukin 15 (IL-15).

In a preferred technical solution of the present disclosure, in the step (3), a concentration of the cytokine is 1 ng/mL - 6000 ng/mL, preferably 5 ng/mL - 100 ng/mL, and more preferably 10 ng/mL - 30 ng/mL.

In a preferred technical solution of the present disclosure, in step (3), the cytokine includes but is not limited to an interleukin, an interferon and a tumor necrosis factor.

In a preferred technical solution of the present disclosure, the interleukin includes but is not limited to interleukin 2 (IL-2), interleukin 7 (IL-7), interleukin 12 (IL-12), interleukin 15 (IL-15), interleukin 17 (IL-17), and interleukin 21 (IL-21).

In a preferred technical solution of the present disclosure, a concentration of the antibody is 1 ng/mL - 6000 ng/mL, preferably 5 ng/mL -100 ng/mL, and more preferably 10 ng/mL -30 ng/mL.

In a preferred technical solution of the present disclosure, the antibody includes but is not limited to any one of an αCD3 antibody, an αCD28 antibody, an αCD80 antibody, an αCD86 antibody, and an α0X40 antibody, or a combination thereof.

In a preferred technical solution of the present disclosure, time of the co-incubation of the nanoparticle and/or the microparticle with a mixture of the antigen-presenting cell and the T cell is at least 1 hour, and preferably 4 hours to 96 hours.

In a preferred technical solution of the present disclosure, time of the co-incubation of the nanoparticle and/or the microparticle with the antigen-presenting cell alone is at least 1 hour, and preferably 4 hours to 96 hours.

In a preferred technical solution of the present disclosure, time of the co-incubation of a mixture of the activated antigen-presenting cell and the T cell is at least 1 hour, and preferably 6 hours to 96 hours.

In a preferred technical solution of the present disclosure, time of the expansion culture is at least 1 day, and preferably 4 days to 72 days.

In a preferred technical solution of the present disclosure, when the nanoparticle and/or the microparticle is co-incubated with the antigen-presenting cell alone, a concentration of the nanoparticle and/or the microparticle is 10 ng/mL to 5 mg/mL.

In a preferred technical solution of the present disclosure, when the nanoparticle and/or the microparticle is co-incubated with the antigen-presenting cell and the T cell, a concentration of the nanoparticle and/or the microparticle is 2.5 ng/mL to 50 mg/mL.

In a preferred technical solution of the present disclosure, a content of the protein and peptide components in the antigen component loaded by the nanoparticle and/or the microparticle is higher than 10 ng/mL.

In a preferred technical solution of the present disclosure, in the steps (2) and (3), the nanoparticle or microparticle is PLGA, a molecular weight is selected from 7 KDa to 54 KDa, and the immune adjuvant is selected from any one of poly(I:C), polyICLC, BCG, and CpG, or a combination thereof.

In a preferred technical solution of the present disclosure, in the steps (2) and (3), the nanoparticle or microparticle is PLGA, a molecular weight is selected from 7 KDa to 51 KDa, and the immune adjuvant is selected from any one of poly(I:C), polyICLC, BCG, and CpG, or a combination thereof.

In a preferred technical solution of the present disclosure, a molecular weight of the nanoparticle PLGA adopted is 24 KDa to 38 KDa, the immune adjuvant adopted is poly(I:C), and the poly(I:C) is only distributed in the interior of the nanoparticle.

In a preferred technical solution of the present disclosure, a molecular weight of the nanoparticle PLGA adopted is 7 Da to 17 KDa, the immune adjuvants adopted are poly(I:C) and CpG1018, and a mass ratio of the two immune adjuvants is preferably 1:1.

In a preferred technical solution of the present disclosure, a molecular weight of the nanoparticle PLGA adopted is 7 KDa to 17 KDa, the immune adjuvant adopted is poly(I:C), CpG2006 and CpG2216, and a mass ratio of the three immune adjuvants is preferably 1:1:1.

In a preferred technical solution of the present disclosure, a molecular weight of the nanoparticle PLGA adopted is 24 KDa to 38 KDa, the immune adjuvant adopted is poly(I:C), the poly(I:C) is distributed in the interior of the nanoparticle and loaded onto the surface of the nanoparticle, and the interior and outside of the nanoparticle are loaded with a lysate modified by cryosilicification and addition of a cationic substance.

In a preferred technical solution of the present disclosure, a molecular weight of the microparticle PLGA adopted is 24 KDa to 38 KDa, the immune adjuvant adopted is poly(I:C), the poly(I:C) is distributed in the interior of the microparticle and loaded onto the surface of the microparticle, and the interior and outside of the microparticle are loaded with a lysate modified by cryosilicification and addition of a cationic substance.

In a preferred technical solution of the present disclosure, a molecular weight of the nanoparticle PLA adopted is 20 KDa, the immune adjuvants adopted are poly(I:C) and CpG1018, and a mass ratio of the two immune adjuvants is preferably 1:1.

In a preferred technical scheme of the present disclosure, a molecular weight of the nanoparticle PLGA adopted is 24 KDa to 38 KDa, the immune adjuvants adopted are Poly(I:C) and CpG1018, and the antigen component and the immune adjuvant are simultaneously distributed in the interior and on the surface of the nanoparticle.

In a preferred technical solution of the present disclosure, a molecular weight of the microparticle PLGA adopted is 38 KDa to 54 KDa, CpG and Poly ICLC are used as immune adjuvants, and in order to promote lysosome escape, the system may be added with arginine.

In a preferred technical solution of the present disclosure, a molecular weight of the nanoparticle PLGA adopted is 24 KDa to 38 KDa, the immune adjuvants are CpG and Poly(I:C), and in order to promote lysosome escape, the system may be added with KALA peptide (WEAKLAKALAKALAKHLAKALAKALKACEA), and a mass ratio of the two immune adjuvants is preferably 1:1.

In a preferred technical solution of the present disclosure, a molecular weight of the nanoparticle PLGA adopted is 7 KDa to 17 KDa, the immune adjuvant adopted is BCG, and BCG is loaded into the interior of the nanoparticle.

In a preferred technical solution of the present disclosure, the nanoparticles adopted are PLGA and mannose-modified PLGA, a mass ratio is 4:1, a molecular weight is 7 KDa to 17 KDa, the immune adjuvants adopted are Poly(I:C) and CpG, and a mass ratio of the two immune adjuvants is preferably 1:1.

In a preferred technical solution of the present disclosure, a molecular weight of the nanoparticle PLGA adopted is 24 KDa to 38 KDa, the immune adjuvants adopted are BCG and Poly(I:C), and a mass ratio of the two immune adjuvants is preferably 1:1.

In a preferred technical solution of the present disclosure, the biocalcified nanoparticles are loaded with a whole-cell antigen in the interior and on the surface of the nanoparticles, a molecular weight of PLGA is 7 KDa to 17 KDa, the immune adjuvants adopted are CpG and Poly(I:C) and GALA peptide (WEAALAEALAEALAEHLAEALAEALEALAA) for enhancing lysosome escape is loaded into the interior of the nanoparticle, and a mass ratio of the two immune adjuvants is preferably 1:1.

In a preferred technical solution of the present disclosure, a molecular weight of the nanoparticle PLGA adopted is 7 KDa to 17 KDa, the immune adjuvants adopted are poly(I:C) and CpG, and in order to promote lysosome escape, the system is added with melittin, and the adjuvant and melittin are wrapped in the nanoparticle, and a mass ratio of the two immune adjuvants is preferably 1:1.

In a preferred technical solution of the present disclosure, the nanoparticles adopted are PLGA and mannan-modified PLGA, both of which have a molecular weight of 24 KDa to 38 KDa, a mass ratio of PLGA and mannan-modified PLGA is 9:1, the immune adjuvants adopted are poly(I:C) and CpG, and in order to promote lysosome escape, the system is added with polyarginine and polylysine, and the adjuvant, polyarginine and polylysine are encapsulated in the nanoparticles, and a mass ratio of the two immune adjuvants is preferably 1:1.

In a preferred technical solution of the present disclosure, a molecular weight of the microparticle PLGA adopted is 38 KDa to 54 KDa, and the immune adjuvants adopted are CpG1018 and Poly ICLC, and in order to promote lysosome escape, the system is added with KALA peptide, and a mass ratio of the two immune adjuvants is preferably 1:1.

In a preferred technical solution of the present disclosure, skeleton materials of the microparticle are unmodified PLA and mannose-modified PLA, both of which have a molecular weight of 40 KDa, and a ratio of the unmodified PLA and the mannose-modified PLA is 9:1. The immune adjuvants adopted are CpG2395 and Poly ICLC, and in order to promote lysosome escape, the system is added with arginine and/or histidine, and a mass ratio of the two immune adjuvants is preferably 1:1.

In a preferred technical solution of the present disclosure, a molecular weight of the nanoparticle PLGA adopted is 7 KDa to 17 KDa, the immune adjuvants adopted are poly(I:C) and CpG1018, and in order to promote lysosome escape, the system is added with R8 peptide, and the adjuvant and R8 peptide are wrapped in the nanoparticle, and a mass ratio of the two immune adjuvants is preferably 1:1.

In a preferred technical solution of the present disclosure, a molecular weight of the nanoparticle PLGA adopted is 7 KDa to 17 KDa, Poly(I:C) and CpG1018 are used as adjuvants, and in order to promote lysosome escape, the system is added with NH₄HCO₃, and the adjuvant and NH₄HCO₃ are loaded into the nanoparticle, and a mass ratio of the two immune adjuvants is preferably 1:1.

In a preferred technical solution of the present disclosure, a molecular weight of the nanoparticle PLGA adopted is 20 KDa to 40 KDa, and the immune adjuvant adopted is poly(I:C).

In a preferred technical solution of the present disclosure, the nanoparticles adopted are PLA (molecular weight of 30 KDa to 40 KDa) and mannan-PEG2000-PLA (molecular weight of 30 KDa to 40 KDa), and a mass ratio of PLA (molecular weight of 30 KDa to 40 KDa) and mannan-PEG2000-PLA (molecular weight of 30 KDa to 40 KDa) is 9:1, the immune adjuvants adopted are CpG2006 (class B), CpG2216 (class A) and Poly ICLC, and a mass ratio of the three immune adjuvants is preferably 1:1:1.

In a preferred technical solution of the present disclosure, a molecular weight of the nanoparticle PLGA adopted is 10 KDa to 20 KDa, the immune adjuvants adopted are poly(I:C), CpG 7909 and CpG2395, and a mass ratio of the three immune adjuvants is preferably 1:1:1.

In a preferred technical solution of the present disclosure, a molecular weight of the nanoparticle PLGA adopted is 10 KDa to 20 KDa, the immune adjuvants loaded are poly(I:C) and CpG7909, and a mass ratio of the two immune adjuvants is preferably 1:1.

In a preferred technical solution of the present disclosure, a molecular weight of the microparticle PLGA adopted is 38 KDa to 54 KDa, and the immune adjuvants adopted are CpG1018 and Poly ICLC, and in order to promote lysosome escape, the system is added with KALA peptide, and a mass ratio of the two immune adjuvants is preferably 1:1.

In a preferred technical solution of the present disclosure, the tumor antigen component contains a protein/peptide component in a cell lysis component of tumor tissue and/or a cancer cell, and a preparation method thereof is: (1) first, preparing a lysate of the tumor tissue/the cancer cell; then, preparing a water-soluble component and a water-insoluble component in the lysate; then, preparing the protein/peptide component in the water-soluble component and the water-insoluble component by methods such as salting-out, heating and enzymatic hydrolysis, separately; and then, loading the protein/peptide component on the nanoparticle or the microparticle as the antigen component; (2) or directly adopting a solubilizing solution containing a solubilizing agent to directly lyse the cell or the tissue and dissolving a whole-cell component, and then preparing the protein/peptide component therein separately by methods such as salting-out, heating and enzymatic hydrolysis, and loading the protein/peptide component on the nanoparticle or the microparticle as the antigen component. The water-insoluble component and a precipitate obtained after treatment such as salting-out, heating and enzymatic hydrolysis are dissolved by a dissolving solution containing a dissolving agent.

In a preferred technical solution of the present disclosure, a method for preparing a whole-cell component is: (1) first, lysing the cancer cell/the tumor tissue; then, preparing a water-soluble component and a water-insoluble component separately; and then, dissolving the water-insoluble component using a specific dissolving agent containing the dissolving solution for use; or (2) lysing the cell using the dissolving solution containing the dissolving agent, and then dissolving a lysed whole-cell component using the dissolving solution containing the dissolving agent.

In a preferred technical solution of the present disclosure, a method for preparing a portion of a whole-cell component containing whole-cell protein and peptide components in the whole-cell component of a cancer cell is: (1) first, lysing the cancer cell/the tumor tissue; then, preparing the water-soluble component and the water-insoluble component separately; then, dissolving the water-insoluble component using the specific dissolving agent containing the dissolving solution for use; then, isolating and extracting the protein and peptide components in the water-soluble component from the water-soluble component using an appropriate method; and then, using the protein and peptide components isolated and extracted from the water-soluble component together with all the water-insoluble component as the antigen component; (2) first, lysing the cancer cell/the tumor tissue; then, preparing the water-soluble component and the water-insoluble component separately; then, dissolving the water-insoluble component using the specific dissolving agent containing the dissolving solution for use; then, isolating and extracting the protein and peptide components in the water-soluble component from the water-insoluble component using an appropriate method; and then, using the protein and peptide components isolated and extracted from the water-insoluble component together with all the water-soluble component as the antigen component; (3) first, lysing the cancer cell/the tumor tissue; then, preparing the water-soluble component and the water-insoluble component separately; then, dissolving the water-insoluble component using the specific dissolving agent containing the dissolving solution; then, isolating and extracting the protein and peptide components in the water-soluble component from the water-soluble component and the water-insoluble component using an appropriate method, respectively; and then, using the protein and peptide components isolated and extracted from the water-soluble component and the water-insoluble component together as the antigen component; or (4) lysing the cell using a dissolving solution containing a dissolving agent; then, dissolving a lysed whole-cell component using the dissolving solution containing the dissolving agent; and then, isolating and extracting protein and peptide components therein using an appropriate method.

The appropriate method for isolating and extracting the above protein and peptide components includes but is not limited to salting-out, heating, enzymatic hydrolysis, etc.

The protein and peptide components are isolated and extracted, and then re-dissolved in the dissolving solution containing the dissolving agent.

In a preferred technical solution of the present disclosure, in the step (2), the tumor antigen component is prepared by first isolating a tumor cell or tissue, and lysing the tumor cell or the tissue to obtain any one of a water-soluble component, a water-insoluble component and a whole component, or a combination thereof.

In a preferred technical solution of the present disclosure, in the step (2), the tumor antigen component is appropriately treated by methods such as salting-out, heating and enzymatic hydrolysis.

In a preferred technical solution of the present disclosure, both the water-soluble portion and the water-insoluble portion may be solubilized by an aqueous solubilizing solution containing the solubilizing agent or the organic solvent.

In a preferred technical solution of the present disclosure, the solubilizing agent is at least one of solubilizing agents that may increase solubility of the protein or the peptide in the aqueous solution; and the organic solvent is an organic solvent that may dissolve the protein or the peptide.

In a preferred technical solution of the present disclosure, the water-insoluble portion is changed from insoluble in pure water to soluble in an aqueous solution containing a solubilizing agent or in an organic solvent by an appropriate solubilizing method; and the solubilizing agent adopted is selected from one or more of a compound with a structure of structural formula 1, a deoxycholate, a lauryl sulfate, glycerol, a protein-degrading enzyme, albumin, lecithin, a peptide, an amino acid, a glycoside, and a choline,
where the structural formula 1 is as follows: the structure of structural formula 1 is as follows: 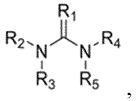 R₁ is C, N, S or O, and R₂-R₅ are independently selected from at least one of hydrogen, alkyl, amino, carboxyl, and substituted or unsubstituted guanidino.

The compound with the structure of structural formula 1 includes but is not limited to metformin hydrochloride, metformin sulfate, metformin sulfonate, a metformin salt, metformin, urea, guanidine hydrochloride, guanidine sulfate, guanidine sulfonate, a guanidine salt, other compounds containing guanidino, guanidine carbonate, arginine, guanidinoacetic acid, guanidinophosphoric acid, guanidine sulfamate, guanidinosuccinic acid, semicarbazide hydrochloride, carbamoylurea, acetylurea, a sulfonylurea compound (glibenclamide, gliclazide, gliquidone, glimepiride, etc.), a thiourea compound (thiouracil, imidazole, etc.), nitrosoureas, etc.

Urea, guanidine hydrochloride,etc. with the structure of structural formula 1, and the inventors have found that a substance with the structure of structural formula 1 may dissolve a water-insoluble component in a cell or tumor tissue as a dissolving agent in a dissolving solution, and thus, in addition to common compounds with a guanidine group structure such as urea, guanidine hydrochloride and metformin, other compounds with the structure have the ability to dissolve the water-insoluble component as a dissolving agent.

In a preferred technical solution of the present disclosure, the immune adjuvant is co-loaded with the cell component in the nanoparticle or the microparticle, and may better the cancer-specific T cell after the nanoparticle or the microparticle is phagocytosed by the antigen-presenting cell.

In a preferred technical solution of the present disclosure, a method for preparing the water-soluble component is: cutting the tumor tissue or the cancer cell into pieces and then grinding, filtering to obtain a single-cell suspension, adding water to freeze and thaw repeatedly for 1-5 times, ultrasonically lysing, centrifuging a lysate at a rotational speed of 5000-10000 g for 5-10 minutes, and taking a supernatant as the water-soluble component and a precipitated portion as the water-insoluble component.

In a preferred technical solution of the present disclosure, the antigen component is a soluble component obtained by dissolving the water-insoluble component by adding a dissolving agent, and the dissolving agent is any one of urea, sodium deoxycholate, guanidine hydrochloride, octyl glucoside, arginine, glycerol, semicarbazide hydrochloride, and agmatine sulfate, or a combination thereof.

In a preferred technical solution of the present disclosure, the antigen component is a mixture obtained by mixing a soluble component obtained by dissolving the water-insoluble component by adding an aqueous urea solution with the water-soluble component at a ratio of 3-1:1.

In a preferred technical solution of the present disclosure, the antigen component is a water-soluble component of the tumor cell and a water-soluble component of the cancer cell mixed at a ratio of 1:1, or a soluble component obtained by dissolving the water-insoluble component of the tumor cell after adding a dissolving agent and a soluble component obtained by dissolving the water-insoluble component of the cancer cell after adding a dissolving agent mixed at a ratio of 1:1.

In a preferred technical solution of the present disclosure, the tumor tissue or the cancer cell is subjected to any one of inactivation and denaturation treatment by ultraviolet high-temperature heating and inactivation treatment by adding nuclease, or a combination thereof in advance.

In a preferred technical solution of the present disclosure, the antigen component is a component obtained after the water-soluble component is subjected to salting-out and precipitation by heating.

In a preferred technical solution of the present disclosure, the antigen component is a soluble component obtained by adding the dissolving agent into the precipitated portion after lysis and centrifugation of the tumor tissue for dissolution to obtain a precipitate, and then adding a solubilizing agent to the precipitate for secondary dissolution, and the dissolving agent is any one of Tween 80 and guanidine sulfate, or a combination thereof.

Another purpose of the present disclosure is to provide a method for preparing a pharmaceutical composition including the specific T cell of the present disclosure, including a step of adding a substance enhancing an innate immune system, such as albumin, an NK cell, a neutrophil, a γδ T cell, and an NK T cell, to the cancer-specific T cell before reinfusing the cancer-specific T cell into the patient.

In a preferred technical solution of the present disclosure, a cell concentration of the specific T cell is (0.01-100) × 10⁷ cells/mL, and preferably (0.1-8) × 10⁷ cells/mL.

In a preferred technical solution of the present disclosure, the pharmaceutical composition further includes any one of hydroxyethyl starch, sugar and salt, or a combination thereof.

Another purpose of the present disclosure is an application of the specific T cell of the present disclosure in preparation of a medicament for treating or preventing cancer.

In a preferred technical solution of the present disclosure, the specific T cell is administered multiple times before cancer occurrence, after cancer occurrence or after surgical excision of tumor tissue.

Another purpose of the present disclosure is an application of the specific T cell of the present disclosure in preparation of a medicament for preventing cancer recurrence or preventing cancer metastasis.

Another purpose of the present disclosure is to provide an application of the specific T cell of the present disclosure in preparation of a product for immunotherapy against a tumor.

In a preferred technical solution of the present disclosure, the tumor is selected from a solid tumor, a hematoma and a lymphoma. The tumor includes but is not limited to any one of lung cancer, ovarian cancer, colon cancer, rectal cancer, melanoma, kidney cancer, bladder cancer, breast cancer, liver cancer, lymphoma, a malignant hematoma such as leukemia, brain tumor, head and neck cancer, glioma, gastric cancer, nasopharyngeal cancer, laryngeal cancer, cervical cancer, a uterine body tumor, osteosarcoma, bone cancer, pancreatic cancer, skin cancer, prostate cancer, uterine cancer, anal region cancer, testicular cancer, oviduct cancer, endometrial cancer, vaginal cancer, vulval cancer, Hodgkin's disease, non-Hodgkin's lymphoma, esophageal cancer, small intestine cancer, endocrine system cancer, thyroid cancer, parathyroid cancer, adrenal cancer, soft tissue sarcoma, urethra cancer, penile cancer, chronic or acute leukemia, a pediatric solid tumor, lymphocytic lymphoma, bladder cancer, kidney or ureter cancer, renal pelvis cancer, a central nervous system (CNS) tumor, primary CNS lymphoma, tumor angiogenesis, spinal tumor, brain stem glioma, pituitary adenoma, Kaposi's sarcoma, epidermoid carcinoma, squamous cell carcinoma, T-cell lymphoma, environmentally induced cancer, metastatic cancer, and a circulating tumor cell, or a combination thereof.

Preferably, the tumor is selected from any one of melanoma, colon cancer, triple-negative breast cancer, pancreatic cancer, metastatic cancer, liver cancer, colon cancer, lymphoma, esophageal cancer, and non-small cell lung cancer, or a combination thereof.

In a preferred technical solution of the present disclosure, the immunotherapy is selected from any one of immunotherapy in anti-tumor therapy or immunotherapy after radical surgery, or a combination thereof.

In a preferred technical solution of the present disclosure, the immunotherapy is selected from immunotherapy after radical surgery of primary hepatocellular carcinoma.

In a preferred technical solution of the present disclosure, the medicament is used for an adult patient or a pediatric patient.

Another purpose of the present disclosure is to provide an application of the specific T cell of the present disclosure in immunotherapy.

In a preferred technical solution of the present disclosure, an administration method of the immunotherapy is any one of intravenous injection, subcutaneous injection, intratumoral injection, intraperitoneal injection, intramuscular injection, and intradermal injection, or a combination thereof.

Another purpose of the present disclosure is to provide an application of the specific T cell of the present disclosure in combination with any one of radiotherapy, chemotherapy, targeted therapy, surgical therapy, or immunotherapy for anti-tumor or tumor immunotherapy.

Another purpose of the present disclosure is to provide an application of the specific T cell in preparation of a medicament for enhancing an antiviral capacity.

Another purpose of the present disclosure is to provide an application of the specific T cell in preparation of a medicament for enhancing treatment of an autoimmune disease.

In a preferred technical solution of the present disclosure, the autoimmune disease is selected from any one of systemic lupus erythematosus, rheumatoid arthritis, scleroderma, hyperthyroidism, juvenile diabetes, primary platelet purpura, autoimmune hemolytic anemia, ulcerative colitis, and a skin disease, or a complication thereof.

Unless otherwise specified, when the present disclosure relates to a percentage between liquids, the percentage is a volume/volume percentage; when the present disclosure relates to a percentage between a liquid and a solid, the percentage is a volume/weight percentage; when the present disclosure relates to a percentage between a solid and a liquid, the percentage is a weight/volume percentage; and the rest is a weight/weight percentage.

Compared with the prior art, the present disclosure has the following beneficial technical effects.
1. The nanoparticle or the microparticle prepared by the present disclosure is loaded with the tumor antigen component in the microparticle and/or the nanoparticle, which may activate a broad-spectrum cancer-specific T cell, and the cancer-specific T cell is sorted by utilizing the characteristics of the cancer-specific T cell after being activated, expanded, and then reinfused into patients for cancer treatment or prevention of recurrence or metastasis. The obtained cancer-specific T cell has a broad spectrum and high specificity, and may prevent or treat cancer by killing cancer cells after expansion.
2. The antigen component and the nanoparticle are obtained by screening through a specific method, and the T cells obtained by scientifically screening, sorting and expanding conditions have high viability, good cell stability, and strong immune cell targeting and anti-tumor activity. After being used for the treatment of cancer patients, it may prevent tumor recurrence, prolong a tumor growth rate, prevent cancer metastasis, and prolong survival time.
3. The method of the present disclosure has the advantages of simple operation, controllable quality, suitability for industrial production, etc.

### BRIEF DESCRIPTION OF THE DRAWINGS

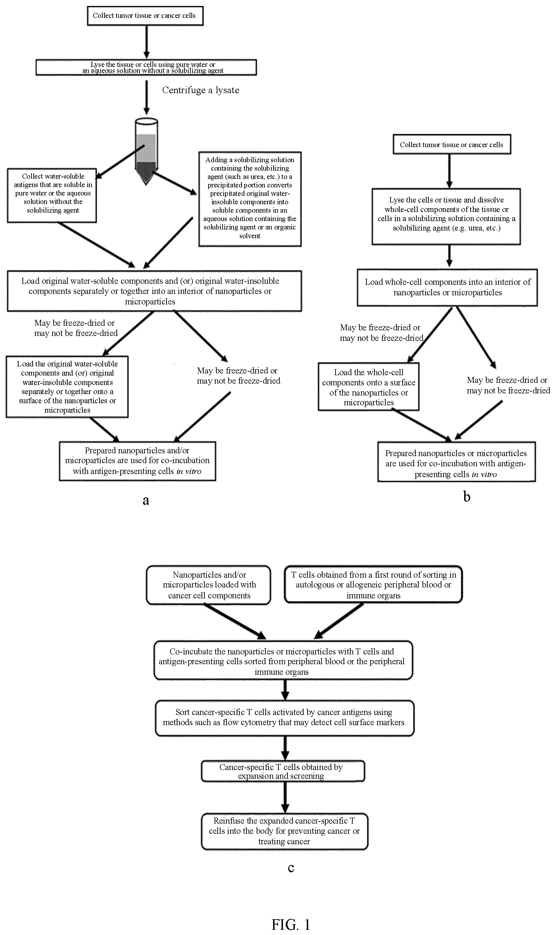

FIG. 1 is a schematic diagram of a representative preparation process and an application field of a T cell system of the present disclosure, which may also be other similar or improved alternative preparation processes in practical application, where a is a schematic diagram of separately collecting water-soluble components and water-insoluble components and preparing nanoparticles or microparticles; b is a schematic diagram of dissolving whole-cell components using a solubilizing solution containing a solubilizing agent and preparing nanoparticles or microparticles; and c is a schematic diagram of using the above particles prepared in a or b to assist in sorting peripheral cancer-specific T cells, and then expanding the T cells, and using the cells to prevent or treat cancer; and

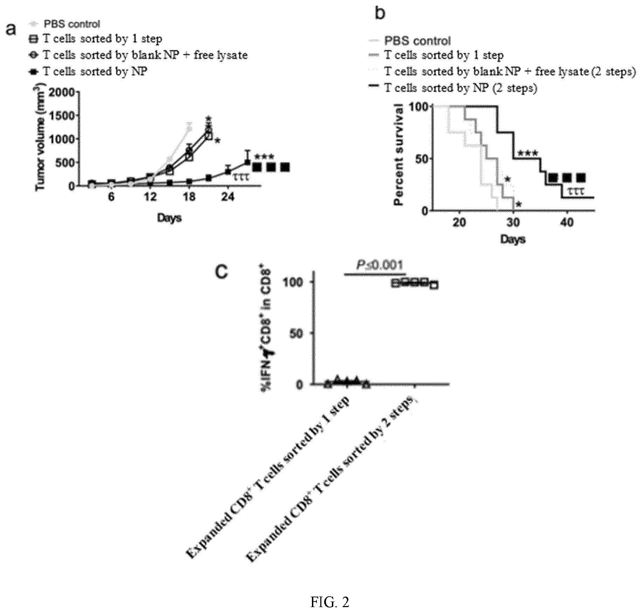

FIGS. 2 to 30 show experimental results of tumor growth rates and survival time in mice when cancer is prevented or treated with isolated and expanded cancer-specific T cells, or results of monitoring the number of cancer metastases when cancer metastasis is prevented, or results of a proportion of cancer-specific T cells to T cells according to Examples 1 to 29 of the present disclosure, where a or b is a diagram of experimental results of tumor growth rates and mouse survival time when preventing or treating cancer (n ≥ 8); and c shows results of experiments comparing specific T cell subsets using flow cytometry analysis (n ≥ 8). Each data point in FIG. a is mean ± standard error (mean ± SEM); and significant differences in tumor growth inhibition experiments in a were analyzed by the ANOVA method, and significant differences in b were analyzed by the Kaplan-Meier and log-rank test.

Unless otherwise specified, in the above figures, **★★★** indicates p < 0.005 compared with the PBS blank control group, with a significant difference; ****** indicates p < 0.001 compared with the PBS blank control group, with a significant difference; **indicates p < 0.001 compared with the PBS blank control group, with a significant difference; τττ represents p < 0.005 compared with the cancer-specific T cell group sorted in one step without nanoparticle stimulation, with a significant difference; ■■■ indicates p < 0.005 compared with the cell control group obtained by assisted sorting of blank nanoparticles containing immune adjuvants + free lysate, with a significant difference; λ represents p < 0.05 compared with the cancer-specific T cell group sorted without adding IL-7 during co-incubation, with a significant difference; *ε* represents p < 0.05 compared with the cancer-specific T cell group sorted and expanded by co-incubation with only one antigen-presenting cell, with a significant difference; *εε* represents p < 0.01 compared with the cancer-specific T cell group sorted and expanded by co-incubation with only one antigen-presenting cell, with a significant difference; *η* represents p < 0.05 compared with the cancer-specific T cell group sorted and expanded with nanoparticles and/or microparticles loaded with one adjuvant, with a significant difference; $ represents p < 0.05 compared with the cancer-specific T cell group obtained by assisted sorting of peptide nanoparticles or microparticles, with a significant difference; $$ represents p < 0.01 compared with the cancer-specific T cell group obtained by assisted sorting of peptide nanoparticles or microparticles, with a significant difference; δ represents p < 0.05 compared with the cancer-specific T cell group obtained by nanoparticle-assisted sorting without adjuvants, with a significant difference; σ represents p < 0.05 compared with the CD8⁺ cancer-specific T cell group obtained by Nanoparticle 1-assisted sorting, with a significant difference; and Δ represents p < 0.05 compared with the CD8⁺ cancer-specific T cell + CD4⁺ cancer-specific T cell group obtained by Nanoparticle 2-assisted sorting, with a significant difference. # represents p < 0.05, with a significant difference; ## represents p < 0.01, with a significant difference; and ### represents p < 0.005, with a significant difference. ns represents no significant difference.

### DESCRIPTION OF THE EMBODIMENTS

In the following embodiments, an antigen component is first prepared, and the antigen component may be (1) a whole-cell component of a cancer cell; (2) or a portion of a whole-cell component containing whole-cell protein and peptide components in a whole-cell component of a cancer cell; (3) or protein and peptide components plus an mRNA component in a whole-cell component of a cancer cell/tumor tissue.

A method for preparing the whole-cell component is: (1) first, lysing the cancer cell/the tumor tissue; then, preparing a water-soluble component and a water-insoluble component separately; and then, solubilizing the water-insoluble component using a specific solubilizing agent containing the solubilizing solution for use; or (2) lysing the cell using the solubilizing solution containing the solubilizing agent, and then solubilizing a lysed whole-cell component using the solubilizing solution containing the solubilizing agent.

A method for preparing the portion of the whole-cell component containing the whole-cell protein and peptide components in the whole-cell component of the cancer cell is: (1) first, lysing the cancer cell/the tumor tissue; then, preparing the water-soluble component and the water-insoluble component separately; then, dissolving the water-insoluble component using the specific dissolving agent containing the dissolving solution for use; then, isolating and extracting the protein and peptide components in the water-soluble component from the water-soluble component using an appropriate method; and then, using the protein and peptide components isolated and extracted from the water-soluble component together with all the water-insoluble component as the antigen component; (2) first, lysing the cancer cell/the tumor tissue; then, preparing the water-soluble component and the water-insoluble component separately; then, dissolving the water-insoluble component using the specific dissolving agent containing the dissolving solution for use; then, isolating and extracting the protein and peptide components in the water-soluble component from the water-insoluble component using an appropriate method; and then, using the protein and peptide components isolated and extracted from the water-insoluble component together with all the water-soluble component as the antigen component; (3) first, lysing the cancer cell/the tumor tissue; then, preparing the water-soluble component and the water-insoluble component separately; then, dissolving the water-insoluble component using the specific dissolving agent containing the dissolving solution; then, isolating and extracting the protein and peptide components in the water-soluble component from the water-soluble component and the water-insoluble component using an appropriate method, respectively; and then, using the protein and peptide components isolated and extracted from the water-soluble component and the water-insoluble component together as the antigen component; or (4) lysing the cell using a dissolving solution containing a dissolving agent; then, dissolving a lysed whole-cell component using the dissolving solution containing the dissolving agent; and then, isolating and extracting protein and peptide components therein using an appropriate method. In the above preparation method, a step of isolating and extracting whole-cell mRNA may be added, and the whole-cell mRNA may be used as a portion of the antigen component.

The appropriate method for isolating and extracting the above protein and peptide components includes but is not limited to salting-out, heating, enzymatic hydrolysis, etc.

The protein and peptide components are isolated and extracted, and then re-dissolved in the dissolving solution containing the dissolving agent.

A method for preparing the protein and peptide components plus the mRNA component in the whole-cell component of the cancer cell/the tumor tissue is: (1) first, lysing the cancer cell/the tumor tissue; then, preparing a water-soluble component and a water-insoluble component separately; and then, dissolving the water-insoluble component using a specific dissolving agent containing a dissolving solution for use; then, isolating and extracting the protein and peptide components and the mRNA component in the water-soluble component and/or the water-insoluble component separately; and then, mixing the protein and peptide components and the mRNA component and then using the mixture as the antigen component.

The above dissolving agent is selected from one or more of a compound with a structure of structural formula 1, a deoxycholate, a lauryl sulfate, glycerol, a protein-degrading enzyme, albumin, lecithin, a peptide, an amino acid, a glycoside, and a choline,

Where structural formula 1 is as follows: the structure of structural formula 1 is as follows:

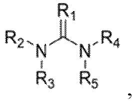

R₁ is C, N, S or O, and R₂-R₅ are independently selected from at least one of hydrogen, alkyl, amino, carboxyl, and substituted or unsubstituted guanidino.

The compound with the structure of structural formula 1 includes but is not limited to metformin hydrochloride, metformin sulfate, metformin sulfonate, a metformin salt, metformin, polyhexamethyleneguanidine hydrochloride, agmatine sulfate, methylguanidine hydrochloride, tetramethylguanidine hydrochloride, urea, guanidine hydrochloride, guanidine sulfate, guanidine sulfonate, a guanidine salt, other compounds containing guanidino or urea, guanidine carbonate, arginine, guanidinoacetic acid, guanidinophosphoric acid, guanidine sulfamate, guanidinosuccinic acid, semicarbazide hydrochloride, carbamoylurea, acetylurea, a sulfonylurea compound (glibenclamide, gliclazide, gliquidone, glimepiride, etc.), a thiourea compound (thiouracil, imidazole, etc.), nitrosoureas, and other compounds with the structure of structural formula 1.

The antigen component is then loaded onto a nanoparticle or a microparticle. The cell component-loaded nanoparticle or microparticle may be prepared by any preparation method that loads the antigen component onto the nanoparticle and/or the microparticle, including but not limited to any one of methods such as solvent evaporation, dialysis, microfluidic method, extrusion, hot melt, etc.

The antigen component may be loaded into an interior of the nanoparticle and/or the microparticle, or onto a surface of the nanoparticle and/or the microparticle, or simultaneously into an interior and onto a surface of the nanoparticle and/or the microparticle.

In an embodiment of the present disclosure, the solvent evaporation method is used to illustrate the specific implementation, and any other feasible preparation method may also be used in practical application.

A method for preparing the nanoparticle or microparticle includes the following steps:
(1) adding a first predetermined volume of an aqueous phase solution containing a first predetermined concentration to a second predetermined volume of an organic phase containing a second predetermined concentration of a medical polymer material;
(2) subjecting the mixed solution obtained in step 1 to ultrasonic treatment for more than 2 seconds or stirring or homogenization treatment or microfluidic treatment for more than 1 minute;
(3) adding the mixture obtained after the treatment of step 2 to a third predetermined volume of an aqueous solution containing a third predetermined concentration of an emulsifier and performing ultrasonic treatment for more than 2 seconds or stirring for more than 1 minute or performing homogenization treatment or microfluidic treatment;
(4) adding the liquid obtained after the treatment in step 3 to a fourth predetermined volume of an aqueous emulsifier solution having a fourth predetermined concentration, and stirring until a predetermined stirring condition is satisfied.
(5) centrifuging the mixed solution that satisfies the predetermined stirring condition in step 4 at a rotational speed greater than 100 RPM for more than 1 minute, then removing the supernatant, and resuspending the remaining precipitate in a fifth predetermined volume of a fifth predetermined concentration of an aqueous solution containing a freeze-drying protective agent or in a sixth predetermined volume of PBS (or normal saline).
(6) freeze-drying the suspension containing the freeze-drying protective agent obtained in step 5, and leaving the freeze-dried substance for later use.
(7) directly using the sixth predetermined volume of the nanoparticle-containing suspension resuspended in PBS (or normal saline) obtained in step 5 or the freeze-dried substance containing nanoparticles or microparticles and the freeze-drying protective agent after freeze-drying obtained in step 6 by resuspension using the sixth predetermined volume of PBS (or normal saline); or mixing the above sample with a seventh predetermined volume of a water-soluble component or a solubilized original water-insoluble component for use.

In the method for preparing the nanoparticle or microparticle, the aqueous phase solution may contain each component in a cancer cell lysate and immune-enhancing adjuvants poly(I:C), BCG, manganese adjuvant, calcium adjuvant or CpG; and each component in the cancer cell lysate is a water-soluble component or an original water-insoluble component dissolved in urea or guanidine hydrochloride separately at the time of preparation. The aqueous phase solution contains a concentration of the water-soluble component derived from cancer cells or a concentration of the original water-insoluble component derived from cancer cells dissolved in urea or guanidine hydrochloride, i.e. a first predetermined concentration, which requires a protein and peptide concentration content greater than 1 ng/mL to be capable of being loaded with sufficient tumor antigens to activate the relevant immune response. A concentration of the immune-enhancing adjuvants in the initial aqueous phase is greater than 0.01 ng/mL.

The aqueous phase solution contains each component in a tumor tissue lysate and immune-enhancing adjuvants poly(I:C), BCG, manganese adjuvant, calcium adjuvant or CpG; and each component in the tumor tissue lysate is a water-soluble component or an original water-insoluble component dissolved in urea or guanidine hydrochloride separately at the time of preparation. The aqueous phase solution contains a concentration of the water-soluble component derived from tumor tissue or a concentration of the original water-insoluble component derived from tumor tissue dissolved in urea or guanidine hydrochloride, i.e. a first predetermined concentration, which requires a protein and peptide concentration content greater than 0.01 ng/mL to be capable of being loaded with sufficient tumor antigens to activate the relevant immune response. A concentration of the immune-enhancing adjuvants in the initial aqueous phase is greater than 0.01 ng/mL.

The medical polymer material is dissolved in an organic solvent to obtain a second predetermined volume of an organic phase containing a second predetermined concentration of the medical polymer material. In some examples, the medical polymer material is PLGA, and the organic solvent selected is dichloromethane. Additionally, in some embodiments, the second predetermined concentration of the medical polymer material ranges from 0.5 mg/mL to 5000 mg/mL, and preferably 100 mg/mL.

In practice, the second predetermined volume of the organic phase is set according to its ratio to the first predetermined volume of the aqueous phase, and in the present disclosure, the ratio of the first predetermined volume of the aqueous phase to the second predetermined volume of the organic phase ranges from 1:1.1 to 1:5000, and preferably 1:10. In the specific implementation process, the first predetermined volume, the second predetermined volume, and the ratio of the first predetermined volume to the second predetermined volume may be adjusted as needed to adjust the size of the prepared nanoparticles or microparticles.

Preferably, when the aqueous phase solution is a lysate component solution, where the concentration of proteins and peptides is greater than 1 ng/mL, and preferably 1 mg/mL to 100 mg/mL; and when the aqueous phase solution is a lysate component/immune adjuvant solution, where the concentration of proteins and peptides is greater than 1 ng/mL, and preferably 1 mg/mL to 100 mg/mL, and the concentration of the immune adjuvant is greater than 0.01 ng/mL, and preferably 0.01 mg/mL to 20 mg/mL. In the organic phase solution of the polymer material, the solvent is DMSO, acetonitrile, ethanol, chloroform, methanol, DMF, isopropanol, dichloromethane, propanol, ethyl acetate, etc., and preferably dichloromethane; and the concentration of the polymer material is 0.5 mg/mL to 5000 mg/mL, and preferably 100 mg/mL. The first emulsifier solution is preferably an aqueous solution of polyvinyl alcohol at a concentration of 10 mg/mL to 50 mg/mL, and preferably 20 mg/mL. The second emulsifier solution is preferably an aqueous solution of polyvinyl alcohol at a concentration of 1 mg/mL to 20 mg/mL, and preferably 5 mg/mL. The dispersion is PBS buffer solution or normal saline or pure water.

Step 2, subjecting the mixed solution obtained in step 1 to ultrasonic treatment for more than 2 seconds or stirring or homogenization treatment or microfluidic treatment for more than 1 minute. Preferably, when the stirring is mechanical stirring or magnetic stirring, the stirring speed is greater than 50 rpm and the stirring time is greater than 1 minute, for example, the stirring speed is 50 rpm to 1500 rpm and the stirring time is 0.1 hours to 24 hours; during the ultrasonic treatment, the ultrasonic power is greater than 5 W and the time is greater than 0.1 seconds, such as 2 seconds to 200 seconds; a high pressure/ultra-high pressure homogenizer or a high shear homogenizer is used for the homogenization treatment, the pressure is greater than 5 psi, such as 20 psi to 100 psi, when the high pressure/ultra-high pressure homogenizer is used, and the rotational speed is greater than 100 rpm, such as 1000 rpm to 5000 rpm, when the high shear homogenizer is used; and the microfluidic treatment is used with a flow rate greater than 0.01 mL/min, such as 0.1 mL/min to 100 mL/min. Ultrasonic, stirring, homogenization, or microfluidic treatment is used for nanocrystallization and/or micronization, the length of ultrasonic time, stirring speed, homogenization treatment pressure and time can control the size of the prepared microparticles and nanoparticles, and being too large and too small can cause changes in the particle size.

Step 3, adding the mixture obtained after the treatment of step 2 to a third predetermined volume of an aqueous solution containing a third predetermined concentration of an emulsifier and performing ultrasonic treatment for more than 2 seconds or stirring for more than 1 minute or performing homogenization treatment or microfluidic treatment. In this step, the mixture obtained in step 2 is added to the aqueous emulsifier solution to continue ultrasonic treatment or stirring for nanocrystallization or micronization. This step is to perform nanocrystallization or micronization, the size of the prepared nanoparticle or microparticle can be controlled by the length of ultrasonic time or the stirring speed and time, and being too long or too short will bring about changes in the particle size. Therefore, it is necessary to select an appropriate ultrasonic time. In the present disclosure, the ultrasonic time is greater than 0.1 seconds, such as 2 seconds to 200 seconds, the stirring speed is greater than 50 rpm, such as 50 rpm to 500 rpm, and the stirring time is greater than 1 minute, such as 60 seconds to 6000 seconds. Preferably, when the stirring is mechanical stirring or magnetic stirring, the stirring speed is greater than 50 rpm and the stirring time is greater than 1 minute, for example, the stirring speed is 50 rpm to 1500 rpm and the stirring time is 0.5 hours to 5 hours; during the ultrasonic treatment, the ultrasonic power is 50 W to 500 W and the time is greater than 0.1 seconds, such as 2 seconds to 200 seconds; a high pressure/ultra-high pressure homogenizer or a high shear homogenizer is used for the homogenization treatment, the pressure is greater than 20 psi, such as 20 psi to 100 psi, when the high pressure/ultra-high pressure homogenizer is used, and the rotational speed is greater than 1000 rpm, such as 1000 rpm to 5000 rpm, when the high shear homogenizer is used; and the microfluidic treatment is used with a flow rate greater than 0.01 mL/min, such as 0.1 mL/min to 100 mL/min. Ultrasonic, stirring, homogenization, or microfluidic treatment is used for nanocrystallization or micronization, the length of ultrasonic time, stirring speed, homogenization treatment pressure and time can control the size of the prepared microparticles or nanoparticles, and being too large and too small can cause changes in the particle size.

In the present disclosure, the aqueous emulsifier solution is an aqueous polyvinyl alcohol (PVA) solution, the third predetermined volume is 5 mL, and the third predetermined concentration is 20 mg/mL. The third predetermined volume is adjusted according to its ratio to the second predetermined volume. In the present disclosure, the ratio of the second predetermined volume and the third predetermined volume is set to range from 1:1.1 to 1:1000, and preferably 2:5. In the specific implementation process, the ratio of the second predetermined volume to the third predetermined volume may be adjusted in order to control the size of the nanoparticles or microparticles. Similarly, the ultrasonic time or stirring time, and the volume and concentration of the aqueous emulsifier solution in this step are all determined to obtain nanoparticles or microparticles with appropriate sizes.

Step 4, adding the liquid obtained after the treatment in step 3 to a fourth predetermined volume of an aqueous emulsifier solution having a fourth predetermined concentration, and stirring until a predetermined stirring condition is satisfied.

In this step, the aqueous emulsifier solution is still PVA.

The fourth predetermined concentration is 5 mg/mL, and the selection of the fourth predetermined concentration is based on obtaining nanoparticles or microparticles with appropriate sizes. The selection of the fourth predetermined volume is determined according to the ratio of the third predetermined volume to the fourth predetermined volume. In the present disclosure, the ratio of the third predetermined volume to the third predetermined volume ranges from 1:1.5 to 1:2000, and preferably 1:10. In the specific implementation process, the ratio of the third predetermined volume to the fourth predetermined volume may be adjusted to control the size of nanoparticles or microparticles.

In the present disclosure, the predetermined stirring condition of this step is until the volatilization of the organic solvent is completed, that is, the volatilization of dichloromethane in step 1 is completed.

Step 5, centrifuging the mixed solution that satisfies the predetermined stirring condition in step 4 at a rotational speed greater than 100 RPM for more than 1 minute, then removing the supernatant, and resuspending the remaining precipitate in a fifth predetermined volume of a fifth predetermined concentration of an aqueous solution containing a freeze-drying protective agent or in a sixth predetermined volume of PBS (or normal saline).

In some implementations of the present disclosure, when the precipitate obtained in step 5 is resuspended in the sixth predetermined volume of PBS (or normal saline), freeze-drying is not required, and subsequent experiments on adsorption of cancer cell lysates on the surface of nanoparticles or microparticles may be directly performed.

In some implementations of the present disclosure, when the precipitate obtained in step 5 is resuspended in an aqueous solution containing a freeze-drying protective agent, freeze-drying is required, followed by subsequent experiments on the adsorption of cancer cell lysates on the surface of nanoparticles or microparticles.

In the present disclosure, the freeze-drying protective agent selected is trehalose.

In the present disclosure, the fifth predetermined concentration of the freeze-drying protective agent in this step is 4% in percentage by mass, which is set so as not to affect the freeze-drying effect during subsequent freeze-drying.

Step 6, freeze-drying the suspension containing the freeze-drying protective agent obtained in step 5, and leaving the freeze-dried substance for later use.

Step 7, directly using the sixth predetermined volume of the nanoparticle-containing suspension resuspended in PBS (or normal saline) obtained in step 5 or the freeze-dried substance containing nanoparticles or microparticles and the freeze-drying protective agent after freeze-drying obtained in step 6 by resuspension using the sixth predetermined volume of PBS (or normal saline); or mixing the sample with a seventh predetermined volume of water-soluble antigens or dissolved original water-insoluble antigens for use.

In the present disclosure, the volume ratio of the sixth predetermined volume to the seventh predetermined volume is 1:10000 to 10000:1, preferably the volume ratio is 1:100 to 100:1, and most preferably the volume ratio is 1:30 to 30:1.

In some embodiments, when the volume of the resuspended nanoparticle suspension is 10 mL, the volume containing the water-soluble component or the solubilized original water-insoluble component in the cancer cell lysate or the tumor tissue lysate is 1 mL. In actual use, the volume and ratio of the two may be adjusted as needed.

In a preferred technical solution of the present disclosure, a method for preparing nanoparticles or microparticles by the double emulsion method includes the following steps:
(1) adding a first predetermined volume of an aqueous phase solution containing a first predetermined concentration to a second predetermined volume of an organic phase containing a second predetermined concentration of a medical polymer material.
(2) subjecting the mixed solution obtained in step 1 to ultrasonic treatment for more than 2 seconds or stirring or homogenization treatment or microfluidic treatment for more than 1 minute.
(3) adding the mixture obtained after the treatment of step 2 to a third predetermined volume of an aqueous solution containing a third predetermined concentration of an emulsifier and performing ultrasonic treatment for more than 2 seconds or stirring for more than 1 minute or performing homogenization treatment or microfluidic treatment.
(4) adding the liquid obtained after the treatment in step 3 to a fourth predetermined volume of an aqueous emulsifier solution having a fourth predetermined concentration, and stirring until a predetermined stirring condition is satisfied, or the subsequent treatment may be performed directly without stirring.
(5) centrifuging the mixed solution that satisfies the predetermined stirring condition in step 4 at a rotational speed greater than 100 RPM for more than 1 minute, removing the supernatant, and resuspending the remaining precipitate in a fifth predetermined volume of a solution containing a fifth predetermined concentration of water-soluble and/or water-insoluble components in whole-cell components, or resuspending the remaining precipitate in a fifth predetermined volume of a solution containing a fifth predetermined concentration of water-soluble and/or water-insoluble components in whole-cell components mixed with an adjuvant.
(6) centrifuging the mixed solution that satisfies the predetermined stirring condition in step 5 at a rotational speed of more than 100 RPM for more than 1 minute, removing the supernatant, re-suspending the remaining precipitate in a sixth predetermined volume of a solidification treatment reagent or a mineralization treatment reagent for reaction for a certain period of time, then centrifuging and washing, and then adding a seventh predetermined volume of positively charged or negatively charged species for reaction for a certain period of time.
(7) drying the suspension containing the drying protective agent obtained in step 6, and leaving the dried substance for later use.
(8) directly using the eighth predetermined volume of the nanoparticle-containing suspension resuspended in PBS (or normal saline) obtained in step 6 or the dried substance containing nanoparticles or microparticles and the drying protective agent after drying obtained in step 7 by resuspension using the eighth predetermined volume of PBS (or normal saline); or mixing with a ninth predetermined volume of water-soluble components or water-insoluble components for use.

In some embodiments, the aqueous phase solution may contain each component in a cancer cell lysate and immune-enhancing adjuvant poly(I:C), manganese adjuvant, calcium adjuvant, BCG or CpG; and each component in the cancer cell lysate is a water-soluble component or an original water-insoluble component dissolved in urea or guanidine hydrochloride separately at the time of preparation. The aqueous phase solution contains a concentration of the water-soluble component derived from cancer cells or a concentration of the original water-insoluble component derived from cancer cells dissolved in urea or guanidine hydrochloride, i.e. a first predetermined concentration, which requires a protein and peptide concentration content greater than 0.01 ng/mL to be capable of being loaded with sufficient tumor antigens to activate the relevant immune response. A concentration of the immune-enhancing adjuvants in the initial aqueous phase is greater than 0.01 ng/mL.

In some embodiments, the aqueous phase solution contains each component in a tumor tissue lysate and immune enhancing adjuvants poly(I:C), manganese adjuvant, calcium adjuvant, BCG or CpG; and each component in the tumor tissue lysate is a water-soluble component or an original water-insoluble component dissolved in urea or guanidine hydrochloride separately at the time of preparation. The aqueous phase solution contains a concentration of the water-soluble component derived from tumor tissue or a concentration of the original water-insoluble component derived from tumor tissue dissolved in urea or guanidine hydrochloride, i.e. a first predetermined concentration, which requires a protein and peptide concentration content greater than 0.01 ng/mL to be capable of being loaded with sufficient tumor antigens to activate the relevant immune response. A concentration of the immune-enhancing adjuvants in the initial aqueous phase is greater than 0.01 ng/mL.

In the present disclosure, the medical polymer material is dissolved in an organic solvent to obtain a second predetermined volume of an organic phase containing a second predetermined concentration of the medical polymer material. In some embodiments, the medical polymer material is PLGA, and the organic solvent selected is dichloromethane. Additionally, in some embodiments, the second predetermined concentration of the medical polymer material ranges from 0.5 mg/mL to 5000 mg/mL, and preferably 100 mg/mL.

In the present disclosure, PLGA or modified PLGA is selected because the material is biodegradable and has been approved by the FDA for use as a drug dressing. Studies have shown that PLGA has a certain immunomodulatory function, so it is suitable as an excipient in the preparation of nanoparticles or microparticles.

In practice, the second predetermined volume of the organic phase is set according to its ratio to the first predetermined volume of the aqueous phase, and in the present disclosure, the ratio of the first predetermined volume of the aqueous phase to the second predetermined volume of the organic phase ranges from 1:1.1 to 1:5000, and preferably 1:10. In the specific implementation process, the first predetermined volume, the second predetermined volume, and the ratio of the first predetermined volume to the second predetermined volume may be adjusted as needed to adjust the size of the prepared nanoparticles or microparticles.

Preferably, when the aqueous phase solution is a lysate component solution, where the concentration of proteins and peptides is greater than 1 ng/mL, and preferably 1 mg/mL to 100 mg/mL; and when the aqueous phase solution is a lysate component/immune adjuvant solution, where the concentration of proteins and peptides is greater than 1 ng/mL, and preferably 1 mg/mL to 100 mg/mL, and the concentration of the immune adjuvant is greater than 0.01 ng/mL, and preferably 0.01 mg/mL to 20 mg/mL. In the organic phase solution of the polymer material, the solvent is DMSO, acetonitrile, ethanol, chloroform, methanol, DMF, isopropanol, dichloromethane, propanol, ethyl acetate, etc., and preferably dichloromethane; and the concentration of the polymer material is 0.5 mg/mL to 5000 mg/mL, and preferably 100 mg/mL. The first emulsifier solution is preferably an aqueous solution of polyvinyl alcohol at a concentration of 10 mg/mL to 50 mg/mL, and preferably 20 mg/mL. The second emulsifier solution is preferably an aqueous solution of polyvinyl alcohol at a concentration of 1 mg/mL to 20 mg/mL, and preferably 5 mg/mL. The dispersion is PBS buffer solution or normal saline or pure water.

Step 2, subjecting the mixed solution obtained in step 1 to ultrasonic treatment for more than 2 seconds or stirring or homogenization treatment or microfluidic treatment for more than 1 minute. Preferably, when the stirring is mechanical stirring or magnetic stirring, the stirring speed is greater than 50 rpm and the stirring time is greater than 1 minute, for example, the stirring speed is 50 rpm to 1500 rpm and the stirring time is 0.1 hours to 24 hours; during the ultrasonic treatment, the ultrasonic power is greater than 5 W and the time is greater than 0.1 seconds, such as 2 seconds to 200 seconds; a high pressure/ultra-high pressure homogenizer or a high shear homogenizer is used for the homogenization treatment, the pressure is greater than 5 psi, such as 20 psi to 100 psi, when the high pressure/ultra-high pressure homogenizer is used, and the rotational speed is greater than 100 rpm, such as 1000 rpm to 5000 rpm, when the high shear homogenizer is used; and the microfluidic treatment is used with a flow rate greater than 0.01 mL/min, such as 0.1 mL/min to 100 mL/min. Ultrasonic, stirring, homogenization, or microfluidic treatment is used for nanocrystallization and/or micronization, the length of ultrasonic time, stirring speed, homogenization treatment pressure and time can control the size of the prepared microparticles and nanoparticles, and being too large and too small can cause changes in the particle size.

Step 3, adding the mixture obtained after the treatment of step 2 to a third predetermined volume of an aqueous solution containing a third predetermined concentration of an emulsifier and performing ultrasonic treatment for more than 2 seconds or stirring for more than 1 minute or performing homogenization treatment or microfluidic treatment. In this step, the mixture obtained in step 2 is added to the aqueous emulsifier solution to continue ultrasonic treatment or stirring for nanocrystallization or micronization. This step is to perform nanocrystallization or micronization, the size of the prepared nanoparticle or microparticle can be controlled by the length of ultrasonic time or the stirring speed and time, and being too long or too short will bring about changes in the particle size. Therefore, it is necessary to select an appropriate ultrasonic time. In the present disclosure, the ultrasonic time is greater than 0.1 seconds, such as 2 seconds to 200 seconds, the stirring speed is greater than 50 rpm, such as 50 rpm to 500 rpm, and the stirring time is greater than 1 minute, such as 60 seconds to 6000 seconds. Preferably, when the stirring is mechanical stirring or magnetic stirring, the stirring speed is greater than 50 rpm and the stirring time is greater than 1 minute, for example, the stirring speed is 50 rpm to 1500 rpm and the stirring time is 0.5 hours to 5 hours; during the ultrasonic treatment, the ultrasonic power is 50 W to 500 W and the time is greater than 0.1 seconds, such as 2 seconds to 200 seconds; a high pressure/ultra-high pressure homogenizer or a high shear homogenizer is used for the homogenization treatment, the pressure is greater than 20 psi, such as 20 psi to 100 psi, when the high pressure/ultra-high pressure homogenizer is used, and the rotational speed is greater than 1000 rpm, such as 1000 rpm to 5000 rpm, when the high shear homogenizer is used; and the microfluidic treatment is used with a flow rate greater than 0.01 mL/min, such as 0.1 mL/min to 100 mL/min. Ultrasonic, stirring, homogenization, or microfluidic treatment is used for nanocrystallization or micronization, the length of ultrasonic time, stirring speed, homogenization treatment pressure and time can control the size of the prepared microparticles or nanoparticles, and being too large and too small can cause changes in the particle size.

In the present disclosure, the aqueous emulsifier solution is an aqueous polyvinyl alcohol (PVA) solution, the third predetermined volume is 5 mL, and the third predetermined concentration is 20 mg/mL. The third predetermined volume is adjusted according to its ratio to the second predetermined volume. In the present disclosure, the ratio of the second predetermined volume and the third predetermined volume is set to range from 1:1.1 to 1:1000, and preferably 2:5. In the specific implementation process, the ratio of the second predetermined volume to the third predetermined volume may be adjusted in order to control the size of the nanoparticles or microparticles. Similarly, the ultrasonic time or stirring time, and the volume and concentration of the aqueous emulsifier solution in this step are all determined to obtain nanoparticles or microparticles with appropriate sizes.

Step 4, adding the liquid obtained after the treatment in step 3 to a fourth predetermined volume of an aqueous emulsifier solution having a fourth predetermined concentration, and stirring until a predetermined stirring condition is satisfied, or the subsequent treatment may be performed directly without stirring.

In this step, the aqueous emulsifier solution is still PVA.

The fourth predetermined concentration is 5 mg/mL, and the selection of the fourth predetermined concentration is based on obtaining nanoparticles or microparticles with appropriate sizes The selection of the fourth predetermined volume is determined according to the ratio of the third predetermined volume to the fourth predetermined volume. In the present disclosure, the ratio of the third predetermined volume to the third predetermined volume ranges from 1:1.5 to 1:2000, and preferably 1:10. In the specific implementation process, the ratio of the third predetermined volume to the fourth predetermined volume may be adjusted to control the size of nanoparticles or microparticles.

In the present disclosure, the predetermined stirring condition of this step is that the volatilization of the organic solvent is completed, that is, the volatilization of dichloromethane in step 1 is completed. Subsequent tests are also performed without stirring.

Step 5, centrifuging the mixed solution that satisfies the predetermined stirring condition in step 4 at a rotational speed greater than 100 RPM for more than 1 minute, removing the supernatant, and resuspending the remaining precipitate in a fifth predetermined volume of a solution containing a fifth predetermined concentration of water-soluble and/or water-insoluble components in whole-cell components, or resuspending the remaining precipitate in a fifth predetermined volume of a solution containing a fifth predetermined concentration of water-soluble and/or water-insoluble components in whole-cell components mixed with an adjuvant.

Step 6, centrifuging the mixed solution that satisfies the predetermined stirring condition in step 5 at a rotational speed of more than 100 RPM for more than 1 minute, removing the supernatant, re-suspending the remaining precipitate in a sixth predetermined volume of a solidification treatment reagent or a mineralization treatment reagent for reaction for a certain period of time, then centrifuging and washing, and then adding a seventh predetermined volume of positively charged or negatively charged species for reaction for a certain period of time.

In some implementations of the present disclosure, after the precipitate obtained in step 6 may be re-suspended in a seventh predetermined volume of charged species, freeze-drying is not required, and subsequent experiments on loading of cancer cell/tissue lysates onto the surface of nanoparticles or microparticles may be directly performed.

In some implementations of the present disclosure, the precipitate obtained in step 6 is re-suspended in an aqueous solution containing a drying protective agent, then subjected to vacuum drying at room temperature or freeze vacuum drying, and then subsequent experiments on adsorption of cancer cell lysates on the surface of nanoparticles or microparticles are performed after drying.

In the present disclosure, the freeze-drying protective agent selected is trehalose or a mixed solution of mannitol and sucrose. In the present disclosure, the concentration of the drying protective agent in this step is 4% in percentage by mass, which is set so as not to affect the drying effect during subsequent drying.

Step 7, drying the suspension containing the drying protective agent obtained in step 6, and leaving the dried substance for later use.

Step 8, directly using the eighth predetermined volume of the nanoparticle-containing suspension resuspended in PBS (or normal saline) obtained in step 6 or the dried substance containing nanoparticles or microparticles and the drying protective agent after drying obtained in step 7 by resuspension using the eighth predetermined volume of PBS (or normal saline); or mixing with a ninth predetermined volume of water-soluble components or water-insoluble components for use.

In the present disclosure, the modification and antigen-loading steps of steps 5-8 may be repeated multiple times to increase the antigen-loading capacity. Moreover, when adding positively charged or negatively charged species, species with the same charges may be added multiple times or species with different charges may be added alternately.

In some embodiments, when the volume of the resuspended nanoparticle suspension is 10 mL, the volume containing water-soluble components or original water-insoluble components in the cancer cell lysate or the tumor tissue lysate is 0.1 mL to 100 mL. In actual use, the volume and ratio of the two may be adjusted as needed.

In the present disclosure, water-soluble components or original water-insoluble components in the cancer cell lysate or the tumor tissue lysate adopted contain poly(I:C), a manganese adjuvant, Bacillus Calmette-Guerin (BCG) or CpG, and the concentration of the poly(I:C), calcium adjuvant, BCG or CpG is greater than 0.01 ng/mL.

Furthermore, when the nanoparticles or microparticles of the present disclosure are prepared, the nanoparticles and/or microparticles loaded with only water-soluble components and the nanoparticles and/or microparticles loaded with only water-insoluble components may be used simultaneously, the nanoparticles and/or microparticles loaded with only water-soluble components may be used, the nanoparticles and/or microparticles loaded with only water-insoluble components may be used, or the nanoparticles and/or microparticles loaded with both water-soluble components and water-insoluble components may be used simultaneously when activating cancer-specific T cells *in vitro.*

After preparing nanoparticles or microparticles loaded with tumor antigens by any one method, the nanoparticles or microparticles are simultaneously co-incubated with antigen-presenting cells and T cells to activate cancer-specific T cells. Alternatively, nanoparticles and/or microparticles loaded with tumor antigen components are first co-incubated with antigen-presenting cells to activate the antigen-presenting cells, and then the activated antigen-presenting cells are then co-incubated with T cells alone to activate cancer-specific T cells.

Before T cells are co-incubated with nanoparticles and/or microparticles and antigen-presenting cells, the T cells may be cultured separately at rest for a period of time or appropriately sorted; or before T cells are co-incubated with the activated antigen-presenting cells, the T cells may be cultured separately at rest for a period of time or appropriately sorted.

After nanoparticles and/or microparticles loaded with tumor antigen components are first co-incubated with antigen-presenting cells to activate the antigen-presenting cells, the antigen-presenting cells may be co-incubated with T cells to activate specific T cells without special treatment, or the antigen-presenting cells may be treated with fixation, irradiation, irradiation, modification, inactivation, mineralization, etc., and then co-incubated with T cells to activate specific T cells.

After cancer-specific T cells are activated, the activated cancer-specific T cells are sorted from the incubated cells using a cell isolation method such as flow cytometry or a magnetic bead sorting method, and then the cancer-specific T cells are expanded *in vitro* for a certain period of time, and the obtained expanded cancer-specific T cells are used for preventing or treating cancer.

During sorting of the activated cancer-specific T cells, one activation marker for T cell activation may be used, or a combination of more than one different marker may be used as an activation marker.

Molecules that may be used as surface markers include but are not limited to: any one of CD69, CD137, CD25, CD134, CD80, CD86, OX40L, OX40, CD28, FAS-L, IL-2R, HLA- DR, CD127 (IL-7R), CD150, CD107A, CD83, CD166, CD39, CD178, CD212, CD229, CD100, CD107b, CD108, CD109, CD113, CD122, CD126, CD253, CD197, PD-1, TIM3, LAG-3, TIGIT, CD62L, CD70, CTLA-4 (CD152), CD27, CD26, CD30, TNFRSF9, CD74, PD-L1 (CD274), CD258, CD261, 4-1BB, CD154, ICAM-1, LFA-1, LFA-2, VLA-4, CD160, CD71, CXCR3, TNFRSF14, TNFRSF18, TNFSF4, TNFSF9, TNFSF14, CD11a, CD101, CD48, CD244, CD49a, CD95, CD44, CXCR 1, CD103, CD45RO, ICOS (CD278), VTCN1, HLA2, LGAL59, CCR7, CD357, BCL6, TCF-1, CD38, and CD27, or any combination thereof.

The present disclosure will be further described below in conjunction with the accompanying drawings and specific embodiments in order to enable those skilled in the art to better understand and implement the present disclosure, but the embodiments provided are not intended to limit the present disclosure.

### Example 1: Isolated and expanded T cells for melanoma prevention

This example used mouse melanoma as a cancer model to illustrate how nanoparticles were used to isolate and expand peripheral cancer-specific T cells for cancer prevention. In this example, B16F10 melanoma tumor tissue was lysed to prepare water-soluble components and water-insoluble components of the tumor tissue, then nanoparticles loaded with water-soluble components and water-insoluble components of the tumor tissue were prepared by a solvent evaporation method using organic polymer material PLGA as a nanoparticle skeleton material and polyinosinic-polycytidylic acid (poly(I:C)) as an immune adjuvant, then the nanoparticles were used to assist in isolating cancer-specific T cells in organs, and the isolated cancer-specific T cells were expanded and injected into the body to prevent melanoma. In this example, immune cells in peripheral splenocytes of the mice were used, and peripheral blood or peripheral lymph node cells can be directly used in practical application.

### (1) Preparation of antigen components

Each C57BL/6 mouse was subcutaneously inoculated with 1.5×10⁵ B16F10 cells on the back, and the mice were sacrificed and the tumor tissue removed when the tumors each grew to a volume of approximately 1000 mm³. The tumor tissue was cut into pieces and then ground, and passed through a cell strainer to prepare a tumor tissue single-cell suspension (containing cancer cells). Then an appropriate amount of pure water was added to the tumor tissue single-cell suspension, and freezing and thawing were repeated 5 times, accompanied by ultrasound to destroy and lyse the cells. After the cells were lysed, the lysate was centrifuged at a rotational speed of 5000 g for 5 minutes and the supernatant was taken as water-soluble components; and adding an 8 M aqueous urea solution (containing 500 mM sodium chloride) to the obtained precipitated portion to dissolve the precipitated portion can convert the water-insoluble components insoluble in pure water into soluble components in the 8 M aqueous urea solution. The above were the antigen components for preparing the nanoparticles.

### (2) Preparation of nanoparticles

In this example, the nanoparticles were prepared by a double emulsion method in the solvent evaporation method. At the time of preparation, the nanoparticles loaded with the water-soluble components in whole-cell components and the nanoparticles loaded with the water-insoluble components in the whole-cell components were prepared separately and then used together when used. The molecular weight of the nanoparticle preparation material PLGA adopted was 24 KDa to 38 KDa, and the immune adjuvant adopted was poly(I:C). The preparation method was as previously described. In the preparation process, the antigen components and the adjuvant were first loaded into the interior of the nanoparticles by the double emulsion method, and then 100 mg of the nanoparticles were centrifuged at 10000 g for 20 minutes, resuspended with 10 mL of ultrapure water containing 4% trehalose and freeze-dried for 48 h. The average particle size of the nanoparticles was about 280 nm, and the surface potential of the nanoparticles was about -3 mV; and approximately 100 µg of protein or peptide components were loaded per 1 mg of PLGA nanoparticles, and 0.02 mg of poly(I:C) was loaded per 1 mg of PLGA nanoparticles. The preparation materials and preparation method of the blank nanoparticles were the same, and the particle size was about 260 nm. During the preparation of the blank nanoparticles, pure water or 8 M urea containing an equivalent amount of poly(I:C) was adopted to replace the corresponding water-soluble components and water-insoluble components, respectively.

### (3) Sorting and expansion of cancer-specific T cells

Each C57BL/6 mouse was subcutaneously inoculated with 0.5×10⁵ B16F10 cells on the back, and the mice were sacrificed and the mouse spleen was harvested when the tumors grew to about 1000 mm³. A single-cell suspension of mouse splenocytes was prepared, and erythrocyte lysis treatment was performed to remove red blood cells in the single-cell suspension. First, the above cells were co-incubated with a T cell magnetic bead sorting reagent (CD3⁺ magnetic bead sorting reagent, CD8⁺ magnetic bead sorting reagent) or a B cell magnetic bead sorting reagent in sequence, and then CD3⁺ CD8⁺ T cells and CD19⁺ B cells were sorted from the mouse splenocytes using a magnetic bead sorter.

In the one-step sorting control group, the above sorted CD3⁺ CD8⁺ T cells (10000 cells, cell viability 60%) were co-incubated with IL-2 (500 U/mL), IL-7 (200 U/mL), IL-15 (200 U/mL), and αCD3/αCD28 (10 ng/mL) for 12 days in 10 mL of RPMI 1640 complete medium at 37°C (5% CO₂) (medium containing the above cytokines and antibodies was used for medium change every three days) to expand the obtained CD8⁺ T cells (cell viability 60%).

In the two-step sorting method, 1 million CD8⁺ T cells and 10 million B cells obtained by sorting were co-incubated with nanoparticles loaded with whole component antigens of the tumor tissue (250 µg of nanoparticles loaded with water-soluble components + 250 µg of nanoparticles loaded with water-insoluble components) or blank nanoparticles (500 µg) + equivalent quantity of a free lysate in 10 mL of DMEM high glucose complete medium for 96 hours, then the incubated cells were co-incubated sequentially with a CD3⁺ T cell magnetic bead sorting reagent, a CD8⁺ T cell magnetic bead sorting reagent, and a CD69 magnetic bead sorting reagent in a magnetic bead sorting method, and CD3⁺ CD8⁺ CD69⁺ T cells (cell viability 60%), i.e., the cancer-specific T cells activated by the tumor antigens, in the incubated T cells were sorted by the magnetic bead sorting method. The above sorted cancer-specific T cells (10000 cells) were co-incubated with IL-2 (500 U/mL), IL-7 (200 U/mL), IL-15 (200 U/mL), and αCD3/αCD28 (10 ng/mL) for 12 days in RPMI 1640 complete medium at 37°C (5% CO₂) (medium containing the above cytokines and antibodies was used for medium change every three days) to expand the sorted cancer-specific T cells (cell viability 60%).

The CD8⁺ T cells (500000 cells) expanded after the one-step sorting method or cancer-specific T cells (500000 cells) expanded after the two-step sorting method in the control group were co-incubated with B cells (2 million cells) and the nanoparticles loaded with the whole-cell components (30 µg nanoparticles loaded with the water-soluble components + 30 µg nanoparticles loaded with the water-insoluble components) for 48 hours in 3 mL DMEM high glucose complete medium. Then the incubated cells were collected, and the incubated cells were labeled sequentially with CD3 antibodies, CD8 antibodies and IFN-γ antibodies with different fluorescent probes. Then the proportion of CD8⁺ IFN-γ⁺ T cells to CD8⁺ T cells in the expanded cells after the one-step sorting and the two-step sorting was analyzed by flow cytometry. The cancer cell antigens loaded by the nanoparticles can be degraded into antigenic epitopes after being phagocytosed by antigen-presenting cells (B cells) and presented to the surface of the antigen-presenting cells. Specific T cells that can recognize cancer cell antigens can recognize cancer cell antigenic epitopes and then be activated and secrete killer cytokines. IFN-γ is the most important cytokine secreted by antigen-specific T cells that are activated after recognizing antigens. However, because it is a secretory cytokine, it is necessary to first add 4% paraformaldehyde for cell fixation, and then use a membrane rupture agent to rupture the membrane and then use antibodies for intracellular staining (the cells were dead cells after analysis), so that the cells fixed by paraformaldehyde are four cells. The CD8⁺ IFN-γ⁺ T cells (cell viability 0%) obtained by flow cytometry analysis were cancer-specific T cells that can specifically recognize cancer cell antigenic epitopes.

### (4) Cancer-specific T cells for cancer prevention

Female C57BL/6 mice aged 6-8 weeks were selected as model mice to prepare melanoma tumor-bearing mice. One day before adoptive transfer of cells, the recipient mice were intraperitoneally injected with a 100 mg/kg dose of cyclophosphamide to eliminate immune cells in the recipient mice. Then 2 million cancer-specific T cells prepared by the two-step sorting method or 2 million CD3⁺ T cells expanded after the one-step sorting method in step (3) were intravenously injected into the recipient mice. Each recipient mouse was subcutaneously inoculated with 1.5×10⁵ B16F10 cells on the lower right side of the back the next day. The tumor growth rate of the mice and the survival of the mice were monitored. In the experiment, the size of tumor volume in the mice was recorded every 3 days from day 3. The tumor volume was calculated using the formula v = 0.52×a×b², where v was the tumor volume, a was the tumor length, and b was the tumor width. Due to animal experimental ethics, in the mouse survival experiment, when the tumor volume of the mice exceeded 2000 mm³, the mice were regarded as dead and the mice were euthanized.

### (4) Experimental results

As shown in FIGS. 2a and 2b, the tumor growth rate of the mice was the fastest and the survival time of the mice was the shortest in the PBS control group. Compared with the PBS control group, the tumor growth rate and survival time of the mice in the groups treated by T cells obtained by the one-step sorting method and T cells obtained by blank nanoparticle-assisted sorting and expansion were improved. Compared with the above three groups, the tumor growth rate in the mice treated with cancer-specific T cells obtained after assisted sorting and expansion of the nanoparticles loaded with the whole-cell components of the cancer cells was the slowest, and the survival time of the mice was the longest, indicating that the sorted and expanded cell system according to the present disclosure had a good preventive effect on cancer.

As shown in FIG. 2c, almost 100% of the cancer-specific T cells obtained by the two-step sorting method can recognize cancer cell antigens and be activated by tumor antigens after co-incubation with antigen-presenting cells and the nanoparticles loaded with the whole-cell components of the cancer cells, while CD8⁺ IFN-γ⁺ T cells only accounted for less than 5% of CD8⁺ T cells in the T cells expanded after the one-step sorting method after co-incubation, which showed that the sorting method of the present disclosure can effectively enrich the cancer-specific T cells with a killing ability.

In this example, one surface marker was used as a marker for T cell activation, and in practical application, a combination of one or more different markers may be used as an activation marker.

### Example 2: Isolated and expanded peripheral cancer-specific T cells for melanoma prevention

In this example, B16F10 melanoma tumor tissue was lysed to prepare water-soluble components and water-insoluble components of the tumor tissue, then nanoparticles loaded with the water-soluble components and the water-insoluble components of the tumor tissue were prepared by a solvent evaporation method using an organic polymer material as a nanoparticle skeleton material, poly(I:C) and CpG1018 as immune adjuvants, and then the nanoparticles were used to sort and expand peripheral cancer-specific T cells. In this example, immune cells in peripheral splenocytes of the mice were used, and peripheral blood or peripheral lymph node cells can be used in practical application.

### (1) Preparation of antigen components

Each C57BL/6 mouse was subcutaneously inoculated with 1.5×10⁵ B16F10 cells on the back, and the mice were sacrificed and the tumor tissue removed when the tumors grew to approximately 1000 mm³. The tumor tissue was cut into pieces and then ground, and passed through a cell strainer to prepare a single-cell suspension, then an appropriate amount of pure water was added, and freezing and thawing was repeated 5 times, accompanied by ultrasound to destroy and lyse the cells. After the cells were lysed, the lysate was centrifuged at a rotational speed of 5000 g for 5 minutes, and the supernatant was taken as water-soluble components that were soluble in pure water; and adding 8 M urea to the obtained precipitated portion to dissolve the precipitated portion can convert the water-insoluble components that were insoluble in pure water into soluble components in the 8 M aqueous urea solution. The above were the antigen components for preparing the nanoparticles.

### (2) Preparation of nanoparticles

In this example, the nanoparticles and the blank nanoparticles as controls were prepared by the solvent evaporation method. At the time of preparation, the nanoparticles loaded with the water-soluble components in whole-cell components and the nanoparticles loaded with the water-insoluble components in the whole-cell components were prepared separately and then used together when used. The molecular weight of the nanoparticle preparation material PLGA adopted was 7 KDa to 17 KDa, and the immune adjuvants adopted were poly(I:C) and CpG1018. The preparation method was as previously described. In the preparation process, the antigen components and adjuvants were loaded into the interior of the nanoparticles by a double emulsion method, and then 100 mg of the nanoparticles were centrifuged at 10000 g for 20 minutes, and resuspended with 10 mL of ultrapure water containing 4% trehalose and freeze-dried for 48 h. The average particle size of the nanoparticles was about 280 nm, and the surface potential of the nanoparticles was about -3 mV; and approximately 100 µg of protein or peptide components were loaded, and 0.02 mg of poly(I:C) and CpG1018 each were loaded per 1 mg of PLGA nanoparticles. The preparation materials and preparation method of the blank nanoparticles were the same, the particle size was about 260 nm, and the blank nanoparticles were loaded with an equivalent amount of the adjuvants but not loaded with any antigen component.

### (3) Isolation and expansion of cancer-specific T cells

Each C57BL/6 mouse was subcutaneously inoculated with 1.5×10⁵ B16F10 cells on the back, and the mice were subcutaneously injected with 1 mg of PLGA nanoparticles loaded with water-soluble components and 1 mg of PLGA nanoparticles loaded with water-insoluble components on days 4, 7, 10, 15, 20, 25, and 30, separately. On day 34, the mice were sacrificed, the spleen of the mice was collected, and a single-cell suspension of mouse splenocytes was prepared. The above cells were co-incubated sequentially with a T magnetic bead sorting reagent and a B cell magnetic bead sorting reagent separately and sorted sequentially, and CD3⁺ T cells and CD19⁺ B cells were sorted sequentially from the mouse splenocytes using a magnetic bead sorter.

In the one-step sorting control group, the above sorted 1 million CD3⁺ T cells were co-incubated with IL-2 (500 U/mL), IL-7 (200 U/mL), IL-15 (200 U/mL), and αCD3/αCD28 (10 ng/mL each) for 14 days in 10 mL of RPMI 1640 complete medium at 37°C (5% CO₂) (medium containing the above cytokines and antibodies was used for medium change every two days) to expand the obtained T cells (cell viability 65%).

In the two-step sorting method, 5 million CD3⁺ T cells and 10 million B cells obtained by sorting were co-incubated with nanoparticles loaded with whole component antigens of the tumor tissue (250 µg of nanoparticles loaded with water-soluble components + 250 µg of nanoparticles loaded with water-insoluble components) or blank nanoparticles (500 µg) + equivalent quantity of free antigen components for 48 hours in 10 mL of RPMI 1640 complete medium, then the incubated cells were co-incubated with reagents in the magnetic bead sorting method, and CD3⁺ CD137⁺ T cells, i.e., cancer-specific T cells activated by tumor antigens (cell viability 65%), in the incubated T cells were sorted by flow cytometry. The above sorted cancer-specific T cells (1 million cells) were co-incubated with IL-2 (500 U/mL), IL-7 (200 U/mL), IL-15 (200 U/mL), and αCD3/αCD28 (10 ng/mL each) for 14 days in 10 mL of RPMI 1640 complete medium (37°C., 5% CO₂) (medium containing the above cytokines and antibodies was used for medium change every two days) to expand the sorted cancer-specific T cells (cell viability 65%).

The T cells expanded after the one-step sorting method (500000 cells) or the expanded cancer-specific T cells obtained after the two-step sorting method (500000 cells) were co-incubated with B cells (2 million cells) and nanoparticles loaded with whole-cell components (60 µg) for 48 hours in 3 mL RPMI 1640 complete medium. Then the incubated cells were collected, and the incubated cells were labeled sequentially with CD3 antibodies, CD8 antibodies and IFN-y antibodies with different fluorescent probes. Then the proportion of CD3⁺ IFN-γ⁺ T cells to CD3⁺ T cells in the expanded cells after the one-step sorting and the two-step sorting was analyzed by flow cytometry. The CD3⁺ IFN-γ⁺ T (cell viability 0%)cells obtained by flow cytometry analysis were cancer-specific T cells that can specifically recognize cancer cell antigens.

### (4) Cancer-specific T cells for cancer prevention

Female C57BL/6 mice aged 6-8 weeks were selected as model mice to prepare melanoma tumor-bearing mice. One day before adoptive transfer of cells, the recipient mice were intraperitoneally injected with a 100 mg/kg dose of cyclophosphamide to eliminate immune cells in the recipient mice. Then 2 million cancer-specific T cells obtained by the two-step sorting and expansion or 2 million T cells obtained by the one-step sorting and expansion in step (3) were intravenously injected into the recipient mice. Each recipient mouse was subcutaneously inoculated with 1.5×10⁵ B16F10 cells on the lower right side of the back the next day. The methods of monitoring tumor growth rates and survival time in the mice were the same as above.

### (4) Experimental results

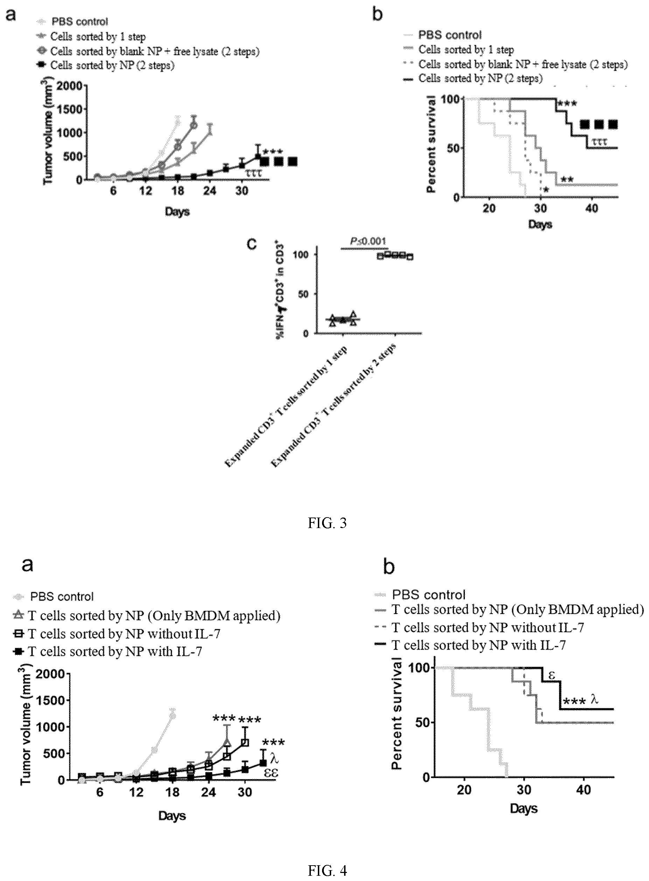

As shown in FIGS. 3a and 3b, the tumor growth rate of the mice was the fastest and the survival time of the mice was the shortest in the PBS control group. Compared with the PBS control group, the tumor growth rate and survival time of the mice in the groups treated by T cells obtained by the one-step sorting method and T cells obtained by blank nanoparticle-assisted sorting were improved. Compared with the above three groups, the tumor growth rate in the mice treated with cancer-specific T cells obtained after sorting and expansion of the nanoparticles loaded with whole-cell antigen components was the slowest, and the survival time of the mice was the longest, indicating that the sorted and expanded cell system according to the present disclosure had a good preventive effect on cancer.

As shown in FIG. 3c, almost 100% of the cancer-specific T cells obtained by the two-step sorting method can recognize cancer cell antigens and be activated by the cancer cell antigens after co-incubation with antigen-presenting cells and the nanoparticles loaded with the whole-cell components of the cancer cells, while CD3⁺ IFN-γ⁺ T cells only accounted for less than 5% of CD3⁺ T cells in the T cells expanded after the one-step sorting method after co-incubation, which showed that the sorting method of the present disclosure can effectively enrich the cancer-specific T cells with a killing ability.

### Example 3: Sorted and expanded cancer-specific T cells for melanoma treatment

In this example, B16F10 melanoma tumor tissue and cancer cells were first lysed to prepare a water-soluble component mixture (mass ratio of 1:1) and a water-insoluble component mixture (mass ratio of 1:1) of tumor tissue and cancer cells, and then a nanoparticle system loaded with the water-soluble component mixture and the water-insoluble component mixture was prepared with PLGA as a nanoparticle backbone material and Poly(I:C), CpG2006 and CpG2216 as adjuvants. Then the nanoparticles were then co-incubated with T cells and antigen-presenting cells *in vitro* to activate pre-existing cancer-specific T cells. After being activated, the cancer-specific T cells highly expressed specific molecules, and were sorted by flow cytometry and then expanded to treat cancer.

### (1) Preparation of antigen components

When tumor tissue was collected, each C57BL/6 mouse was first subcutaneously inoculated with 1.5×10⁵ B16F10 cells on the back. The mice were sacrificed and the tumor tissue was removed when the tumors grew to a volume of about 1000 mm³. The tumor tissue was cut into pieces and ground, passed through the cell strainer and then added with an appropriate amount of pure water, and freezing and thawing was repeated 5 times, accompanied by ultrasound to destroy and lyse the obtained samples. When the cultured B16F10 cancer cell line was collected, the medium was first removed by centrifugation, then the cancer cells were washed twice with PBS, and the cancer cells were collected by centrifugation. The cancer cells were resuspended in ultrapure water, and freezing and thawing was repeated 3 times, accompanied by ultrasound to destroy and lyse the cancer cells. After the tumor tissue or cancer cell line was lysed, the lysate was centrifuged at a rotational speed of 5000 g for 5 minutes, and the supernatant was taken to obtain water-soluble components that were soluble in pure water; and adding 8 M urea to the obtained precipitated portion to dissolve the precipitated portion can convert the water-insoluble components that were insoluble in pure water into soluble components in the 8 M aqueous urea solution. The water-soluble components of the tumor tissue and the water-soluble components of the cancer cell line were mixed at a mass ratio of 1:1; and the water-insoluble components of the tumor tissue and the water-insoluble components of the cancer cell line were mixed at a mass ratio of 1:1. The above were the antigen components for preparing the nanoparticles.

### (2) Preparation of nanoparticles

The nanoparticles in this example were prepared by a double emulsion method. The molecular weight of the nanoparticle preparation material PLGA was 7 KDa to 17 KDa, and the immune adjuvants adopted were poly(I:C), CpG2006 and CpG2216. The preparation method was as previously described. The antigen components and adjuvants were first loaded into the interior of the nanoparticles, and then 100 mg nanoparticles were centrifuged at 12000 g for 25 minutes, resuspended with 10 mL of ultrapure water containing 4% trehalose and freeze-dried for 48 h; before use, the nanoparticles were resuspended in 9 mL of PBS, then 1 mL of lysate components (protein concentration of 80 mg/mL) was added for reaction at room temperature for 10 min to obtain nanoparticles loaded with the antigen components both inside and outside. The average particle size of the nanoparticles was about 280 nm, and the surface potential of the nanoparticles was about -5 mV; and approximately 130 µg of protein or peptide components were loaded per 1 mg of PLGA nanoparticles, and 0.02 mg of poly(I:C), CpG2006 and CpG2216 immune adjuvants each were loaded per 1 mg of PLGA nanoparticles. The preparation materials and preparation method of the blank nanoparticles were the same, the particle size was about 260 nm, and the blank nanoparticles were loaded with an equivalent amount of the adjuvants but not loaded with any cancer cell lysate component.

### (3) Isolation and expansion of cancer-specific T cells

Each C57BL/6 mouse was subcutaneously inoculated with 1.5×10⁵ B16F10 cells on the back, and the mice were subcutaneously injected with 2 mg of PLGA nanoparticles (loaded with the antigen components and the adjuvants) on days 4, 7, 10, 15, and 20, separately. The mice were sacrificed on day 24, mouse peripheral blood was collected, peripheral blood mononuclear cells (PBMCs) were isolated from mouse peripheral blood using gradient centrifugation, and PBMCs were co-incubated with CD3 antibodies and CD19 antibodies labeled with different fluorescent probes, and then sorted using flow cytometry to obtain CD3⁺ T cells and CD19⁺ B cells.

The preparation method of mouse bone marrow-derived macrophages (BMDMs) was a conventional preparation method. The preparation method of BMDMs was as follows: C57 mice were anesthetized and sacrificed by dislocation, the mice were sterilized with 75% ethanol, then a small cut was made on the back of the mice with scissors, the skin was directly torn by hand to the calf joint of the mice, and the foot joint and skin of the mice were removed. Scissors were used to remove the hind limb along the greater trochanter at the root of the mouse thigh, the muscle tissue was removed, and then the leg bone was placed in a culture dish containing 75% ethanol for soaking for 5 min, replaced with a new culture dish containing 75% ethanol and moved into a super-clean bench. The leg bone soaked in ethanol was soaked in cold PBS, and the ethanol on the surface of the tibia and femur was washed away. This process can be repeated 3 times. The cleaned femur and tibia were separated, and both ends of the femur and tibia were cut off with scissors separately. The bone marrow was blown out from the femur and tibia by sucking a cold induction medium with a 1 mL syringe, and the blowing was repeated 3 times until no obvious red color was seen in the leg bone. The culture medium containing bone marrow cells was repeatedly blown with a 5 mL pipette gun to disperse the cell masses, then the cells were sieved using a 70 µm cell strainer, transferred to a 15 mL centrifuge tube, and centrifuged at 1500 rpm/min for 5 minutes, the supernatant was discarded, an erythrocyte lysate was added, and the mixture was resuspended and allowed to stand for 5 min, and then centrifuged at 1500 rpm/min for 5 min. The supernatant was discarded, the mixture was resuspended with a prepared cold bone marrow macrophage induction medium (DMEM high glucose medium containing 15% L929 medium), and the cells were seeded. The cells were cultured overnight to remove other miscellaneous cells that adhere faster to the wall, such as fibroblasts. The non-adherent cells were collected and planted into dishes or cell culture plates according to the experimental design arrangement. Macrophage colony-stimulating factor (M-CSF) exerted a stimulating effect at a concentration of 40 ng/mL to differentiate bone marrow cells into mononuclear macrophages. After 8 days of culture, the morphological changes of macrophages were observed under a light microscope. After 8 days, the cells were digested and collected, and incubated with anti-mouse F4/80 antibodies and anti-mouse CD11b antibodies for 30 min at 4°C in the dark, and then the proportion of successfully induced macrophages was identified by flow cytometry.

The sorted 5 million CD3⁺ T cells, 5 million B cells, 5 million BMDMs, and nanoparticles (500 µg) loaded with whole component antigens of the tumor tissue were co-incubated for 48 hours in 10 mL of RPMI 1640 complete medium; or the sorted 1 million CD3⁺ T cells, 10 million mouse BMDMs and nanoparticles (500 µg) loaded with whole component antigens of the tumor tissue were co-incubated for 48 hours in 10 mL of RPMI 1640 complete medium; or the sorted 1 million CD3⁺ T cells, 5 million B cells, 5 million mouse BMDMs, IL-7 (10 ng/mL), and nanoparticles (500 µg) loaded with whole component antigens of the tumor tissue were co-incubated for 48 hours in 10 mL of RPMI 1640 complete medium. CD3⁺ CD137⁺ T cells, i.e. cancer-specific T cells activated by cancer cell antigens (cell viability 70%), were then sorted using flow cytometry. The sorted 1 million cancer-specific T cells were co-incubated with IL-2 (1000 U/mL) and IL-7 (1000 U/mL) for 14 days in 10 mL of RPMI 1640 complete medium (37°C, 5% CO₂) (the medium was changed every two days) to expand the sorted cancer-specific T cells.

### (4) Cancer-specific T cells for cancer treatment

Female C57BL/6 aged 6-8 weeks were selected as model mice to prepare melanoma-bearing mice. Each mouse was subcutaneously inoculated with 1.5×10⁵ B16F10 cells on the lower right side of the back on day 0. Two million cancer-specific T cells were intravenously injected on day 4, day 7, day 10, day 15, day 20, and day 25 separately after melanoma inoculation. The methods of monitoring tumor volumes and survival time in mice were the same as above.

### (5) Experimental results

As shown in FIG. 4, the mice in the PBS control group had the fastest tumor growth rate and the shortest survival time, and the tumor growth rate and survival time of the mice in the treatment group after expansion of several groups of the sorted T cells were significantly improved compared with those in the PBS control group. The anticancer effect of cancer-specific T cells obtained by the two-step sorting method using B cells and BMDMs as mixed antigen-presenting cells after expansion was better than the anticancer effect of cancer-specific T cells obtained by the two-step sorting method using BMDMs as antigen-presenting cells after expansion. Therefore, when cancer-specific T cells were sorted, the effect of using B cells and BMDMs as mixed antigen-presenting cells was better than that of BMDMs alone as antigen-presenting cells. Moreover, the cancer-specific T cells sorted by adding IL-7 in the process of co-incubation of the nanoparticles with antigen-presenting cells and T cells were better than the cancer-specific T cells sorted without adding IL-7 in the process of incubation.

### Example 4: Cell system for prevention of melanoma lung metastasis

In this example, mouse melanoma lung models were used to illustrate how to use a cell system to prevent cancer metastasis. In this example, B16F10 melanoma tumor tissue was first lysed to prepare water-soluble components and water-insoluble components of the tumor tissue; and then nanoparticles loaded with the water-soluble components and the water-insoluble components of the tumor tissue were prepared. In this example, one round of mineralization treatment was performed. In this example, the nanoparticles were used to activate dendritic cells (DCs) *in vitro,* and then the dendritic cells were co-incubated with cancer-specific T cells to activate and assist in sorting cancer-specific T cells. In practical use, the antigen-presenting cells may be viable or inactivated, for example, fixed with paraformaldehyde or inactivated with radiation.

### (1) Preparation of antigen components

Each C57BL/6 mouse was subcutaneously inoculated with 1.5×10⁵ B16-F10 cells on the back, and the mice were sacrificed and the tumor tissue removed when the tumors grew to approximately 1000 mm³. The tumor tissue was cut into pieces and then ground, and added with collagenase (1 mg/mL) to incubate for 30 min in RPMI 1640 complete medium. Then a single-cell suspension was prepared through a cell strainer, an appropriate amount of pure water was added, and freezing and thawing were repeated 5 times, accompanied by ultrasound to destroy and lyse the cells. After the cells were lysed, the lysate was centrifuged at a rotational speed of 5000 g for 5 minutes and the supernatant was taken as water-soluble components that were soluble in pure water; and adding 10% sodium deoxycholate (containing 0.8 M arginine) to the obtained precipitated portion to dissolve the precipitated portion can convert the water-insoluble components that were insoluble in pure water into soluble components in the 10% aqueous sodium deoxycholate solution, and the water-soluble components and the water-insoluble components were mixed at a mass ratio of 3:1 to obtain the antigen components.

### (2) Preparation of nanoparticles

In this example, the nanoparticles were prepared by a double emulsion method in a solvent evaporation method, and the double emulsion method was appropriately modified and improved. The molecular weight of the nanoparticle preparation material PLGA adopted was 24 KDa to 38 KDa, the immune adjuvant adopted was poly(I:C), and poly(I:C) was distributed in the interior of the nanoparticles and loaded onto the surface of the nanoparticles. The preparation method was as previously described. In the preparation process, the lysed components and the adjuvant were first loaded into the interior of the nanoparticles by the double emulsion method, then 100 mg of the nanoparticles were centrifuged at 10000 g for 20 minutes, then the nanoparticles were resuspended using 7 mL of PBS and mixed with 3 mL of a PBS solution containing the cell lysate (60 mg/mL), and then centrifuged at 10000 g for 20 minutes. Then the nanoparticles were resuspended with 10 mL of a silicate solution (containing 150 mM NaCl, 80 mM tetramethyl orthosilicate and 1.0 mM HCl, pH 3.0) and fixed at room temperature for 10 min, then fix at -80°C for 24 h, centrifuged and washed with ultrapure water and then resuspended with 3 mL of PBS containing protamine (5 mg/mL) and polylysine (10 mg/mL) for reaction for 10 min, then washed by centrifugation at 10000 g for 20 min, resuspended with 10 mL of a PBS solution containing the cell lysate (50 mg/mL) for 10 min, and then freeze-dried for 48 h after centrifugation at 10000 g for 20 min and resuspension with 10 mL of ultrapure water containing 4% trehalose; and before use, the particles were resuspended in 7 mL of PBS, and then added with 3 mL of adjuvant-containing cancer tissue lysate components (protein concentration of 50 mg/mL) for reaction at room temperature for 10 min to obtain nanoparticles loaded with the lysate both inside and outside modified by cryosilicification and addition of cationic species. The average particle size of the nanoparticles was about 350 nm, and the surface potential of the nanoparticles was about -3 mV; and approximately 300 µg of protein or peptide components were loaded per 1 mg of PLGA nanoparticles, and 0.02 mg of poly(I: C) immune adjuvant was loaded per 1 mg of PLGA nanoparticles, with half inside and half outside.

The steps of the preparation method of nanoparticles without modification treatment were essentially the same as those of the preparation of nanoparticles with modification treatment, except that the steps of low-temperature silicification and addition of charged species were not undergone. In the preparation process, the antigens were first loaded into the interior of the nanoparticles by the double emulsion method, and the nanoparticles were centrifuged at 10000 g for 20 min after the internal loading of the antigens (lysis components), and then freeze-dried for 48 h after resuspension using 10 mL of ultrapure water containing 4% trehalose. Before use, the particles were resuspended in 7 mL of PBS and then added with 3 mL of adjuvant-containing cancer tissue lysate components (protein concentration of 50 mg/mL) for reaction at room temperature for 10 min to obtain nanoparticles loaded with the lysate both inside and outside. The average particle size of the nanoparticles was about 320 nm, and the surface potential of the nanoparticles was about -5 mV; and approximately 150 µg of protein or peptide components were loaded per 1 mg of PLGA nanoparticles, and 0.02 mg of poly(I:C) was loaded per 1 mg of PLGA nanoparticles, with half inside and half outside.

The preparation materials and preparation method of the blank nanoparticles were the same, the particle size was about 300 nm, and the blank nanoparticles were loaded with an equivalent amount of the adjuvants but not loaded with any antigen component.

### (3) Preparation of dendritic cells

This example illustrated how to prepare bone marrow-derived dendritic cells (BMDCs) by taking the preparation of dendritic cells from mouse bone marrow cells as an example. First, one 6-8-week-old C57 mouse was sacrificed by cervical dislocation. The tibia and femur of the hind leg were surgically removed and put into PBS, and the muscle tissue around the bone was removed cleanly with scissors and tweezers. Both ends of the bone were cut off with scissors, then a PBS solution was extracted with a syringe, needles were inserted from both ends of the bone into the bone marrow cavity, and the bone marrow was repeatedly rinsed into a culture dish. The bone marrow solution was collected, centrifuged at 400 g for 3 min, and then added with 1 mL of an erythrocyte lysate to lyse red blood cells. 3 mL of RPMI 1640 (10% FBS) medium was added to terminate lysis, centrifugation was performed at 400 g for 3 min, and the supernatant was discarded. Cells were cultured in a 10 mm culture dish using RPMI 1640 (10% FBS) medium with the addition of recombinant mouse GM-CSF (20 ng/mL) at 37°C and 5% CO₂ for 7 days. On Day 3, the culture flask was gently shaken and the same volume of RPMI 1640 (10% FBS) medium containing GM-CSF (20 ng/mL) was replenished. On day 6, the medium was subjected to half-volume change treatment. On day 7, a small number of suspended and semi-adherent cells were collected. When the ratio of CD86⁺ CD80⁺ cells in CD11c⁺ cells was between 15-20% by flow cytometry, the induced cultured BMDC could be used for the next experiment.

### (4) Isolation and expansion of cancer-specific T cells

Each C57BL/6 mouse was subcutaneously inoculated with 1.5×10⁵ B16F10 on the back, and the mice were subcutaneously injected with 100 µL of 1 mg PLGA nanoparticles on day 4, day 7, day 10, day 15, day 20, and day 25, separately. The blank nanoparticles + free lysate control group was injected at the same dose at the corresponding time as the control. The mice were sacrificed on day 24, mouse spleens were collected, a single-cell suspension of mouse splenocytes was prepared, and CD3⁺ T cells in the single-cell suspension of splenocytes were sorted using flow cytometry after incubation of the mouse splenocytes with CD3 antibodies. The BMDCs prepared in step 3 (1 million cells) were mixed with the nanoparticles (40 µg) loaded with whole component antigens of the tumor tissue and then co-incubated for 48 hours in 3 mL RPMI 1640 complete medium (37 °C, 5% CO₂), and then centrifuged at 400 g for 5 minutes to collect the BMDCs. The BMDCs were co-incubated with the sorted CD3⁺ T cells for 24 hours. Then CD3⁺ CD8⁺ CD69⁺ T cells (cell viability 75%) and CD3⁺ CD4⁺ CD25⁺ T cells (cell viability 75%), i.e. cancer-specific T cells activated by the tumor antigens, in the incubated cells were sorted by flow cytometry. The above sorted 500000 cancer-specific T cells were co-incubated with IL-2 (200 U/mL), IL-7 (200 U/mL), IL-15 (200 U/mL), and αCD3/αCD28 for 14 days in 10 mL of RPMI 1640 complete medium (medium change every two days) to expand the sorted cancer-specific T cells (cell viability 75%).

### (5) Allogeneic cell system for prevention of cancer metastasis

Female C57BL/6 aged 6-8 weeks were selected as model mice to prepare melanoma-bearing mice. One day before adoptive transfer of cells, the recipient mice were intraperitoneally injected with a 100 mg/kg dose of cyclophosphamide to eliminate immune cells in the recipient mice. The mice were injected intravenously with 100 µL of 2 million cancer-specific T cells (1 million CD3⁺ CD8⁺ CD69⁺ T cells and 1 million CD3⁺ CD4⁺ CD25⁺ T cells) on day 0. At the same time, each mouse was intravenously injected with 0.5×10⁵ B16F10 cells on day 1, the mice were sacrificed on day 14, and the number of melanoma foci in the lungs of the mice was observed and recorded.

### (6) Experimental results

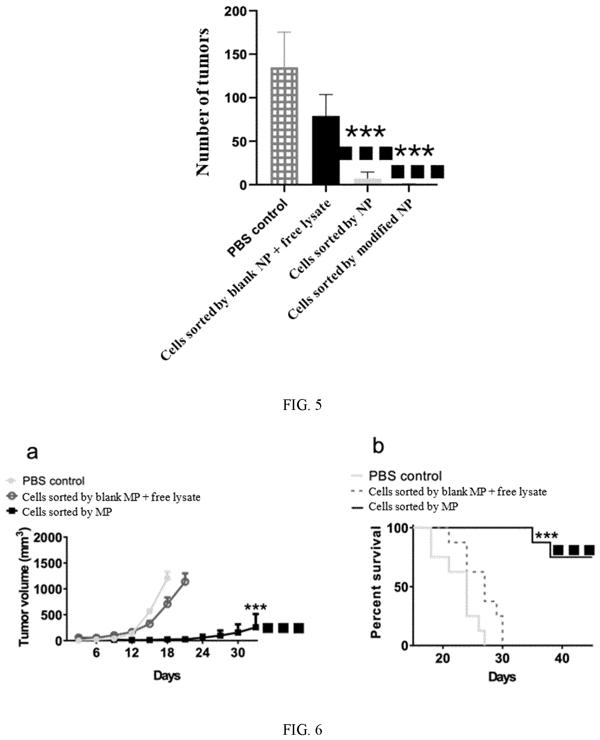

As shown in FIG. 5, the mice in the PBS control group had a large number of cancer foci, while the T cell-treated mice had a small number of cancer foci. Compared with the control group, the cancer foci of the mice treated with cancer-specific T cells obtained by assisted sorting and expansion of modified nanoparticles or unmodified nanoparticles were significantly reduced. This showed that T cells sorted and expanded with modified or unmodified nanoparticles can effectively prevent cancer metastasis.

### Example 5: Sorted and expanded cancer-specific T cells assisted by microparticles for cancer prevention

In this example, two rounds of silicification treatment were performed during the preparation of the microparticles. Cancer-specific T cells were sorted and expanded using antigen-loaded microparticles, and then injected into the mice to prevent cancer.

### (1) Preparation of antigen components

The cultured B16F10 melanoma cancer cell line was harvested and centrifuged at 350 g for 5 minutes, then the supernatant was discarded and washed twice with PBS, and then the cancer cells were resuspended and lysed and the lysed whole-cell components were dissolved with 6 M guanidine hydrochloride, which was the antigen components for preparing the microparticle system.

### (2) Preparation of microparticles

In this example, Microparticle 1 was prepared by a double emulsion method, and the double emulsion method was appropriately modified and improved. The molecular weight of the microparticle preparation material PLGA adopted was 38 KDa to 54 KDa, the immune adjuvant adopted was CpG, and adopted was distributed in the interior of the microparticles and loaded onto the surface of microparticles. The preparation method was as previously described. In the preparation process, the whole-cell antigens were first loaded inside the microparticles by the double emulsion method, after the lysis components were loaded inside, 100 mg of the microparticles were centrifuged at 10000 g for 15 minutes, then the microparticles were resuspended using 7 mL of PBS and mixed with 3 mL of PBS solution containing a cell lysate (50 mg/mL), and then centrifuged at 10000 g for 20 minutes. The microparticles were then resuspended with 10 mL of a silicate solution (containing 120 mM NaCl, 100 mM tetramethyl orthosilicate and 1.0 mM HCl, pH 3.0) and fixed at room temperature for 12 h, centrifuged and washed with ultrapure water, then resuspended with 3 mL of PBS containing polyaspartic acid (10 mg/mL) for 10 min, then centrifuged at 10000 g for 15 min, resuspended with 10 mL of PBS containing the cell lysate (50 mg/mL) for reaction for 10 min, and then centrifuged at 10000 g for 20 min. Then 10 mL of a silicate solution (containing 150 mM NaCl, 80 mM tetramethyl orthosilicate and 1.0 mM HCl, pH 3.0) was used, and the microparticles were fixed at room temperature for 12 h, centrifuged and washed with ultrapure water and then resuspended with 3 mL of PBS containing histone (5 mg/mL) and polyarginine (10 mg/mL) for reaction for 10 min, then washed by centrifugation at 10000 g for 15 min, resuspended with 10 mL of PBS solution containing the cell lysate (50 mg/mL) for 10 min, then centrifuged at 10000 g for 15 min, and resuspended with 10 mL of ultrapure water containing 4% trehalose and then freeze-dried for 48 h; before use, the particles were resuspended in 7 mL of PBS and then added with 3 mL of adjuvant-containing cancer cell lysate components (protein concentration of 50 mg/mL) for reaction at room temperature for 10 min to obtain microparticles loaded with the lysate both inside and outside modified by two rounds of cryosilicification and addition of cationic and anionic species. The average particle size of the microparticles was about 1.1 µm, and the surface potential of the microparticles was about -2 mV; and approximately 340 µg of protein or peptide components were loaded per 1 mg of PLGA microparticles, and 0.02 mg of CpG was loaded per 1 mg of PLGA microparticles, with half inside and half outside.

The preparation materials and preparation method of blank Microparticle 2 were the same, the particle size was about 1.1 µm, the surface potential was about -3 mV, and the blank microparticles were loaded with an equivalent quantity of the adjuvant but not loaded with antigen components.

### (3) Preparation of dendritic cells

This example illustrated how to prepare bone marrow-derived dendritic cells (BMDCs) by taking the preparation of dendritic cells from mouse bone marrow cells as an example. First, one 6-8-week-old C57 mouse was sacrificed by cervical dislocation. The tibia and femur of the hind leg were surgically removed and put into PBS, and the muscle tissue around the bone was removed cleanly with scissors and tweezers. Both ends of the bone were cut off with scissors, then a PBS solution was extracted with a syringe, needles were inserted from both ends of the bone into the bone marrow cavity, and the bone marrow was repeatedly rinsed into a culture dish. The bone marrow solution was collected, centrifuged at 400 g for 3 min, and then added with 1 mL of an erythrocyte lysate to lyse red blood cells. 3 mL of RPMI 1640 (10% FBS) medium was added to terminate lysis, centrifugation was performed at 400 g for 3 min, and the supernatant was discarded. Cells were cultured in a 10 mm culture dish using RPMI 1640 (10% FBS) medium with the addition of recombinant mouse GM-CSF (20 ng/mL) at 37°C and 5% CO₂ for 7 days. On Day 3, the culture flask was gently shaken and the same volume of RPMI 1640 (10% FBS) medium containing GM-CSF (20 ng/mL) was replenished. On day 6, the medium was subjected to half-volume change treatment. On day 7, a small number of suspended and semi-adherent cells were collected. When the ratio of CD86⁺ CD80⁺ cells in CD11c⁺ cells was between 15-20% by flow cytometry, the induced cultured BMDC could be used for the next experiment.

### (4) Isolation and expansion of cancer-specific T cells

Each C57BL/6 mouse was subcutaneously inoculated with 1.5×10⁵ B16F10 on the back, and the mice were subcutaneously injected with 100 µL of 1 mg PLGA microparticles on day 4, day 7, day 10, day 15, day 20, and day 25, separately. The mice were sacrificed on day 30, and the spleens of the mice were collected to prepare a single-cell suspension of mouse splenocytes. The CD3⁺ T cells in the single-cell suspension of splenocytes were first sorted using magnetic bead sorting. The sorted T cells (20 million cells), the BMDCs prepared in step 3 (20 million cells) were mixed with 20 µg Microparticle 1 (or blank Microparticle 2 + equivalent quantity of a free lysate) and then incubated for 24 hours in 10 mL RPMI complete medium (containing 5% FSBS), and then CD3⁺ CD69⁺ T cells were sorted by magnetic bead sorting method, i.e., cancer-specific T cells activated by the tumor antigens (cell viability 75%). The sorted 1 million cancer-specific T cells were co-incubated with IL-2 (2000 U/mL) and αCD3/αCD28 antibody (20 ng/mL) for 7 days in 10 mL of DMEM high glucose complete medium (37°C, 5% CO₂) (medium exchange every two days) to expand the sorted cancer-specific T cells (cell viability 75%).

### (5) Expanded cancer-specific T cells for cancer prevention

Female C57BL/6 aged 6-8 weeks were selected as model mice to prepare melanoma-bearing mice. One day before adoptive transfer of cells, the recipient mice were intraperitoneally injected with a 100 mg/kg dose of cyclophosphamide to eliminate immune cells in the recipient mice. The mice were intravenously injected with 100 µL of 2 million cancer-specific T cells on day 0. At the same time, each mouse was subcutaneously inoculated with 1.5×10⁵ B16F10 cells on day 0. The methods of monitoring tumor volumes and survival time in mice were the same as above.

### (6) Experimental results

As shown in FIG. 6, the tumor growth rate of the mice in the PBS control group was fast and the survival time was short, while the tumor growth rate of the T cell-treated mice was significantly slower and the survival time was significantly prolonged, showing that the T cells sorted and expanded by the microparticles can effectively prevent cancer.

### Example 6 Cancer-specific T cells for cancer prevention

In this example, B16F10 melanoma tumor tissue was first lysed, and the lysate components of the tumor tissue were dissolved using 8 M urea. Then, nanoparticles loaded with whole-cell antigens were prepared with PLA as a nanoparticle backbone material, and Poly(I:C) and CpG1018 as immune adjuvants, and the nanoparticles were used to assist in sorting and expansion of cancer-specific T cells.

### (1) Preparation of antigen components

Each C57BL/6 mouse was subcutaneously inoculated with 1.5×10⁵ B16F10 cells on the back, and the mice were sacrificed and the tumor tissue removed when the tumors grew to approximately 1000 mm³. The tumor tissue was cut into pieces and then ground, and after passing through a cell strainer, an appropriate amount of an 8 M aqueous urea solution was added to a single-cell suspension to lyse the cells and dissolve the cell lysate. The above were the antigen components for preparing the nanoparticles.

### (2) Preparation of nanoparticles

Nanoparticle 1 in this example was prepared by a solvent evaporation method. The molecular weight of the Nanoparticle 1 preparation material PLA was 20 KDa, and the immune adjuvants adopted were poly(I:C) and CpG1018. The preparation method was as previously described. The antigen components and adjuvants were first loaded inside the nanoparticles by a double emulsion method, and then 100 mg of the nanoparticles were centrifuged at 12000 g for 20 minutes, and resuspended with 10 mL of ultrapure water containing 4% trehalose and freeze-dried for 48 h to obtain a freeze-dried powder for later use. The average particle size of the Nanoparticle 1 was about 250 nm, and the surface potential was about -3 mV; and approximately 110 µg of protein or peptide components were loaded, and 0.02 mg of poly(I:C) and CpG1018 each were loaded per 1 mg of PLGA nanoparticles.

The preparation materials and preparation method of Nanoparticle 2 were the same as above, the particle size was about 250nm, and the surface potential was about -3 mV; and approximately 110 µg of protein or peptide components were loaded, and 0.04 mg of poly(I:C) was loaded per 1 mg of PLGA nanoparticles.

### (3) Isolation and expansion of cancer-specific T cells

Each C57BL/6 mouse was subcutaneously inoculated with 1.5×10⁵ B16F10 on the back, and the mice were subcutaneously injected with 100 µL of 1 mg PLA Nanoparticle 1 on day 4, day 7, day 10, day 15, day 20, and day 25, separately. The mice were sacrificed on day 29, mouse spleens were collected and a single-cell suspension of mouse splenocytes was prepared. CD3⁺ T cells in the mouse splenocytes were sorted using magnetic bead sorting. Then, the sorted T cells (10 million cells) were co-incubated with allogeneically derived B cells (15 million cells) and 40 mg of nanoparticles (Nanoparticle 1 or 2) for 48 hours in 20 mL of RPMI 1640 complete medium (37°C, 5% CO₂). Then the incubated cells were labeled with CD3 antibodies and CD69 antibodies labeled with different fluorescent probes, and then the CD3⁺ CD69⁺ T cells, i.e., cancer-specific T cells activated by the cancer cell antigens, in the incubated cells were sorted by flow cytometry (cell viability 75%). The sorted 1 million cancer-specific T cells were co-incubated with IL-2 (1000 U/mL) and αCD3/αCD28 antibody (20 ng/mL) for 11 days in 10 mL of high glucose DMEM complete medium (medium change every two days) to expand the sorted cancer-specific T cells (cell viability 75%).

### (4) T cells for cancer prevention

Female C57BL/6 aged 6-8 weeks were selected as model mice to prepare melanoma-bearing mice. One day before transplantation of mouse cancer-specific T cells, the recipient mice were intraperitoneally injected with a 100 mg/kg dose of cyclophosphamide to eliminate immune cells in the recipient mice. The mice were injected subcutaneously with 100 µL of 2 million expanded cancer-specific T cells on day 0. At the same time, each mouse was subcutaneously inoculated with 1.510⁵ B16F10 cells on day 0, and the methods of monitoring tumor volumes and survival time of the mice were the same as above.

### (5) Experimental results

As shown in FIG. 6, the control mice had the fastest tumor growth rate and the fastest survival time. The cancer-specific T cells sorted and expanded using Nanoparticle 1 and Nanoparticle 2 can slow down the tumor growth rate and prolong the survival time of the mice.

### Example 7 Sorted and expanded cancer-specific T cells for colon cancer treatment

Colon cancer tumor tissue and lung cancer cell lines were first lysed to prepare a water-soluble component mixture (mass ratio 1:1) and a water-insoluble component mixture (mass ratio 1:1), and the water-soluble component mixture and the water-insoluble component mixture were mixed at a mass ratio of 1:1. Then, organic polymer material PLGA was used as a nanoparticle backbone material, CpG1018 and Poly(I:C) were used as immune adjuvants to prepare nanoparticles, and the nanoparticles were used to sort and expand cancer-specific T cells for the treatment of colon cancer.

### (1) Preparation of antigen components

Each C57BL/6 mouse was subcutaneously inoculated with 2×10⁶ MC38 cells on the back and the mice were sacrificed and the tumor tissue removed when the tumors grew to approximately 1000 mm³. The tumor tissue was cut into pieces and then ground, and passed through a cell strainer to prepare a single-cell suspension, then an appropriate amount of pure water was added, and freezing and thawing was repeated 5 times, accompanied by ultrasound to destroy and lyse the cells. After the cells were lysed, the lysate was centrifuged at a rotational speed greater than 5000 g for 5 minutes and the supernatant was taken as water-soluble components that were soluble in pure water; and adding a 10% aqueous solution of octyl glucoside to the obtained precipitated portion to dissolve the precipitated portion can convert water-insoluble components that were insoluble in pure water into soluble components in the 10% aqueous solution of octyl glucoside. The cultured LLC lung cancer cell lines were harvested and centrifuged at 350 g for 5 minutes, then the supernatant was discarded and washed twice with PBS, then the cells were resuspended with ultrapure water, and freezing and thawing were repeated 5 times, accompanied by ultrasound to destroy and lyse the cells. After the cells were lysed, the lysate was centrifuged at a rotational speed of 3000 g for 6 minutes and the supernatant was taken as water-soluble components that were soluble in pure water; and adding a 10% aqueous solution of octyl glucoside to the obtained precipitated portion to dissolve the precipitated portion can convert water-insoluble components that were insoluble in pure water into soluble components in the 10% aqueous solution of octyl glucoside.

The water-soluble components from the colon cancer tumor tissue and lung cancer cells were mixed at a mass ratio of 1:1; and the water-insoluble components dissolved in 10% octyl glucoside were also mixed at a mass ratio of 1:1. Then the water-soluble component mixture and the water-insoluble component mixture were mixed at a mass ratio of 1:1, and the mixture was the antigen components for preparing the nanoparticles.

### (2) Preparation of nanoparticles

The nanoparticles in this example were prepared by a double emulsion method. The molecular weight of the nanoparticle preparation material PLGA was 24 KDa to 38 KDa, and the immune adjuvants adopted were Poly(I: C) and CpG1018, and the antigen components and the adjuvants were simultaneously distributed in the interior and on the surface of the nanoparticles. The preparation method was as previously described. In the preparation process, the antigen components and adjuvants were loaded into the interior of the nanoparticles by a double emulsion method, and then 100 mg of the nanoparticles were centrifuged at 10000 g for 20 minutes, and resuspended with 10 mL of ultrapure water containing 4% trehalose and freeze-dried for 48 h. 20 mg of the nanoparticles were resuspended in 0.9 mL of PBS before use, and mixed and incubated with 0.1 mL of a sample containing the antigen components (80 mg/mL) and the adjuvants for 5 minutes at room temperature for use. The average particle size of the nanoparticles was about 280 nm, and the surface potential was about -3 mV; and approximately 100 µg of protein or peptide components were loaded, and 0.02 mg of CpG1018 and Poly(I:C) immune adjuvants each were loaded per 1 mg of PLGA nanoparticles.

The preparation materials and preparation method of Nanoparticle 2 were the same, the particle size was about 280 nm, and the surface potential was -3 mV; and approximately 100 µg of protein or peptide components were loaded, and 0.04 mg of CpG1018 was loaded per 1 mg of PLGA Nanoparticle 2.

### (3) Sorting and expansion of cancer-specific T cells

Each C57BL/6 mouse was subcutaneously inoculated with 1.5×10⁵ B16F10 on the back, and the mice were subcutaneously injected with 100 µL of 2 mg PLGA nanoparticles on day 4, day 7, day 10, day 15, day 20, and day 25, separately. The mice were sacrificed on day 30, the spleens of each group of the mice were collected separately, a single-cell suspension of mouse splenocytes was prepared, and CD3⁺ CD8⁺ T cells and CD19⁺ B cells were sorted therefrom using flow cytometry. 3 million CD8⁺ T cells and 6 million CD19⁺ B cells were co-incubated with nanoparticles (50 µg) for 96 hours in 2 mL RPMI complete medium (37°C, 5% CO₂), and then CD3⁺ CD8⁺ CD69⁺ T cells (cell viability 70%), i.e., cancer-specific T cells activated by cancer cell antigen, in the incubated cells were sorted by flow cytometry. The sorted 200000 cancer-specific T cells were co-incubated with IL-2 (2000 U/mL), IL-7 (500 U/mL), IL-15 (500 U/mL), and αCD3/αCD28 antibodies for 7 days in 10 mL of high glucose DMEM complete medium (37°C, 5% CO₂) (medium change every two days) to expand the sorted cancer-specific T cells (cell viability 70%).

### (4) Cancer-specific T cells for cancer treatment

Female C57BL/6 aged 6-8 weeks were selected as model mice to prepare colon cancer tumor-bearing mice. Each mouse was subcutaneously inoculated with 2×10⁶ MC38 cells on day 0, and mice were injected with 100 µL containing 100000 cancer-specific T cells on day 4, day 7, day 10, day 15, and day 20, separately. The methods of monitoring tumor growth and survival in mice were the same as above.

### (5) Experimental results

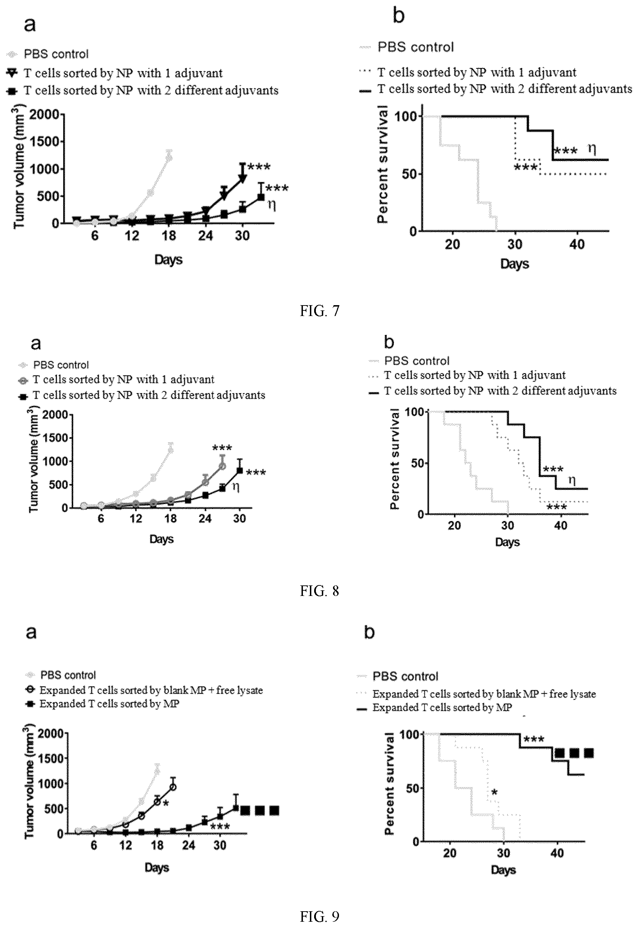

As shown in FIG. 7, the tumor growth rate of PBS control mice was very fast, and the cancer-specific T cells sorted and expanded by Nanoparticle 1 and Nanoparticle 2 can effectively delay the tumor growth rate and improve the survival time of mice.

### Example 8: Sorted and expanded T cells for breast cancer prevention

### (1) Preparation of antigen components

The cultured 4T1 cells were centrifuged at 400 g for 5 minutes, and then washed twice with PBS and resuspended in ultrapure water. The obtained cancer cells were inactivated and denatured by ultraviolet rays and high-temperature heating, respectively, and then the breast cancer cells were lysed with an appropriate amount of 8 M urea and the lysate was dissolved, which was the antigen components for preparing particles.

### (2) Preparation of microparticles

In this example, the microparticles were prepared by a double emulsion method. The molecular weight of the Microparticle 1 skeleton material PLGA was 38 KDa to 54 KDa, the immune adjuvants adopted were CpG and Poly ICLC, and the substance that increased lysosome immune escape was arginine. During preparation, the microparticles internally loaded with the antigen components, adjuvants and arginine were prepared by the double emulsion method, and then 100 mg of the microparticles were centrifuged at 9000 g for 20 minutes, and resuspended using 10 mL of ultrapure water containing 4% trehalose and then dried for 48 h for later use. The average particle size of the microparticle system was about 2.1 µm, and the surface potential of the microparticles was about -5 mV; and approximately 110 µg of protein or peptide components were loaded, and 0.01 mg of CpG and Poly ICLC each were loaded, and 0.05 mg of arginine was loaded per 1 mg of PLGA microparticles. The preparation materials and preparation method of blank Microparticle 2 were the same, the particle size was about 2.0 µm, and the blank microparticles were loaded with an equivalent amount of CpG, Poly ICLC and arginine but not loaded with any antigen component.

### (3) Isolation and expansion of cancer-specific T cells

Female BALB/c mice aged 6-8 weeks were selected and subcutaneously injected with 100 µL of Microparticle 1 containing 2 mg PLGA on day 0, day 4, day 7, day 14, day 21, and day 28, separately. The mice were sacrificed on day 32, the peripheral blood of the mice was collected, and then peripheral blood mononuclear cells (PBMCs) were isolated from the peripheral blood. CD3⁺ CD8⁺ T cells and B220⁺ B cells were first sorted from PBMCs by flow cytometry in the first step, and the sorted 200000 CD8⁺ T cells, 300000 B cells and IL-7 (10 ng/mL) were co-incubated with 40 µg microparticles (Microparticle 1 or Microparticle 2 + equivalent quantity of a free lysate) for 96 hours in 2 mL RPMI 1640 complete medium. Then CD3⁺ CD8⁺ CD69⁺ T cells (cell viability 85%), i.e., cancer-specific T cells that can recognize cancer cell antigens, in the incubated cells were further sorted by flow cytometry. The cancer-specific T cells obtained by the above two-step sorting were co-incubated with IL-2 (2000 U/mL), IL-7 (200 U/mL), IL-15 (200 U/mL), and αCD3/αCD28 antibodies for 7 days to expand the sorted cancer-specific T cells (cell viability 85%).

### (4) Sorted and expanded T cells for cancer prevention

Female BALB/c at 6-8 weeks were selected as model mice to prepare breast cancer tumor-bearing mice. One day before adoptive transfer of cells, the recipient mice were intraperitoneally injected with a 100 mg/kg dose of cyclophosphamide to eliminate immune cells in the recipient mice. The mice were intravenously injected with 2 million expanded cancer-specific CD8⁺ T cells on day 0. At the same time, each mouse was subcutaneously injected and inoculated with 4×10⁵ 4T1 cells on day 0, and the methods of monitoring tumor growth and survival time of the mice were the same as above.

### (5) Experimental results

As shown in FIG. 9, compared with the control group, the tumor growth rate of the cancer-specific T cell treatment group was significantly slower and the survival time of the mice was significantly prolonged. Moreover, the preventive effect of cancer-specific T cells obtained by assisted sorting and expansion of the microparticles loaded with whole-cell components was better than that of cancer-specific T cells obtained by assisted sorting and expansion of blank microparticles + free lysate. Thus, the cancer-specific T cells subjected to the two-step sorting and expansion according to the present disclosure had an excellent preventive effect on breast cancer.

### Example 9: Cancer-specific T cells for prevention of cancer metastasis

This example illustrated the prevention of cancer metastasis using cancer-specific T cells after sorting and expansion with a mouse melanoma mouse lung metastasis cancer model. In practical application, the specific dosage form, adjuvant, incubation time, incubation concentration, administration time, administration times, and administration regimen can be adjusted according to situations. In this example, mouse melanoma tumor tissue and cancer cell lines were lysed and then dissolved with 8 M urea, then the tumor tissue lysis components and the cancer cell line lysis components were loaded onto nanoparticles at a mass ratio of 1:2, and the particles were used to activate and assist in sorting cancer-specific T cells, and the obtained T cells were expanded to prevent cancer metastasis in mice. In this example, nanoparticles loaded with four peptide neoantigens
B16-M20 (Tubb3, FRRKAFLHWYTGEAMDEMEFTEAESNM),
B16-M24 (Dag1, TAVITPPTTTTKKARVSTPKPATPSTD),
B16-M46 (Actn4, NHSGLVTFQAFIDVMSRETTDTDTADQ), and TRP2:180-188 (SVYDFFVWL) were used as control nanoparticles to analyze the efficacy of cancer-specific T cells obtained by assisted sorting and expansion of nanoparticles loaded with whole-cell antigens and nanoparticles loaded with multiple peptide neoantigens in preventing cancer lung metastasis.

### (1) Preparation of antigen components

After collecting the mouse B16F10 melanoma tumor tissue and cultured B16F10 cancer cell lines, the whole-cell components of the tumor tissue and the cancer cell were lysed and dissolved with 8 M urea, and then the tumor tissue components and the cancer cell line lysate components were miscible at a mass ratio of 1:2 to obtain the antigen components.

### (2) Preparation of nanoparticles

Nanoparticle 1 in this example was prepared by a solvent evaporation method, the molecular weight of the Nanoparticle 1 preparation material PLGA was 24 KDa to 38 KDa, the immune adjuvants adopted were CpG7909 and Poly(I:C), and the lysosome escape-increasing substance adopted was KALA peptide (WEAKLAKALAKALAKHLAKALAKALKACEA). The preparation method was as previously described. In the preparation process, the antigen components, adjuvants and KALA peptide were first loaded inside the nanoparticles by a double emulsion method. After the antigen components and adjuvants were loaded inside, 100 mg nanoparticles were centrifuged at 10000 g for 20 minutes, and resuspended with 10 mL of ultrapure water containing 4% trehalose and freeze-dried for 48 h for later use. The average particle size of the Nanoparticle 1 was about 470 nm; and approximately 10 µg of protein and peptide components of lysate, containing 0.02 mg of CpG7909 and Poly(I:C) each and 0.04 mg of KALA peptide, were loaded per 1 mg of PLGA Nanoparticle 1. The preparation method of control Nanoparticle 2 loaded with four antigen peptides was the same as above. The particle size of the control Nanoparticle 2 was about 460 nm, and about 10 µg of the antigen peptides and an equivalent quantity of the adjuvants and KALA peptide were loaded per 1 mg of PLGA nanoparticle.

### (3) Sorting and expansion of cancer-specific T cells

Female C57BL/6 mice aged 6-8 weeks were selected and subcutaneously injected with 200 µL of 2 mg PLGA nanoparticles on day 0, day 4, day 7, day 14, day 21, and day 28, separately. The mice were sacrificed on day 32, the peripheral blood of the mice was collected, and then peripheral blood mononuclear cells (PBMCs) were isolated from the peripheral blood. CD3⁺ T cells and CD19⁺ B cells were first sorted from PBMCs, and then 7 million T cells and 7 million B cells obtained from the first sorting were co-incubated with 4 mg of Nanoparticle 1 or Nanoparticle 2 for 96 hours in 10 mL of RPMI 1640 complete medium. Then CD3⁺ CD8⁺ CD69⁺ T cells and CD3⁺ CD4⁺ CD69⁺ T cells, i.e., cancer-specific T cells that can recognize cancer cell antigens (cell viability 80%), in the incubated cells were sorted by flow cytometry. One million CD3⁺ CD8⁺ CD69⁺ T cells or 1 million CD3⁺ CD4⁺ CD69⁺ T cells sorted above were co-incubated with IL-2 (1000 U/mL), IL-12 (1000 U/mL) and αCD3/αCD28 antibodies (10 ng/mL) for 14 days in 10 mL of RPMI 1640 complete medium to expand cancer-specific T cells (cell viability 80%).

### (4) Cancer-specific T cells for prevention of cancer metastasis

Female C57BL/6 aged 6-8 weeks were selected as model mice to prepare melanoma-bearing mice. One day before adoptive transfer of cells, the recipient mice were intraperitoneally injected with a 100 mg/kg dose of cyclophosphamide to eliminate immune cells in the recipient mice. The mice were intravenously injected with 1.5 million cancer-specific CD8⁺ T cells and 500000 cancer-specific CD4⁺ T cells on day 0. At the same time, each mouse was intravenously injected and inoculated with 0.5×10⁵ B16F10 cells on day 1, the mice were sacrificed on day 14, and the number of melanoma foci in the lungs of the mice was observed and recorded.

### (5) Experimental results

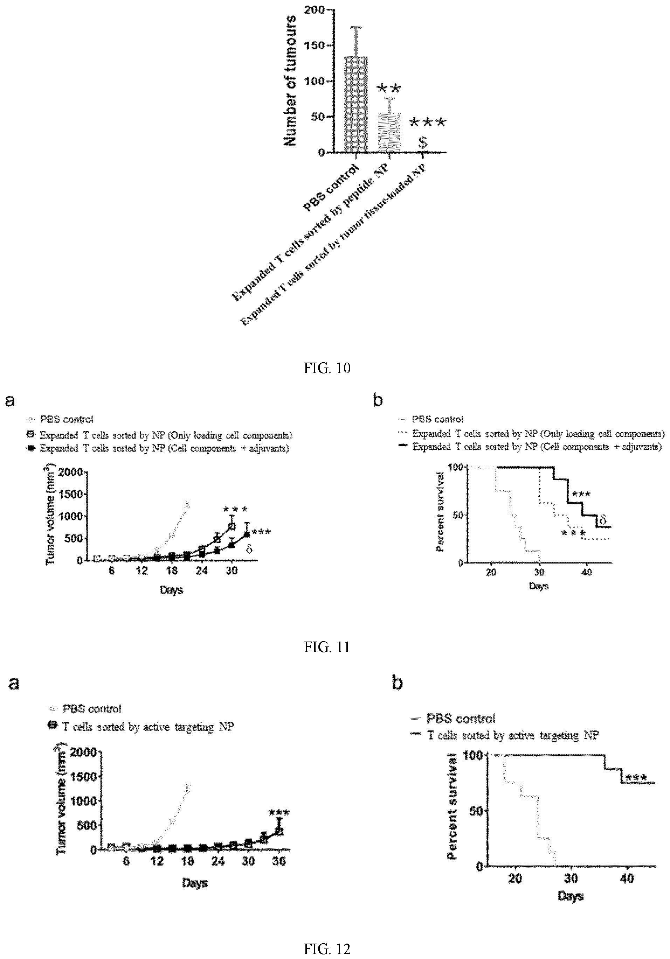

As shown in FIG. 10, the cancer-specific T cells obtained by the Nanoparticle 1 and Nanoparticle 2 assisted sorting can effectively prevent cancer metastasis after expansion. Moreover, cancer-specific T cells obtained by assisted sorting and expansion of the Nanoparticle 1 loaded with the whole-cell components were better than those obtained by the Nanoparticle 2 loaded with the four neoantigen peptides.

### Example 10: Sorted and expanded T cells for pancreatic cancer treatment

In this example, mouse Pan02 pancreatic cancer tumor tissue and MC38 colon cancer tumor tissue lysis components were loaded onto nanoparticles at a ratio of 3:1, and the nanoparticles were used to assist in isolation of cancer-specific T cells in peripheral blood of mice, and then the cancer-specific T cells were expanded for treatment of pancreatic cancer. In the experiment, mouse pancreatic cancer and colon cancer tumor tissue were first obtained and lysed to prepare water-soluble components and original water-insoluble components dissolved in 6 M guanidine hydrochloride. In preparing the particles, the water-soluble components were a 3:1 mixture of the water-soluble components of the pancreatic cancer tumor tissue and the water-soluble components of the colon cancer tumor tissue; and the water-insoluble components were a 3:1 mixture of the water-insoluble components of the pancreatic cancer tumor tissue and the water-insoluble components of the colon cancer tumor tissue. The nanoparticles were prepared with PLGA as a nanoparticle backbone material and BCG as an adjuvant, and the nanoparticles were used to assist sorting and expansion of cancer-specific T cells.

### (1) Preparation of antigen components

Each C57BL/6 mouse was subcutaneously inoculated with 2×10⁶ MC38 colon cancer cells or 1×10⁶ Pan02 pancreatic cancer cells under the axillary area, and the mice were sacrificed and the tumor tissue removed when the inoculated tumor in each mouse grew to about 1000 mm³. The lysis method and the collection method of each component were the same as in Example 1, except that 6 M guanidine hydrochloride was used instead of 8 M urea to dissolve the water-insoluble components, and the tumor tissue lysate components were the antigen components. The method of lysing BCG was the same as the method of lysing the tumor tissue.

### (2) Preparation of nanoparticles

The nanoparticles in this example were prepared by a double emulsion method. The molecular weight of the nanoparticle preparation material PLGA adopted in Nanoparticle 1 was 7 KDa to 17 KDa, and the immune adjuvant adopted was BCG lysate components; and the preparation method was as previously described. The antigen components and adjuvant were first loaded inside the nanoparticles, and then 100 mg of the nanoparticles were centrifuged at 12000 g for 20 minutes, and resuspended with 10 mL of ultrapure water containing 4% trehalose and freeze-dried for 48 hours to obtain a freeze-dried powder for later use; 20 mg of nanoparticles were dissolved in 0.9 mL of PBS before Nanoparticle 1 injection, mixed with 0.1 mL of a sample containing the antigen components (80 mg/mL) and GM-CSF (50 ng/mL) and reacted at room temperature for 10 min for use; the average particle size of the Nanoparticle 1 was about 160 nm, and the surface potential was about -4 mV; and approximately 130 µg of protein or peptide components were loaded per 1 mg of PLGA Nanoparticle 1, and 0.02 mg of BCG was loaded per 1 mg of PLGA Nanoparticle 1. The preparation materials and preparation method of the Nanoparticle 2 were the same as those of the Nanoparticle 1, and the particle size of the Nanoparticle 2 was about 160 nm and the surface charge was about -4 mV; and each 1 mg of PLGA Nanoparticle 2 was loaded with 130 µg of the protein or peptide components in the tumor tissue lysate but not loaded with any adjuvant.

### (3) Preparation of cancer-specific T cells

Female C57BL/6 mice aged 6-8 weeks were selected and subcutaneously injected with 200 µL of 2 mg PLGA nanoparticles on day 0, day 4, day 7, day 14, day 21, and day 28, separately. The mice were sacrificed on day 32, the peripheral blood of the mice was collected, and then peripheral blood mononuclear cells (PBMCs) were isolated from the peripheral blood. CD3⁺ T cells and CD19⁺ B cells were first sorted from PBMCs using a magnetic bead sorting method, and 9 million CD3⁺ T cells and 12 million CD19⁺ B cells sorted in the first step were co-incubated with *50µg* nanoparticles (Nanoparticle 1 or Nanoparticle 2) for 6 hours. Then CD3⁺ CD69⁺ T cells (cell viability 75%), i.e., cancer-specific T cells, were sorted by flow cytometry. One million CD3⁺ CD69⁺ T cells obtained by the above two-step sorting were co-incubated with IL-2 (1000 U/mL) and granulocyte-macrophage colony-stimulating factor (GM-CSF, 10 ng/mL) for 14 days in 15 mL of high glucose DMEM complete medium (37°C, 5% CO₂) (medium change every two days) to expand the sorted cancer-specific T cells.

### (4) Cancer-specific T cells for pancreatic cancer treatment

Female C57BL/6 aged 6-8 weeks were selected as model mice to prepare pancreatic cancer tumor-bearing mice. At the same time, each mouse was subcutaneously inoculated with 1×10⁶ Pan02 pancreatic cancer cells on day 0. The mice were intravenously injected with 2 million expanded cancer-specific T cells on day 6, day 9, day 12, day 17, and day 23, separately. The methods of monitoring and recording tumor volumes in mice were the same as above.

### (5) Experimental results

As shown in FIG. 11, cancer-specific T cells sorted and expanded using Nanoparticle 1 and Nanoparticle 2 according to the present disclosure were effective in treating pancreatic cancer.

### Example 11: Sorted and expanded T cells for cancer treatment

This example used mannose as an active targeting target head as an example to illustrate how to sort and expand cancer-specific T cells. It can be adjusted according to situations in practical application. The nanoparticle system can be ingested into dendritic cells through mannose receptors on the surface of dendritic cells, and the antigens loaded by the particles can activate cancer-specific T cells after being presented by the dendritic cells. In practical application, mannan, CD32 antibodies, CD19 antibodies, CD20 antibodies, B220 antibodies, CD11c antibodies, and other target head-modified nanoparticles or microparticles that can target antigen-presenting cells can also be used.

### (1) Preparation of antigen components

After the cultured B16F10 cancer cells were collected, the cancer cells were lysed with an 8 M aqueous urea solution, and then whole-cell components of the lysed cancer cells, i.e. antigen components, were dissolved with the 8 M aqueous urea solution.

### (2) Preparation of nanoparticles

Nanoparticle 1 in this example was prepared using a double emulsion method. The nanoparticle preparation materials adopted were PLGA and mannose-modified PLGA. When the nanoparticles with target heads were prepared, the mass ratio of the two materials was 4:1 when used together, and the molecular weight was 7 KDa to 17 KDa for both. The immune adjuvants adopted were Poly(I:C) and CpG7909. The preparation method was as previously described. The antigen components and adjuvants were first co-loaded inside the nanoparticles, and then 100 mg of the nanoparticles were centrifuged at 12000 g for 25 minutes, and resuspended with 10 mL of ultrapure water containing 4% trehalose and freeze-dried for 48 h for later use. The average particle size of the Nanoparticle 1 was about 120 nm, and approximately 80 µg of protein or peptide components were loaded per 1 mg of PLGA nanoparticles, containing 0.02 mg of Poly(I:C) and CpG7909 each.

### (3) Preparation of dendritic cells (DCs)

This example used bone marrow-derived dendritic cells (BMDCs) as antigen-presenting cells. The preparation method was the same as above.

### (4) Activation of dendritic cells

Mouse BMDCs were plated into cell culture plates, 5 mL of RPMI 1640 (10% FBS) medium was added to every 100000 BMDC cells, and then 30 µg of the Nanoparticle 1 was added and incubated with BMDCs for 48 h (37°C, 5% CO₂). Then the BMDCs were collected and centrifuged at 300 g for 5 minutes, washed twice with phosphate buffer saline (PBS), and then resuspended in PBS for later use.

### (5) Preparation of cancer-specific T cells

Female C57BL/6 mice aged 6-8 weeks were selected and subcutaneously injected with 200 µL of 2 mg Nanoparticle 1 on day 0, day 4, day 7, day 14, day 21, and day 28, separately. The mice were sacrificed on day 32, the peripheral blood of the mice was collected, then peripheral blood mononuclear cells (PBMCs) were isolated from the peripheral blood, and CD3⁺ CD8⁺ T cells were sorted from the PBMCs by magnetic bead sorting. The sorted 2 million CD8⁺ T cells were co-incubated with 5 million BMDCs prepared in step (4), 40 µg of the Nanoparticle 1 and 10 ng/mL of IL-7 for 18 hours in 4 mL of DMEM high glucose complete medium. Then CD8⁺ CD69⁺ T cells (cell viability 75%), i.e., cancer-specific T cells that can recognize tumor antigens, in the incubated CD8⁺ T cells were sorted by flow cytometry. The 200000 CD8⁺ CD69⁺ T cells obtained by the above two-step sorting were co-incubated with IL-2 (1000 U/mL) and IL-7 (1000 U/mL) for 10 days to expand cancer-specific T cells (cell viability 75%).

### (6) Cancer-specific T cells for cancer prevention

Female C57BL/6 mice aged 6-8 weeks were selected as model mice to prepare melanoma tumor-bearing mice. One day before adoptive transfer of cells, the recipient mice were intraperitoneally injected with a 100 mg/kg dose of cyclophosphamide to eliminate immune cells in the recipient mice. Then 2 million cancer-specific T cells prepared in step (5) were subcutaneously injected into the recipient mice. Each recipient mouse was subcutaneously inoculated with 1.5×10⁵ B16F10 cells on the lower right side of the back the next day. The methods of monitoring tumor growth rates and survival time in the mice were the same as above.

### (7) Experimental results

As shown in FIG. 12, the tumor growth rate of the T cell-treated mice was significantly slower compared with the PBS control group.

### Example 12: Sorted and expanded cancer-specific T cells for liver cancer prevention

In this example, Hepa1-6 hepatocellular carcinoma cells were first lysed, PLGA was used as a nanoparticle skeleton material, and Poly(I:C) and bacterial Bacille Calmette-Guerin (BCG) were used as immune adjuvants to prepare nanoparticles loaded with whole-cell antigens of hepatocellular carcinoma cells by a solvent evaporation method, then the particles were mixed with B cells and T cells, and cancer-specific T cells were obtained after sorting and expansion.

### (1) Preparation of antigen components

The cultured Hepa 1-6 hepatoma cells were collected and washed twice with PBS, treated with heating and ultraviolet irradiation, and then whole-cell components of cancer cells, i.e. antigen components, were lysed and dissolved with 8 M urea. The method of lysing BCG was the same as above, and the lysate of BCG was used as an adjuvant.

### (2) Preparation of nanoparticles

In this example, the nanoparticles were prepared by the solvent evaporation method, the molecular weight of the nanoparticle preparation material PLGA adopted was 24 KDa to 38 KDa, and the immune adjuvants adopted were BCG and Poly(I:C). The preparation method was as previously described. The antigen components and adjuvants were first loaded inside the nanoparticles, and then 100 mg of the nanoparticles were centrifuged at 10000 g for 25 minutes, and resuspended with 10 mL of ultrapure water containing 4% trehalose and freeze-dried for 48 h for later use. The particle size of the nanoparticles was about 270 nm; and 100 µg of protein or peptide components were loaded, and 0.02 mg of BCG and Poly(I:C) each were loaded per 1 mg of PLGA nanoparticles.

### (3) Preparation of cancer-specific T cells

Female C57BL/6 mice aged 6-8 weeks were selected and subcutaneously injected with 200 µL of 2 mg PLGA nanoparticles on day 0, day 4, day 7, day 14, day 21, and day 28, separately. The mice were sacrificed on day 32, the peripheral blood of the mice was collected, then peripheral blood mononuclear cells (PBMCs) were isolated from the peripheral blood, and CD8⁺ T cells were sorted from the PBMCs by magnetic bead sorting. The sorted 2 million CD8⁺ T cells, nanoparticles (500 µg), 8 million BAF3 mouse B cell lines, and IL-7 (10 ng/mL) were co-incubated for 48 hours in 2 mL of RPMI 1640 complete medium. Then CD8⁺ CD69⁺ T cells (cell viability 80%), i.e., cancer-specific T cells that can recognize tumor antigens, in the incubated CD8⁺ T cells were sorted by flow cytometry. The above sorted CD8⁺ CD69⁺ T cells were co-incubated with IL-2 (1000 U/mL), IL-7 (500 U/mL), IL-15 (500 U/mL), and αCD28 antibodies for 7 days to expand the sorted cancer-specific T cells (cell viability 80%).

### (4) Cancer-specific T cells for cancer prevention

Female C57BL/6 at 6-8 weeks were selected as model mice to prepare hepatocellular carcinoma tumor-bearing mice. One day before adoptive transfer of cells, the recipient mice were intraperitoneally injected with a 100 mg/kg dose of cyclophosphamide to eliminate immune cells in the recipient mice. The mice were injected with 2 million expanded cancer-specific T cells on day 0. At the same time, each mouse was subcutaneously injected and inoculated with 1.0×10⁶ Hepa1-6 hepatocellular carcinoma cells on the back on day 0, and the tumor growth and survival time of the mice were recorded in the same manner as above.

### (5) Experimental results

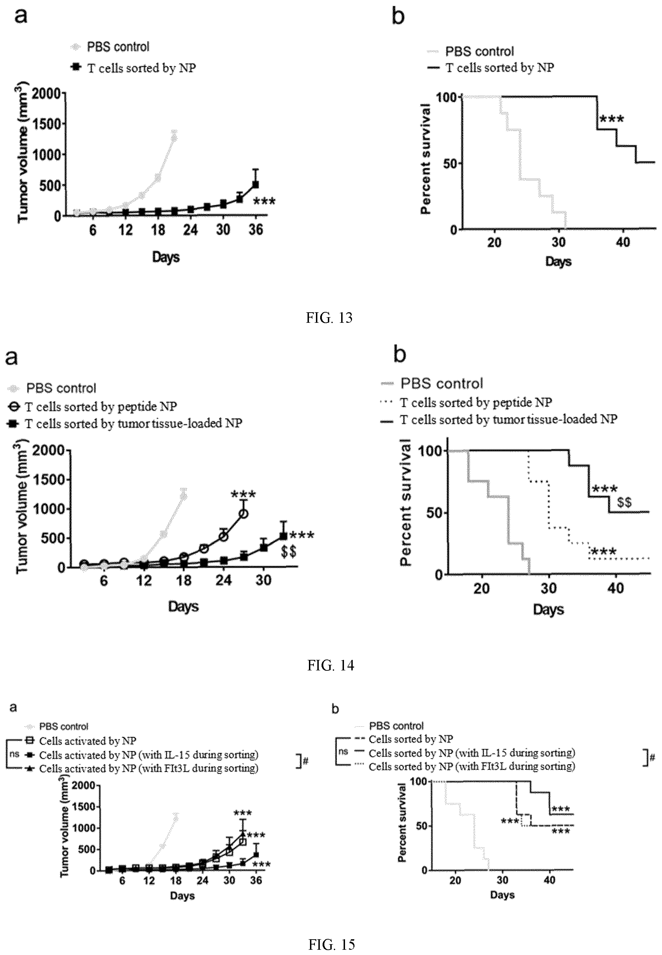

As shown in FIG. 13, the tumor growth rate of the cancer-specific T cell-treated mice was significantly slower compared with the control group.

### Example 13: Sorted T cells assisted by calcified nanoparticles for cancer prevention

In this example, calcified nanoparticles were used to sort cancer-specific T cells, and other biomineralization techniques, crosslinking, gelation, etc. may also be used to modify the particles in actual use. In practical application, the specific dosage form, adjuvant, particle size, administration time, administration times, administration regimen, etc. can be adjusted according to situations. In this example, mouse melanoma tumor tissue and cancer cell lines were lysed and then dissolved with 8 M urea, and then the tumor tissue lysis components and the cancer cell line lysis components were loaded onto Nanoparticle 1 at a mass ratio of 1:1. In this example, nanoparticles loaded with four peptide neoantigens
B16-M20 (Tubb3, FRRKAFLHWYTGEAMDEMEFTEAESNM),
B16-M24 (Dag1, TAVITPPTTTTKKARVSTPKPATPSTD),
B16-M46 (Actn4, NHSGLVTFQAFIDVMSRETTDTDTADQ), and
TRP2:180-188 (SVYDFFVWL) were used as control Nanoparticle 2.

### (1) Preparation of antigen components

After collecting mouse B16F10 melanoma tumor tissue and cultured B16F10 cancer cell lines, the whole-cell components of the tumor tissue and the cancer cell lines were lysed and dissolved with 8 M urea, and then the tumor tissue components and the cancer cell line components were miscible at a mass ratio of 1:1 to obtain the antigen components.

### (2) Preparation of nanoparticles

This example biocalcified the nanoparticles after loading whole-cell antigens into the interior of and onto the surface of the nanoparticles. The Nanoparticle 1 in this example was prepared by a double emulsion method, the molecular weight of the Nanoparticle 1 preparation material PLGA was 7 KDa to 17 KDa, the immune adjuvants were CpG2006 and Poly(I:C), and the lysosome escape-enhancing substance was GALA peptide (WEAALAEALAEALAEHLAEALAEALEALAA). The preparation method was as follows. The antigen components, adjuvants and GALA peptide were first loaded inside the nanoparticles, then 100 mg of PLGA nanoparticles were centrifuged at 13000 g for 20 minutes and then resuspended with 18 mL of PBS, then 2 mL of the antigen components dissolved in 8 M urea (60 mg/mL) was added, and the precipitate was collected after centrifugation at 12000 g for 20 minutes after 10 minutes at room temperature. The 100 mg PLGA nanoparticles were then resuspended in 20 mL of DMEM medium, and then 200 µL of CaCl₂ (1 mM) was added and reacted at 37°C for two hours. The precipitate was then collected after centrifugation at 10000 g for 20 minutes and resuspended with ultrapure water and centrifuged twice. The average particle size of the nanoparticles was about 290 nm; and approximately 230 µg of protein or peptide components in the antigen components were loaded, 0.02 mg of CpG and Poly(I:C) each were loaded, and 0.001 mg of GALA peptide were loaded per 1 mg of PLGA nanoparticles.

The preparation materials and preparation method of control Nanoparticle 2 loaded with various antigen peptides were the same as above. The particle size of the control nanoparticles was about 290 nm, and about 230 µg of antigen peptides were loaded, and an equivalent quantity of the adjuvants and GALA peptide was loaded per 1 mg of PLGA nanoparticles.

### (3) Preparation of cancer-specific T cells

Female C57BL/6 mice aged 6-8 weeks were selected, and 1.5×10⁵ B16F10 were subcutaneously inoculated on the back of the mice on day 0, followed by intraperitoneal injection of PD-1 antibodies at a dose of 10 mg/kg on day 10, day 13, day 17, day 21, and day 28, separately. The mice were sacrificed on day 30, the peripheral blood of the mice was collected, then peripheral blood mononuclear cells (PBMCs) were isolated from the peripheral blood, and CD8⁺ T cells and B cells were first sorted from the PBMCs using flow cytometry. The sorted CD8⁺ T cells (5 million cells), nanoparticles (500 µg), B cells (20 million cells), and IL-7 (10 ng/mL) were co-incubated for 48 hours in 20 mL of RPMI 1640 complete medium. Then CD8⁺ CD137⁺ T cells (cell viability 75%), i.e., cancer-specific T cells that can recognize tumor antigens, in the incubated CD8⁺ T cells were sorted by flow cytometry. The above sorted 500000 CD8⁺ CD137⁺ T cells were co-incubated with IL-2 (1000 U/mL), IL-7 (500 U/mL), IL-15 (500 U/mL), and αCD3 antibody (10 ng/mL) for 14 days to expand cancer-specific T cells (cell viability 75%).

### (4) Cancer-specific T cells for cancer prevention

Female C57BL/6 mice aged 6-8 weeks were selected, and one day before adoptive transfer of cells in the mice, the recipient mice were intraperitoneally injected with a 100 mg/kg dose of cyclophosphamide to eliminate immune cells in the recipient mice. The mice were injected with 1 million expanded cancer-specific T cells on day 0. At the same time, each mouse was subcutaneously injected and inoculated with 1.5×10⁵ B16F10 melanoma cells on the back on day 0, and the ways of monitoring tumor growth and survival time of the mice were the same as above.

### (5) Experimental results

As shown in FIG. 14, compared with the control group, cancer-specific T cells activated by the calcified nanoparticles and sorted with the assistance of the calcified nanoparticles can prolong the survival of the mice and effectively prevent cancer. Moreover, the effect of nanoparticles loaded with whole-cell components was better than that of nanoparticles loaded with 4 neoantigen peptides.

### Example 14 Cancer-specific T cells for melanoma treatment

In this example, B16F10 melanoma tumor tissue was first lysed to prepare water-soluble components and water-insoluble components of the tumor tissue, and then PLGA was used as a nanoparticle skeleton material, Poly(I:C) and CpG2395 were used as immune adjuvants, and melittin (GIGAVLKVLTTGLPALISWIKRKRQQ-amide) was used as a lysosome escape-promoting component to prepare nanoparticles by a solvent evaporation method.

### (1) Preparation of antigen components

When collecting tumor tissue, 1.5×10⁵ B16F10 cells were subcutaneously inoculated on the back of each C57BL/6 mouse. The mice were sacrificed and the tumor tissue was removed when the tumors grew to about 1000 mm³. The tumor tissue was cut into pieces and then ground. After preparing a single-cell suspension through a cell strainer, an appropriate amount of pure water was added, and freezing and thawing was repeated 5 times, accompanied by ultrasound to destroy and lyse the obtained sample. After the tumor tissue was lysed, the lysate was centrifuged at a rotational speed of 5000 g for 5 minutes and the supernatant was taken as water-soluble components that were soluble in pure water; and adding 2.0 M arginine and a 10% aqueous sodium chloride solution of sodium deoxycholate to the obtained precipitated portion to dissolve the precipitated portion can convert the water-insoluble components that were insoluble in pure water into soluble components in the aqueous solution. The above were the antigen components for preparing the nanoparticle system.

### (2) Preparation of nanoparticles

The nanoparticles in this example were prepared by a double emulsion method. The nanoparticles loaded with the water-soluble components and the nanoparticles loaded with the water-insoluble components were prepared separately at the time of preparation and used together at the time of application. The molecular weight of the nanoparticle preparation material PLGA adopted was 7 KDa to 17 KDa, the immune adjuvants adopted were poly(I:C) and CpG2395, and the lysosome escape-enhancing substance was melittin. The preparation method was as previously described. The antigen components, adjuvants and melittin were first loaded into the interior of the nanoparticles, and then 100 mg nanoparticles were centrifuged at 10000 g for 20 minutes, resuspended with 10 mL of ultrapure water containing 4% trehalose and freeze-dried for 48 h; before use, the nanoparticles were resuspended in 9 mL of PBS, then 1 mL of the antigen components (protein concentration of 80 mg/mL) was added for reaction at room temperature for 10 min to obtain nanoparticles loaded with the lysate both inside and outside. The average particle size of the nanoparticles was about 290 nm, and approximately 140 µg of protein or peptide components in the antigen components were loaded, 0.02 mg of poly(I:C) and CpG2395 immune adjuvants each were loaded, and 0.05 mg of melittin was loaded per 1 mg of PLGA nanoparticles.

### (3) Preparation of cancer-specific T cells

Female C57BL/6 mice aged 6-8 weeks were selected, and 1.5×10⁵ B16F10 were subcutaneously inoculated on the back of the mice on day 0, and then the mice were subcutaneously injected with 1 mg of water-soluble component-loaded nanoparticles and 1 mg of water-insoluble component-loaded nanoparticles on day 7, day 10, day 15, day 20, and day 25, separately. The mice were sacrificed on day 30, PBMCs of the mice were collected, and CD8⁺ T cells, CD4⁺ T cells and B cells were sorted from the PBMCs using flow cytometry. The sorted CD8⁺ T cells (1 million cells), CD4⁺ T cells (1 million cells), nanoparticles (200 µg of water-soluble component-loaded nanoparticles and water-insoluble component-loaded nanoparticles each), B cells (3 million cells), and IL-15 (50 ng/mL) were co-incubated for 72 hours in 2 mL of RPMI 1640 complete medium; or the sorted CD8⁺ T cells (1 million cells), CD4⁺ T cells (1 million cells), nanoparticles (200 µg of water-soluble component-loaded nanoparticles and water-insoluble component-loaded nanoparticles each), and B cells (3 million cells) were co-incubated for 72 hours in 2 mL of RPMI 1640 complete medium; or the sorted CD8⁺ T cells (1 million cells), CD4⁺ T cells (1 million cells), nanoparticles (200 µg of water-soluble component-loaded nanoparticles and water-insoluble component-loaded nanoparticles each), B cells (3 million cells), and Flt3L (50 ng/mL) were co-incubated for 72 hours in 2 mL of RPMI 1640 complete medium. Then CD8⁺ CD69⁺ T cells in the incubated CD8⁺ T cells (cell viability 80%) and CD4⁺ CD69⁺ T cells in the incubated CD4⁺ T cells (cell viability 80%), i.e., cancer-specific T cells that can recognize tumor antigens, were sorted by flow cytometry. The above sorted 100000 CD8⁺ CD69⁺ T cells or 100000 CD4⁺ CD69⁺ T cells were co-incubated with IL-2 (1000 U/mL), IL-7 (500 U/mL), IL-15 (500 U/mL), and αCD3 antibodies (10 ng/mL) for 11 days in 20 mL of RPMI 1640 complete medium (37°C, 5% CO₂) (medium change every two days) to expand cancer-specific T cells.

### (4) Expanded cancer-specific T cells for cancer treatment

Female C57BL/6 aged 6-8 weeks were selected as model mice to prepare melanoma-bearing mice. Each mouse was subcutaneously inoculated with 1.5×10⁵ B16F10 cells on the lower right side of the back on day 0. After melanoma inoculation, 800000 cancer-specific CD8⁺ T cells and 200000 cancer-specific CD4⁺ T cells were intravenously injected on day 4, day 7, day 10, day 15, and day 20, separately. In the experiment, the methods of monitoring tumor volumes and survival time in mice were the same as above.

### (5) Experimental results

As shown in FIG. 15, the cancer-specific T cells sorted and expanded according to the present disclosure had a good therapeutic effect on melanoma.

### Example 15: Cancer-specific T cells for melanoma treatment

In this example, B16F10 melanoma tumor tissue was first lysed, and then nanoparticles were prepared with PLGA as a nanoparticle skeleton material, Poly(I:C) and CpG7909 as immune adjuvants, and polyarginine and polylysine as components to increase lysosome escape.

### (1) Preparation of antigen components

When the tumor tissue was collected, 1.5×10⁵ B16F10 cells were subcutaneously inoculated on the back of each C57BL/6 mouse, the mice were sacrificed when the tumors grew to about 1000 mm³ and the tumor tissue was removed, the tumor tissue was pulverized using a tissue homogenizer, then an appropriate amount of ultrapure water was added, and freezing and thawing was repeated to lyse the cells, then nuclease was added for 5 minutes, and then the nuclease was inactivated at 95°C for 10 minutes to obtain whole-cell antigen components with whole-cell nucleic acid components removed (mainly proteins and peptides). Then the antigen components were centrifuged at 8000 g for 3 minutes, and the supernatant portion was water-soluble components; and in the precipitated portion, water-insoluble components were dissolved using 0.1 M metformin hydrochloride and a 0.1 M aqueous arginine solution. The antigen components for preparing the nanoparticles were obtained by mixing the water-soluble components and the water-insoluble components dissolved in the dissolving solution according to a mass ratio of 1:1.

### (2) Preparation of nanoparticles

In this example, Nanoparticle 1 was prepared by a double emulsion method and had the ability to target dendritic cells. The Nanoparticle 1 preparation materials adopted were PLA and mannan-modified PLA, both of which had molecular weights of 20 KDa to 30 KDa, and the mass ratio of unmodified PLA and mannan-modified PLA was 9:1 when used. The immune adjuvants adopted were poly(I:C) and CpG7909, and the substances that increase lysosome immune escape were polyarginine and polylysine. The preparation method was as previously described. The lysate components, adjuvants, polyarginine and polylysine were first loaded inside the nanoparticles. After the above components were loaded inside, 100 mg nanoparticles were centrifuged at 10000 g for 20 minutes and freeze-dried for 48 h after resuspension using 10 mL of ultrapure water containing 4% trehalose. The average particle size of the nanoparticles was 360 nm; and approximately 100 µg of protein or peptide components in the antigen components were loaded, 0.02 mg of poly(I:C) and CpG7909 immune adjuvants each were loaded, and 0.01 mg of polyarginine and polylysine each were loaded per 1 mg of PLGA nanoparticles.

### (3) Preparation of cancer-specific T cells

Female C57BL/6 mice aged 6-8 weeks were selected, the mice were subcutaneously inoculated with 1.5×10⁵ B16F10 on the back on day 0, and then the mice were subcutaneously injected with 2 mg of the Nanoparticle 1 on day 7, day 10, day 15, day 20, and day 25, separately. The mice were sacrificed on day 30, PBMCs of mice were collected, and CD3⁺ T cells and B cells were sorted from the PBMCs using flow cytometry. The sorted CD3⁺ T cells (2 million cells), Nanoparticle 1 (40 µg), B cells (3 million cells), and IL-15 (10 ng/mL) were co-incubated for 6 hours in 2 mL of RPMI 1640 complete medium. Then CD3⁺ CD69⁺ T cells (cell viability 85%), i.e., cancer-specific T cells that can recognize tumor antigens, in the incubated T cells were sorted by flow cytometry. The above sorted 300000 CD3⁺ CD69⁺ T cells were co-incubated with IL-2 (1000 U/mL), IL-7 (1000 U/mL), IL-15 (1000 U/mL), and αCD3 antibodies (20 ng/mL) for 14 days in 20 mL of RPMI 1640 complete medium to expand cancer-specific T cells.

### (4) T cells for cancer treatment

Female C57BL/6 aged 6-8 weeks were selected as model mice to prepare melanoma-bearing mice. Each mouse was subcutaneously inoculated with 1.5×10⁵ B16F10 cells on the lower right side of the back on day 0. After melanoma inoculation, 800000 expanded CD3⁺ T cells were intravenously injected on day 4, day 7, day 10, day 15, and day 20, separately. The methods of monitoring tumor volumes and survival time in mice were the same as above.

### (5) Experimental results

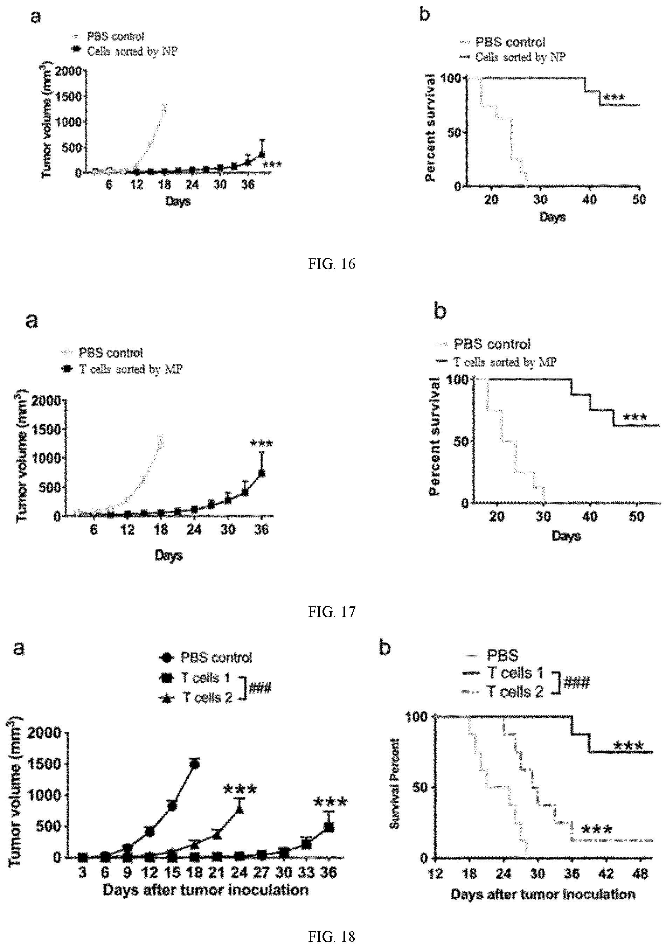

As shown in FIG. 16, the tumors of the PBS control group grew rapidly, and the tumor growth rate of mice treated with cancer-specific T cells sorted and expanded by the nanoparticles was significantly slower.

### Example 16: Sorted and expanded T cells for breast cancer prevention

In this example, breast cancer cells were first subjected to inactivation and denaturation treatment, then the cells were lysed, and water-insoluble components in the lysed cancer cells were dissolved with octyl glucoside. Then, microparticles loaded with whole-cell antigens were prepared by using PLGA as a microparticle backbone material, and CpG1018 and Poly ICLC as immune adjuvants.

### (1) Preparation of antigen components

The cultured 4T1 cells were centrifuged at 400 g for 5 minutes, and then washed twice with PBS and resuspended in ultrapure water. The obtained cancer cells were inactivated and denatured by ultraviolet and high-temperature heating respectively, then ultrapure water was added, and freezing and thawing was repeated 5 times, accompanied by ultrasound to lyse the cancer cells, the cell lysate was centrifuged at 5000 g for 10 minutes, the supernatant was water-soluble components, the precipitate was dissolved by using 10% octyl glucoside to obtain dissolved original water-insoluble components, and the water-soluble components and the water-insoluble components were mixed at a mass ratio of 2:1 to obtain antigen components 1 required for preparing the microparticles.

### (2) Preparation of microparticles

In this example, the Microparticle 1 was prepared by a double emulsion method, the molecular weight of the Microparticle 1 skeleton material PLGA was 38 KDa to 54 KDa, and the immune adjuvants adopted were CpG1018 and Poly ICLC. During preparation, the microparticles internally loaded with the antigen components 1 and adjuvants were prepared by the double emulsion method, and then 100 mg of the microparticles were centrifuged at 9000 g for 20 minutes, and resuspended using 10 mL of ultrapure water containing 4% trehalose and then dried for 48 h for later use. The average particle size of the microparticle system was about 5.0 µm; and approximately 410 µg of protein or peptide components of the cancer cells were loaded, 0.01 mg of CpG1018 and Poly ICLC each were loaded per 1 mg of PLGA microparticles.

### (3) Preparation of dendritic cells

This example used BMDCs as antigen-presenting cells. The preparation method was as previously described.

### (4) Activation of dendritic cells

5 million BMDCs, 2 mg microparticles and IL-15 (20 ng/mL) were co-incubated for 8 h in 5 mL RPMI 1640 (10% FBS) medium, and then the BMDCs were collected and the activated dendritic cells were irradiated with radiation to inactivate the dendritic cells, and the inactivated dendritic cells were used to activate T cells.

### (5) Preparation of cancer-specific T cells

Female BALB/c mice aged 6-8 weeks were selected and subcutaneously injected with 100 µL of 2 mg Microparticle 1 on day 0, day 4, day 7, day 14, day 21, and day 28, separately. The mice were sacrificed on day 32, PBMCs of mice were collected, and CD3⁺ T cells were sorted from the PBMCs using flow cytometry. The sorted CD3⁺ T cells (2 million cells), the inactivated BMDCs prepared in step 4 (3 million cells) and IL-7 (10 ng/mL) were co-incubated for 18 hours in 10 mL of RPMI 1640 complete medium. Then CD3⁺ CD69⁺ T cells (cell viability 85%), i.e., cancer-specific T cells that can recognize tumor antigens, in the incubated T cells were sorted by flow cytometry. The above sorted 300000 CD3⁺ CD69⁺ T cells were co-incubated with IL-2 (1000 U/mL), IL-7 (1000 U/mL), IL-15 (1000 U/mL), and αCD3 antibodies (10 ng/mL) for 14 days in 10 mL of DMEM complete medium (37°C, 5% CO₂)to expand cancer-specific T cells (cell viability 85%).

### (6) Cancer-specific T cells for cancer prevention

Female BALB/c at 6-8 weeks were selected as model mice to prepare breast cancer tumor-bearing mice. One day before adoptive transfer of cells, the recipient mice were intraperitoneally injected with a 100 mg/kg dose of cyclophosphamide to eliminate immune cells in the recipient mice. The mice were subcutaneously injected with 100 µL containing 1.2 million expanded CD3⁺ T cells on day 0. At the same time, each mouse was subcutaneously inoculated with 1×10⁶ 4T1 cells on day 0, and the tumor volume size of the mice was recorded every 3 days from day 3.

### (7) Experimental results

As shown in FIG. 17, compared with the control group, the tumor growth rate of the cancer-specific T cell treatment group obtained by microparticle sorting and expansion was significantly slower and the survival time of the mice was significantly prolonged. It can be seen that the cancer-specific T cells of the present disclosure had a preventive effect on breast cancer.

### Example 17: Sorted and expanded T cells by microparticles for breast cancer prevention

In this example, breast cancer cells were first lysed and the lysed components were dissolved using an 8 M urea solution. Then the microparticles were prepared by using PLA as a microparticle framework material, and CpG2395 and Poly ICLC as immune adjuvants.

### (1) Preparation of antigen components

The cultured 4T1 cells were centrifuged at 400 g for 5 minutes, and then washed twice with PBS and resuspended in ultrapure water. The obtained cancer cells were inactivated and denatured by ultraviolet rays and high-temperature heating, respectively, and then the cancer cells were lysed and the lysate components were dissolved by an 8 M aqueous urea solution, which was the antigen components for preparing the microparticles.

### (2) Preparation of microparticles

Microparticle 1 in this example was prepared by a double emulsion method. The molecular weight of the Microparticle 1 framework material PLA was 40 KDa, and CpG2395 and Poly ICLC were immune adjuvants. During preparation, the antigen components and adjuvants were first loaded inside, and then 100 mg microparticles were centrifuged at 9000 g for 20 minutes, resuspended with 10 mL of ultrapure water containing 4% trehalose and dried for 48 h for later use. The average particle size of the microparticles was about 2.5 µm, and approximately 600 µg of protein or peptide components were loaded, and 0.02 mg of CpG2395 and Poly ICLC each were loaded per 1 mg of PLGA microparticles.

### (3) Preparation of cancer-specific T cells

Female C57BL/6 aged 6-8 weeks were selected and subcutaneously injected with 100 µL of Microparticle 1 containing 2 mg PLGA on day 0, day 4, day 7, day 14, day 21, and day 28, separately. The mice were sacrificed on day 32, PBMCs in the peripheral blood of the mice were collected, and CD3⁺ T cells and CD19⁺ B cells were sorted from the PBMCs using flow cytometry. The sorted CD3⁺ T cells (8 million cells), Microparticle 1 (500 µg), B cells (9 million cells), IL-7 (10 ng/mL), and IL-15 (10 ng/mL) were co-incubated for 24 hours in 5 mL of RPMI 1640 complete medium, and then CD3⁺ CD69⁺ T cells (cell viability 80%), i.e., cancer-specific T cells that can recognize tumor antigens, in the incubated CD3⁺ T cells were sorted by flow cytometry. The above sorted 500000 CD3⁺ CD69⁺ T cells were co-incubated with IL-2 (1000 U/mL), IL-7 (1000 U/mL), IL-15 (1000 U/mL), and αCD3 antibodies (10 ng/mL) for 10 days in 10 mL of DMEM complete medium (37°C, 5% CO₂) to expand cancer-specific T cells (cell viability 80%), and the obtained cells were T cells 1.

Alternatively, the sorted CD3⁺ T cells (2 million cells), Microparticle 1 (50 µg), B cells (6 million cells), IL-7 (10 ng/mL), and IL-15 (10 ng/mL) were co-incubated for 24 hours in 5 mL of RPMI1640 complete medium, then the T cells were isolated without any sorting and expansion, and the obtained cells were T cells 2 (cell viability 80%).

### (4) Cancer-specific T cells for cancer prevention

Female BALB/c at 6-8 weeks were selected as model mice to prepare breast cancer tumor-bearing mice. One day before adoptive transfer of cells, the recipient mice were intraperitoneally injected with a 100 mg/kg dose of cyclophosphamide to eliminate immune cells in the recipient mice. The mice were subcutaneously injected with 1 million of the sorted and expanded T cells 1 or T cells 2 on day 0. At the same time, each mouse was subcutaneously inoculated with 1×10⁶ 4T1 cells on day 0, and methods of monitoring the tumor volume and survival time of the mice were the same as above.

### (5) Experimental results

As shown in FIG. 18, compared with the control group, the tumor growth rate of the T cell treatment group was significantly slower and the survival time was significantly prolonged, and the effect of the T cells 1 was significantly better than that of the T cells 2.

### Example 18: Cancer-specific T cells for melanoma treatment

### (1) Preparation of antigen components

When tumor tissue was collected, each C57BL/6 mouse was first subcutaneously inoculated with 1.5×10⁵ B16F10 cells on the back. The mice were sacrificed and the tumor tissue was removed when the tumors grew to about 1000 mm³. The tumor tissue was cut into pieces and ground, and an appropriate amount of pure water was added through the cell strainer and frozing and thawing was repeated 5 times (with ultrasound) to destroy and lyse the obtained sample. After adding nuclease for 10 minutes, the nuclease was inactivated by heating at 95°C for 10 minutes. When the cultured B16F10 cancer cell line was collected, the culture medium was removed by centrifugation, then the cancer cells were washed twice with PBS, collected by centrifugation, resuspended in ultrapure water, and repeatedly frozen and thawed 3 times, accompanied by ultrasound to destroy and lyse the cancer cells, and then nuclease was added to the sample for 10 minutes and then heated at 95°C for 5 minutes to inactivate the nuclease. After the tumor tissue or cancer cell line was treated by enzyme action, the lysate was centrifuged at a rotational speed of 5000 g for 5 minutes and the supernatant was taken as water-soluble components that were soluble in pure water; and adding 50% glycerol to the obtained precipitated portion to dissolve the precipitated portion converted insoluble components to soluble components. The water-soluble components of the tumor tissue and the water-soluble components of the cancer cell line were mixed at a mass ratio of 1:1; and the water-insoluble components of the tumor tissue and the water-insoluble components of the cancer cell line were mixed at a mass ratio of 1:1. The above were the antigen components for preparing the nanoparticles.

### (2) Preparation of nanoparticles

Nanoparticle 1 in this example was prepared by a double emulsion method. The nanoparticles loaded with the water-soluble component mixture and the nanoparticles loaded with the water-insoluble component mixture were prepared separately at the time of preparation and used together at the time of application. The molecular weight of the nanoparticle preparation material PLGA was 7 KDa to 17 KDa, the immune adjuvants adopted were poly(I:C) and CpG1018, and R8 peptide (RRRRRRRR) was a substance that increased lysosome escape. The preparation method was as previously described. The lysate components, adjuvants and R8 peptide were first loaded inside the nanoparticles by the double emulsion method, and then 100 mg nanoparticles were centrifuged at 12000 g for 25 minutes, and resuspended with 10 mL of ultrapure water containing 4% trehalose and freeze-dried for 48 h; and before use, the nanoparticles were resuspended in 9 mL of PBS and then 1 mL of the lysate components (protein/peptide concentration of 80 mg/mL) was added for reaction at room temperature for 10 min to obtain nanoparticles loaded with the lysate both inside and outside. The average particle size of the nanoparticles was about 290 nm; and approximately 140 µg of protein or peptide components in the antigen components were loaded, 0.02 mg of poly(I:C) and CpG1018 immune adjuvants each were loaded, and 0.1 mg of R8 peptide was loaded per 1 mg of PLGA nanoparticles.

### (3) Preparation of cancer-specific T cells

Female C57BL/6 mice aged 6-8 weeks were selected and subcutaneously injected with 100 µL of 1 mg PLGA nanoparticles loaded with water-soluble components and 1 mg PLGA nanoparticles loaded with water-insoluble components on day 0, day 4, day 7, day 14, day 21, and day 28, separately. The mice were sacrificed on day 32, PBMCs of the mice were collected, and CD8⁺ T cells and CD4⁺ T cells were sorted from the PBMCs using flow cytometry. The sorted CD8⁺ T cells (2 million cells), CD4⁺ T cells (1 million cells), nanoparticles (50 µg; of which 25 µg were nanoparticles loaded with water-soluble components, and 25 µg were nanoparticles loaded with water-insoluble components), DC2.4 cell line (300000 cells), and IL-7 (10 ng/mL) were co-incubated for 96 hours in 2 mL RPMI 1640 complete medium, and then CD8⁺ CD69⁺ T cells (cell viability 80%) in the incubated CD8⁺ T cells and CD4⁺ CD69⁺ T cells (cell viability 80%) in the incubated CD4⁺ T cells, i.e., cancer-specific T cells that can recognize tumor antigens, were sorted by flow cytometry. The above sorted 200000 CD8+ CD69+ cancer-specific T cells or 100000 CD4+ CD69+ cancer-specific T cells were co-incubated with IL-2 (1000 U/mL), IL-7 (1000 U/mL), IL-21 (1000 U/mL), and αCD3 antibodies (10 ng/mL) for 14 days in 10 mL of DMEM complete medium (37°C, 5% CO₂) to expand cancer-specific T cells (cell viability 80%).

Alternatively, 200000 CD8⁺ T cells or 100000 CD4⁺ T cells sorted from mouse PBMCs were directly co-incubated with IL-2 (1000 U/mL), IL-7 (1000 U/mL), IL-21 (1000 U/mL), and αCD3 antibodies (10 ng/mL) without further sorting for 14 days in 10 mL of DMEM complete medium (37°C, 5% CO₂) to expand cancer-specific T cells.

### (6) T cells for cancer treatment

Female C57BL/6 mice aged 6-8 weeks were selected as model mice. Each mouse was subcutaneously inoculated with 1.5×10⁵ B16F10 cells on the lower right side of the back on day 0. After melanoma vaccination, the expanded 800000 cancer-specific CD8⁺ T cells and 400000 cancer-specific CD4⁺ T cells (after two-step sorting) were intravenously injected on day 4, day 7, day 10, day 15, and day 20; or the expanded 800000 CD8⁺ T cells and 400000 CD4⁺ T cells after only one-step sorting were injected on the above days (without nanoparticle sorting). The methods of monitoring tumor growth and survival in mice were the same as above.

### (7) Experimental results

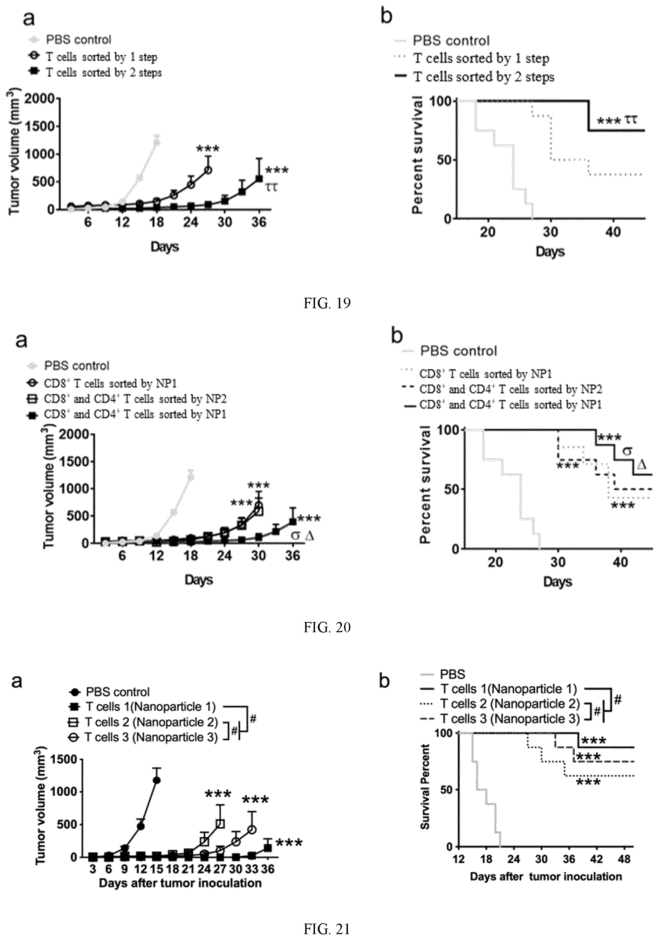

As shown in FIG. 19, the tumor growth rate was significantly slower and the survival time was significantly prolonged in the T cell treatment group compared with the control group. Moreover, cancer-specific T cells sorted and expanded using nanoparticles loaded with lysate components (after two-step sorting) were better than T cells expanded directly without nanoparticle sorting.

### Example 19: Cancer-specific T cells for colon cancer treatment

### (1) Preparation of antigen components

When tumor tissue was collected, each C57BL/6 mouse was subcutaneously inoculated with 2×10⁶ MC38 colon cancer cells on the back. The mice were sacrificed and the tumor tissue was removed when the tumors grew to a volume of about 1000 mm³. The tumor tissue was cut into pieces and then ground. The tumor tissue was lysed and the lysed components were dissolved by adding an 8 M aqueous urea solution through the cell strainer. The above were the antigen components for preparing the nanoparticles.

### (2) Preparation of nanoparticles

The nanoparticles in this example were prepared by a double emulsion method. The molecular weight of the Nanoparticle 1 preparation material PLGA was 7 KDa to 17 Kda, Poly(I:C) and CpG1018 were used as adjuvants, and NH₄HCO₃ was used as a substance to increase lysosome escape. The preparation method was as described previously. In the preparation process, the lysate components, adjuvants and NH₄HCO₃ were first loaded inside the nanoparticles, and then 100 mg nanoparticles were centrifuged at 10000 g for 20 minutes, and resuspended using 10 mL of ultrapure water containing 4% trehalose and freeze-dried for 48 h for later use; the average particle size of the nanoparticles was about 260 nm; and approximately 90 µg of protein and peptide components were loaded, 0.02 mg of poly(I:C) and CpG1018 each were loaded, and 0.01 mg of NH₄HCO₃ was loaded per 1 mg of PLGA nanoparticles. The preparation materials and preparation method of the Nanoparticle 2 were the same as those of the Nanoparticle 1, but no adjuvant was loaded and only the substance that increased lysosome escape was loaded; the particle size was about 260 nm, and the surface potential was about -7 mV; and approximately 90 µg of protein and peptide components were loaded per 1 mg of PLGA nanoparticles, and 0.01 mg of NH₄HCO₃ was loaded per 1 mg of PLGA nanoparticles without adjuvant.

### (3) Preparation of cancer-specific T cells

Female C57BL/6 aged 6-8 weeks were selected and subcutaneously injected with 100 µL of Nanoparticle 1 containing 2 mg PLGA on day 0, day 4, day 7, day 14, day 21, and day 28, separately. The mice were sacrificed on day 32, PBMCs of the mice were collected, and CD8⁺ T cells, CD4⁺ T cells and B cells were sorted from the PBMCs using flow cytometry. The sorted CD8⁺ T cells (2 million cells), CD4⁺ T cells (1 million cells), nanoparticles (50 µg), B cells (3 million cells), and IL-7 (10 ng/mL) were co-incubated for 48 hours in 2 mL RPMI 1640 complete medium, and then CD8⁺ CD69⁺ T cells (cell viability 80%) in the incubated CD8⁺ T cells and CD4⁺ CD69⁺ T cells (cell viability 80%) in the incubated CD4⁺ T cells, i.e., cancer-specific T cells that can recognize tumor antigens, were sorted by flow cytometry. The above sorted 200000 CD8⁺ CD69⁺ T cells or 200000 CD4⁺ CD69⁺ T cells were co-incubated with IL-2 (1000 U/mL), IL-7 (1000 U/mL), IL-15 (1000 U/mL), and αCD3 antibodies (10 ng/mL) for 14 days in 10 mL of DMEM complete medium (37°C, 5% CO₂) to expand cancer-specific T cells (cell viability 80%).

### (4) T cells for cancer treatment

Female C57BL/6 aged 6-8 weeks were selected as model mice to prepare colon cancer mice. Each mouse was subcutaneously inoculated with 2×10⁶ MC38 cells on the lower right side of the back on day 0. After inoculation of colon cancer cells, 1 million CD8⁺ cancer-specific T cells and 500000 CD4⁺ cancer-specific T cells were intravenously injected on day 6, day 9, day 12, day 15, day 20, and day 25, separately; or 1.5 million CD8⁺ cancer-specific T cells were injected on the above days. The methods of monitoring tumor growth and survival in mice were the same as above.

### (5) Experimental results

As shown in FIG. 20, compared with the control group, the tumor growth rate of the cancer-specific T cell treatment group obtained by nanoparticle sorting and expansion was significantly slower and the survival time was significantly prolonged. Moreover, CD8⁺ T cells and CD4⁺ T cells obtained by sorting and expansion using nanoparticles at the same time were better than CD8⁺ T cells obtained by sorting and expansion using nanoparticles alone.

### Example 20: Cancer-specific T cells for melanoma treatment

### (1) Preparation of antigen components

Each C57BL/6 mouse was subcutaneously inoculated with 1.5×10⁵ B16F10 cells on the back, and the mice were sacrificed and the tumor tissue removed when the tumors grew to approximately 1000 mm³. The tumor tissue was cut into pieces and then ground, an appropriate amount of ultrapure water was added through a cell strainer, and freezing and thawing was repeated 5 times, accompanied by ultrasound to destroy and lyse the cells. After lysis, the lysate was centrifuged at a rotational speed of 5000 g for 5 minutes and the supernatant was taken as water-soluble components that were soluble in pure water; and adding a 2 M aqueous solution of semicarbazide hydrochloride and a 0.2 M aqueous solution of agmatine sulfate to the obtained precipitated portion to dissolve the precipitated portion can convert water-insoluble components that were insoluble in pure water into soluble components in the 2 M aqueous solution of semicarbazide hydrochloride and the 0.2 M aqueous solution of agmatine sulfate. A saturated aqueous solution of ammonium sulfate was added dropwise to the water-soluble components in the lysate, the obtained sample was centrifuged at 3000 g for 5 minutes after the precipitation was complete, the precipitate was dissolved in the 2 M aqueous solution of semicarbazide hydrochloride and the 0.2 M aqueous solution of agmatine sulfate for later use, the supernatant was heated at 100°C for 5 minutes, the obtained sample was centrifuged at 3000 g for 5 minutes, the supernatant was discarded and the precipitate was dissolved in the 2 M aqueous solution of semicarbazide hydrochloride and the 0.2 M aqueous solution of agmatine sulfate; and then the precipitate after salting-out dissolved using 2 M semicarbazide hydrochloride and 0.2 M agmatine sulfate and the precipitate after heating dissolved were combined and used as a portion of the water-soluble components. The water-insoluble components in the lysate dissolved by the above 2 M aqueous solution of semicarbazide hydrochloride and the components obtained by precipitation after salting-out and heating among the water-soluble components dissolved by the above 2 M semicarbazide hydrochloride and 0.2 M agmatine sulfate were mixed at a mass ratio of 1:1 to obtain antigen components 1 for preparing Nanoparticle 1.

Each C57BL/6 mouse was subcutaneously inoculated with 1.5×10⁵ B16F10 cells on the back, and the mice were sacrificed and the tumor tissue removed when the tumors grew to a volume of approximately 1000 mm³. The tumor tissue was cut into pieces and then ground, an appropriate amount of ultrapure water was added through a cell strainer, and freezing and thawing was repeated 5 times, accompanied by ultrasound to destroy and lyse the cells. After lysis, the lysate was centrifuged at a rotational speed of 5000 g for 5 minutes and the supernatant was taken as water-soluble components that were soluble in pure water; and adding a 2 M aqueous solution of semicarbazide hydrochloride and a 0.2 M aqueous solution of agmatine sulfate to the obtained precipitated portion to dissolve the precipitated portion can convert water-insoluble components that were insoluble in pure water into soluble components in the 2 M aqueous solution of semicarbazide hydrochloride and the 0.2 M aqueous solution of agmatine sulfate. A saturated aqueous solution of ammonium sulfate was added dropwise to the water-soluble components in the lysate, the obtained sample was centrifuged at 3000 g for 5 minutes after the precipitation was complete, and the precipitate was dissolved in the 2 M aqueous solution of semicarbazide hydrochloride and the 0.2 M aqueous solution of agmatine sulfate as a portion of the water-soluble components. The water-insoluble components in the lysate dissolved in the above 2 M aqueous solution of semicarbazide hydrochloride and 0.2 M aqueous solution of agmatine sulfate and the components obtained by precipitation after salting-out among the water-soluble components dissolved by the above 2 M semicarbazide hydrochloride and 0.2 M agmatine sulfate were mixed at a mass ratio of 1:1 to obtain antigen components 2 for preparing Nanoparticle 2.

Each C57BL/6 mouse was subcutaneously inoculated with 1.5×10⁵ B16F10 cells on the back, and the mice were sacrificed and the tumor tissue removed when the tumors grew to approximately 1000 mm³. The tumor tissue was cut into pieces and then ground, an appropriate amount of ultrapure water was added through a cell strainer, and freezing and thawing was repeated 5 times, accompanied by ultrasound to destroy and lyse the cells. After lysis, the lysate was centrifuged at a rotational speed of 5000 g for 5 minutes and the supernatant was taken as water-soluble components that were soluble in pure water; and adding a 2 M aqueous solution of semicarbazide hydrochloride and a 0.2 M aqueous solution of agmatine sulfate to the obtained precipitated portion to dissolve the precipitated portion can convert water-insoluble components that were insoluble in pure water into soluble components in the 2 M aqueous solution of semicarbazide hydrochloride and the 0.2 M aqueous solution of agmatine sulfate. The water-soluble components in the lysate were heated at 100°C for 5 minutes, and then the obtained sample was centrifuged at 3000 g for 5 minutes. After the supernatant was discarded, the precipitate was dissolved in the 2 M aqueous solution of semicarbazide hydrochloride and the 0.2 M aqueous solution of agmatine sulfate as a portion of the water-soluble components. The water-insoluble components in the lysate dissolved in the above 2 M aqueous solution of semicarbazide hydrochloride and 0.2 M aqueous solution of agmatine sulfate and the components obtained by precipitation after heating among the water-soluble components dissolved by the above 2 M semicarbazide hydrochloride and 0.2 M agmatine sulfate were mixed at a mass ratio of 1:1 to obtain antigen components 3 for preparing Nanoparticle 3.

### (2) Preparation of nanoparticles

Nanoparticle 1 in this example was prepared by a double emulsion method in a solvent evaporation method. The molecular weight of the antigen-delivering nanoparticle preparation material PLGA was 20 Kda to 40 Kda, and the immune adjuvant adopted was poly(I:C). The preparation method was as previously described. The antigen components 1 and adjuvants were first loaded inside the nanoparticles by a double emulsion method, and then 300 mg of the nanoparticles were centrifuged at 14000 g for 30 minutes, and resuspended with 10 mL of ultrapure water containing 4% trehalose and freeze-dried for 48 h. The average particle size of the Nanoparticle 1 was about 100 nm, and approximately 250 µg of protein or peptide components were loaded per 1 mg of PLGA nanoparticles, and 0.01 mg of poly(I:C) was loaded per 1 mg of PLGA nanoparticles.

The preparation method and the preparation materials of the Nanoparticle 2 in this example were the same as those of the Nanoparticle 1. The preparation method was as previously described. The antigen components 2 and adjuvants were first loaded inside the nanoparticles by a double emulsion method, and then 100 mg of the nanoparticles were centrifuged at 14000 g for 30 minutes, and resuspended with 10 mL of ultrapure water containing 4% trehalose and freeze-dried for 48 h. The average particle size of the Nanoparticle 2 was about 300 nm, and approximately 250 µg of protein or peptide components were loaded per 1 mg of PLGA nanoparticles, and 0.01 mg of poly(I:C) was loaded per 1 mg of PLGA nanoparticles.

The preparation method and the preparation materials of the Nanoparticle 3 in this example were the same as those of the Nanoparticle 1. The antigen components and adjuvants were first loaded inside the nanoparticles, and then 100 mg of the nanoparticles were centrifuged at 14000 g for 30 minutes, and resuspended with 10 mL of ultrapure water containing 4% trehalose and freeze-dried for 48 h. The average particle size of the Nanoparticle 3 was about 300 nm, and approximately 250 µg of protein or peptide components were loaded per 1 mg of PLGA nanoparticles, and 0.01 mg of poly(I:C) was loaded per 1 mg of PLGA nanoparticles.

### (3) Preparation of cancer-specific T cells

Female C57BL/6 mice aged 6-8 weeks were selected and subcutaneously inoculated with 1.5×10⁵ B16F10 cells on day 0, and each mouse was intraperitoneally injected with 150 µg PD-1 antibodies on day 6, day 8, day 10, day 12, day 14, day 16, day 18, day 20, and day 22, separately. The mice were sacrificed on day 24, the peripheral blood of the mice was collected, then peripheral blood mononuclear cells (PBMCs) were isolated from the peripheral blood, and all CD69⁻ PBMCs were sorted using flow cytometry. Then 1 million CD69⁻ PBMC cells and 10 mg nanoparticles (Nanoparticle 1, or Nanoparticle 2, or Nanoparticle 3) were co-incubated for 12 hours in 2 mL RPMI 1640 complete medium, and then the incubated CD3⁺ CD69⁺ T cells (cell viability 85%) were sorted by flow cytometry, i.e., cancer-specific T cells that can recognize tumor antigens. The above sorted 1 million CD3⁺ CD69⁺ T cells were co-incubated with IL-2 (1000 U/mL), IL-7 (1000 U/mL) and αCD3 antibodies (10 ng/mL) and αCD28 antibodies (10 ng/mL) for 21 days in 10 mL of DMEM complete medium (37°C, 5% CO₂) to expand cancer-specific T cells (cell viability 85%).

### (4) Cancer-specific T cells for cancer treatment

Female C57BL/6 mice aged 6-8 weeks were selected as model mice. Each mouse was subcutaneously inoculated with 1.5×10⁵ B16F10 cells on the lower right side of the back on day 0. 500000 CD3⁺ cancer-specific T cells were intravenously injected on day 5, day 8, day 11, day 15, day 20, and day 25, separately. The methods of monitoring tumor growth and survival in mice were the same as above.

### (5) Experimental results

As shown in FIG. 21, the tumor volume of the mice in the PBS group in the figure grew rapidly. The tumor growth rate of the mice treated with cancer-specific T cells sorted and expanded by the Nanoparticles, Nanoparticle 2 and Nanoparticle 3 was significantly slower, the survival time was significantly prolonged, and some mice were cured. The cancer-specific T cells sorted by the Nanoparticle 3 were better than the cancer-specific T cells sorted by the Nanoparticle 2, indicating that the effect of the particles prepared by using the antigen components isolated and purified by heating was better than the effect of isolation and purification by salting-out using a specific reagent; the effect of the Nanoparticle 1 was better than that of the Nanoparticle 2 and the Nanoparticle 3, indicating that the effect of the particles prepared by using the antigen components isolated and purified by salting-out and heating at the same time was significantly better than that of the particles prepared by the antigen components isolated and purified by salting-out alone or the particles prepared by the antigen components isolated and purified by heating alone.

In this example, the antigen components mainly used protein and peptide components in whole-cell components, and in practical application, mRNA in the cell lysate can be isolated and extracted, and then mixed with the protein/peptide in the whole-cell components, and then used as a mixed antigen.

### Example 21: Cancer-specific T cells for cancer treatment

### (1) Preparation of antigen components

The cultured E.G7-OVA mouse T lymphoma cells were centrifuged at 400 g for 5 minutes, then resuspended in ultrapure water, then the cancer cells were lysed and the lysate components were dissolved using a 6 M aqueous solution of guanidine sulfate, then a saturated aqueous solution of ammonium sulfate was added until the precipitation was complete, then the supernatant was discarded, and the precipitate was dissolved again in the 6 M aqueous solution of guanidine sulfate to obtain protein and peptide components in the cancer cells dissolved in the 6 M aqueous solution of guanidine sulfate, i.e. antigen components 1.

The cultured E.G7-OVA mouse T lymphoma cells were centrifuged at 400 g for 5 minutes, then resuspended in ultrapure water, then the cancer cells were lysed and the lysate components were solubilized using a 3% aqueous Tween 80 solution, then a saturated aqueous solution of ammonium sulfate was added until the precipitation was complete, then the supernatant was discarded, and the precipitate was solubilized again using the 3% aqueous Tween 80 solution to obtain protein and peptide components dissolved in the 3% aqueous Tween 80 solution, i.e. antigen components 2.

The cultured E.G7-OVA mouse T lymphoma cells were centrifuged at 400 g for 5 minutes, then resuspended in ultrapure water, then the cancer cells were lysed and the lysate components were dissolved using a 6 M aqueous solution of guanidine sulfate, then a saturated aqueous solution of ammonium sulfate was added until the precipitation was complete, then the supernatant was discarded, and the precipitate was solubilized again using a 3% aqueous Tween 80 solution to obtain protein and peptide components dissolved in the 3% aqueous Tween 80 solution, i.e. antigen components 3.

The cultured E.G7-OVA mouse T lymphoma cells were centrifuged at 400 g for 5 minutes, then resuspended in ultrapure water, the cancer cells were lysed with a 3% aqueous Tween 80 solution, then the lysate components were dissolved with the 3% aqueous Tween 80 solution, then a saturated aqueous solution of ammonium sulfate was added until the precipitation was complete, then the supernatant was discarded, and the precipitate was dissolved again with a 6 M aqueous solution of guanidine sulfate to obtain protein and peptide components dissolved in the 6 M aqueous solution of guanidine sulfate, i.e., antigen components 4.

### (2) Preparation of nanoparticles

In this example, Nanoparticle 1 was prepared by a double emulsion method. The skeleton materials of Nanoparticle 1 were PLA (molecular weight of 30-40 KDa) and mannan-PEG2000-PLA (PLA molecular weight of 30-40 KDa), and the mass ratio of PLA (molecular weight of 30-40 KDa) and mannan-PEG2000-PLA (PLA molecular weight of 30-40 KDa) was 9:1. The immune adjuvants adopted were CpG2006 (Class B), CpG2216 (Class A) and Poly ICLC. In preparation, nanoparticles loaded with antigen components 1 and the adjuvants were prepared by the double emulsion method, and then 100 mg of nanoparticles were centrifuged at 13000 g for 25 minutes, resuspended with 10 mL of ultrapure water containing 4% trehalose and dried for 48 h to obtain the Nanoparticle 1, with an average particle size of about 500 nm. Approximately 5 µg of the protein and peptide components of the cancer cells were loaded, and 0.02 mg of CpG2006, CpG2216 and Poly ICLC each were loaded per 1 mg of PLGA Nanoparticle 1.

The preparation method of Nanoparticle 2 was the same as that of the Nanoparticle 1. But internally loaded were antigen components 2 and the adjuvants. The average particle size of the Nanoparticle 2 was about 500 nm, and approximately 5 µg of the protein and peptide components of the cancer cells were loaded, and 0.02 mg of CpG2006, CpG2216 and Poly ICLC each were loaded per 1 mg of PLGA Nanoparticle 2.

The preparation method of Nanoparticle 3 was the same as that of the Nanoparticle 1. But internally loaded were antigen components 3, the adjuvants and R8 peptide. The average particle size of the Nanoparticle 3 was about 500 nm, and approximately 5 µg of the protein and peptide components of the cancer cells were loaded, and 0.02 mg of CpG2006, CpG2216 and Poly ICLC each were loaded per 1 mg of PLGA Nanoparticle 3.

The preparation method of Nanoparticle 4 was the same as that of the Nanoparticle 1. But internally loaded were antigen components 4 and the adjuvants. The average particle size of the Nanoparticle 4 was about 500 nm, and approximately 5 µg of the protein and peptide components of the cancer cells were loaded, and 0.02 mg of CpG2006, CpG2216 and Poly ICLC each were loaded per 1 mg of PLGA Nanoparticle 4.

### (3) Preparation of cancer-specific T cells

Female C57BL/6 mice aged 6-8 weeks were selected and subcutaneously inoculated with 5×10⁵ E.G7-OVA mouse T lymphoma cells on day 0. Each mouse was intraperitoneally injected with 150 µg PD-1 antibodies on day 4, day 6, day 8, day 10, day 12, day 14, day 16, day 18, and day 20, separately. The mice were sacrificed on day 22, the peripheral blood of the mice was collected, then peripheral blood mononuclear cells (PBMCs) were isolated from the peripheral blood, and all CD25⁻ PBMCs were sorted using flow cytometry. Then 50 million CD25-PBMC cells and 5µg nanoparticles (Nanoparticle 1, or Nanoparticle 2, or Nanoparticle 3, or Nanoparticle 4) were co-incubated for 96 hours in 5 mL RPMI 1640 complete medium, and then the incubated CD3⁺ CD25⁺ T cells (cell viability 90%) were sorted by flow cytometry, i.e., cancer-specific T cells that can recognize tumor antigens. The above sorted 100000 CD8⁺ CD25⁺ T cells were co-incubated with IL-2 (1000 U/mL) and αCD3 antibodies (10 ng/mL) and αCD28 antibodies (10 ng/mL) for 28 days in 20 mL of DMEM complete medium (37°C, 5% CO₂) to expand cancer-specific T cells (cell viability 90%).

### (4) T cells for cancer treatment

Female C57BL/6 aged 6-8 weeks were selected as model mice to prepare colon cancer mice. Each mouse was subcutaneously inoculated with 5×10⁵ E. G7-OVA cells on the lower right side of the back on day 0. After inoculation of colon cancer cells, 500000 CD3⁺ cancer-specific T cells were intravenously injected on day 6, day 9, day 12, day 15, day 20, and day 25, separately. The methods of monitoring tumor growth and survival in mice were the same as above.

### (5) Experimental results

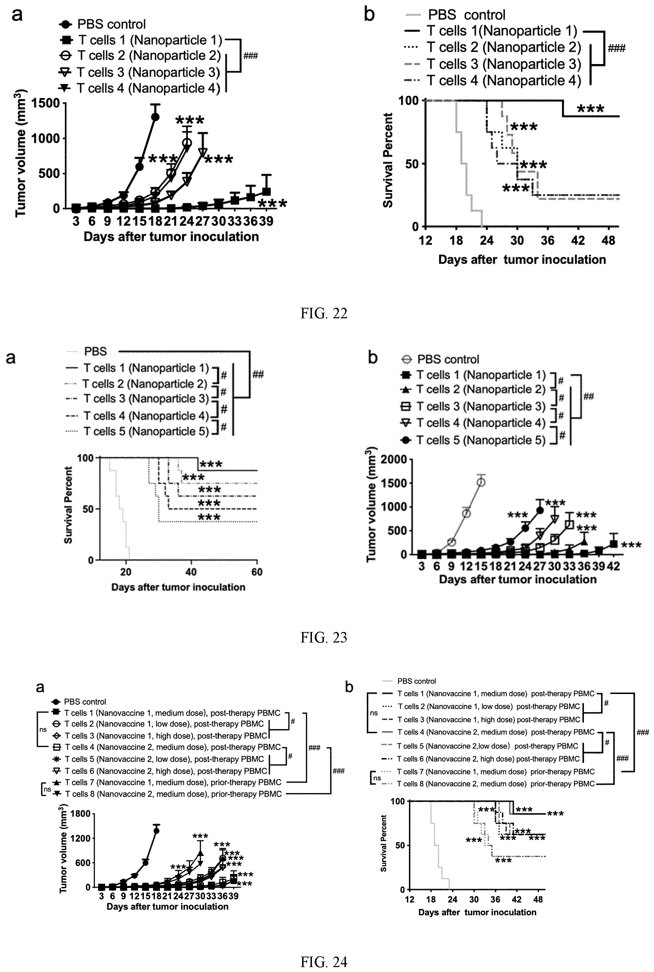

As shown in FIG. 22, the tumor growth rate of the T cell-treated mice was significantly slower and the survival time of the mice was significantly prolonged compared with the PBS control group. Moreover, the effect of cancer-specific T cells sorted and expanded using the Nanoparticle 1 was better than that using the Nanoparticle 2, Nanoparticle 3 and Nanoparticle 4, indicating that it was very critical to prepare cancer cell antigen components of nanoparticles by primary dissolution and secondary dissolution using appropriate dissolving agents.

### Example 22: T cells for cancer treatment

### Collection of antigen components

Each C57BL/6 mouse was subcutaneously inoculated with 1.5×10⁵ B16F10 cells on the back, and the mice were sacrificed and the tumor tissue removed when the tumors grew to approximately 1000 mm³. The tumor tissue was cut into pieces and then ground, an appropriate amount of ultrapure water was added, and freezing and thawing was repeated 5 times, accompanied by ultrasound to destroy and lyse the cells. After lysis, the lysate was centrifuged at a rotational speed of 5000 g for 5 minutes, and the supernatant was taken as water-soluble components that were soluble in pure water; and adding an 8 M urea solution to the obtained precipitated portion to dissolve the precipitated portion can convert the water-insoluble components that were insoluble in pure water into soluble components in the 8 M aqueous urea solution. A saturated aqueous solution of ammonium sulfate was added dropwise to the water-soluble components in the lysate, the obtained sample was centrifuged at 3000 g for 5 minutes after the precipitation was complete, and the precipitate was dissolved in the 8 M aqueous urea solution as a portion of the water-soluble components. The components obtained by precipitation after salting-out among the water-insoluble components in the lysate dissolved in the above 8 M aqueous urea solution and the water-soluble components dissolved in the 8 M urea were mixed at a mass ratio of 1: 2 to obtain antigen components 1 for preparing Nanoparticle 1.

Each C57BL/6 mouse was subcutaneously inoculated with 1.5×10⁵ B16F10 cells on the back, and the mice were sacrificed and the tumor tissue removed when the tumors grew to approximately 1000 mm³. The tumor tissue was cut into pieces and then ground, an appropriate amount of ultrapure water was added, and freezing and thawing was repeated 5 times, accompanied by ultrasound to destroy and lyse the cells. After lysis, the lysate was centrifuged at a rotational speed of 5000 g for 5 minutes, and the supernatant was taken as water-soluble components that were soluble in pure water; and adding an 8 M urea solution to the obtained precipitated portion to dissolve the precipitated portion can convert the water-insoluble antigens that were insoluble in pure water into soluble antigens in the 8 M aqueous urea solution. A saturated aqueous solution of ammonium carbonate dropwise to the water-soluble components in the lysate, the obtained sample was centrifuged at 3000 g for 5 minutes after the precipitation was complete, and the precipitate was dissolved in the 8 M aqueous urea solution as a portion of the water-soluble components. The components obtained by precipitation after salting-out among the water-insoluble components in the lysate dissolved in the above 8 M aqueous urea solution and the water-soluble components dissolved in the 8 M urea were mixed at a mass ratio of 1:2 to obtain antigen components 2 for preparing Nanoparticle 2.

Each C57BL/6 mouse was subcutaneously inoculated with 1.5×10⁵ B16F10 cells on the back, and the mice were sacrificed and the tumor tissue removed when the tumors grew to approximately 1000 mm³. The tumor tissue was cut into pieces and then ground, an appropriate amount of ultrapure water was added, and freezing and thawing was repeated 5 times, accompanied by ultrasound to destroy and lyse the cells. After lysis, the lysate was centrifuged at a rotational speed of 5000 g for 5 minutes, and the supernatant was taken as water-soluble components that were soluble in pure water; and adding an 8 M urea solution to the obtained precipitated portion to dissolve the precipitated portion can convert the water-insoluble antigens that were insoluble in pure water into soluble antigens in the 8 M aqueous urea solution. The water-insoluble components and the water-soluble components in the lysate dissolved in the above 8 M aqueous urea solution were mixed at a mass ratio of 1: 2 to obtain antigen components 3 for preparing Nanoparticle 3.

Each C57BL/6 mouse was subcutaneously inoculated with 1.5×10⁵ B16F10 cells on the back, and the mice were sacrificed and the tumor tissue removed when the tumors grew to approximately 1000 mm³. The tumor tissue was cut into pieces and then ground, an appropriate amount of ultrapure water was added, and freezing and thawing was repeated 5 times, accompanied by ultrasound to destroy and lyse the cells. After lysis, the lysate was centrifuged at a rotational speed of 5000 g for 5 minutes, and the supernatant was taken as water-soluble components that were soluble in pure water; and adding an 8 M urea solution to the obtained precipitated portion to dissolve the precipitated portion can convert the water-insoluble components that were insoluble in pure water into soluble components in the 8 M aqueous urea solution. A saturated aqueous solution of ammonium sulfate was added dropwise to the water-soluble components in the lysate, the obtained sample was centrifuged at 3000 g for 5 minutes after the precipitation was complete, and the precipitate was solubilized with a 5% PEG5000 aqueous solution to be used as a portion of the water-soluble components. The components obtained by precipitation after salting-out among the water-insoluble components in the lysate dissolved in the above 8 M aqueous urea solution and the water-soluble components dissolved in the 5% PEG5000 aqueous solution were mixed at a mass ratio of 1: 2 to obtain antigen components 4 for preparing Nanoparticle 4.

Each C57BL/6 mouse was subcutaneously inoculated with 1.5×10⁵ B16F10 cells on the back, and the mice were sacrificed and the tumor tissue removed when the tumors grew to approximately 1000 mm³. The tumor tissue was cut into pieces and then ground, then an appropriate amount of ultrapure water was added, and freezing and thawing was repeated 5 times, accompanied by ultrasound to destroy and lyse the cells. After lysis, the lysate was centrifuged at a rotational speed of 5000 g for 5 minutes, and the supernatant was taken as water-soluble components that were soluble in pure water; and adding the 5% PEG5000 aqueous solution to the obtained precipitated portion to solubilize the precipitated portion can obtain components of the water-insoluble components that were soluble in the 5% PEG5000 aqueous solution. A saturated aqueous solution of ammonium sulfate was added dropwise to the water-soluble components in the lysate, the obtained sample was centrifuged at 3000 g for 5 minutes after the precipitation was complete, and the precipitate was solubilized with a 5% PEG5000 aqueous solution to be used as a portion of the water-soluble components. The components obtained by precipitation after salting-out among the water-insoluble components in the lysate dissolved in the above 5% PEG5000 aqueous solution and the water-soluble components dissolved in the 5% PEG5000 aqueous solution were mixed at a mass ratio of 1: 2 to obtain antigen components 5 for preparing Nanoparticle 5.

### (2) Preparation of nanoparticles

Nanoparticle 1 in this example was prepared by a double emulsion method in a solvent evaporation method. The molecular weight of the antigen-delivering nanoparticle preparation material PLGA was 10 KDa to 20 KDa, and the immune adjuvant adopted was poly(I:C). The cell antigen components 1 and adjuvants were first loaded inside the nanoparticles by a double emulsion method, and then 100 mg of the nanoparticles were centrifuged at 13000 g for 20 minutes, and resuspended with 10 mL of ultrapure water containing 4% trehalose and freeze-dried for 48 h. The average particle size of the Nanoparticle 1 was about 200 nm, and approximately 15 µg of protein or peptide components were loaded, and 0.2 mg of poly(I:C) was loaded per 1 mg of PLGA nanoparticles.

The preparation method, materials and preparation steps of Nanoparticle 2 were the same as those of the Nanoparticle 1. The preparation method was as previously described. The cell antigen components 2 and adjuvants were first loaded inside the nanoparticles, and then 100 mg of the nanoparticles were centrifuged at 13000 g for 20 minutes, and resuspended with 10 mL of ultrapure water containing 4% trehalose and freeze-dried for 48 h. The average particle size of the Nanoparticle 2 was about 200 nm, and approximately 15 µg of protein or peptide components were loaded, and 0.2 mg of poly(I:C) was loaded per 1 mg of PLGA nanoparticles.

The preparation method, materials and preparation steps of Nanoparticle 3 were the same as those of the Nanoparticle 1. The cell antigen components 3 and adjuvants were first loaded inside the nanoparticles, and then 100 mg of the nanoparticles were centrifuged at 13000 g for 20 minutes, and resuspended with 10 mL of ultrapure water containing 4% trehalose and freeze-dried for 48 h. The average particle size of the Nanoparticle 3 was about 200 nm, and approximately 15 µg of protein or peptide components were loaded, and 0.2 mg of poly(I:C) was loaded per 1 mg of PLGA nanoparticles.

The preparation method, materials and preparation steps of Nanoparticle 4 were the same as those of the Nanoparticle 1. The preparation method was as previously described. The cell antigen components 4 and adjuvants were first loaded inside the nanoparticles, and then 100 mg of the nanoparticles were centrifuged at 13000 g for 20 minutes, and resuspended with 10 mL of ultrapure water containing 4% trehalose and freeze-dried for 48 h. The average particle size of the Nanoparticle 4 was about 200 nm, and approximately 15 µg of protein or peptide components were loaded, and 0.2 mg of poly(I:C) was loaded per 1 mg of PLGA nanoparticles.

The preparation method, materials and preparation steps of Nanoparticle 5 were the same as those of the Nanoparticle 1. The cell antigen components 5 and adjuvants were first loaded inside the nanoparticles, and then 100 mg of the nanoparticles were centrifuged at 13000 g for 20 minutes, and resuspended with 10 mL of ultrapure water containing 4% trehalose and freeze-dried for 48 h. The average particle size of the Nanoparticle 5 was about 200 nm, and approximately 15 µg of protein or peptide components were loaded, and 0.2 mg of poly(I:C) was loaded per 1 mg of PLGA nanoparticles.

### (3) Preparation of T cells

Female C57BL/6 mice aged 6-8 weeks were selected and subcutaneously inoculated with 1.5×10⁵ B16F10 cells on day 0. Each mouse was intraperitoneally injected with 150 µg PD-L1 antibodies on day 4, day 6, day 8, day 10, day 12, day 14, day 16, day 18, and day 20, separately. The mice were sacrificed on day 22, PBMCs of the mice were collected, the PBMCs were cultured *in vitro* for 12 hours, and then all CD69⁻ PBMCs were sorted using flow cytometry. Then, 2.5 million CD69⁻ PBMC cells and 500 µg nanoparticles (Nanoparticle 1, or Nanoparticle 2, or Nanoparticle 3, or Nanoparticle 4, or Nanoparticle 5) were co-incubated for 36 hours in 10 mL RPMI 1640 complete medium, and then the incubated CD3⁺ CD69⁺ T cells (cell viability 90%) were sorted by flow cytometry, i.e., cancer-specific T cells that can recognize tumor antigens. The above sorted 100000 CD3⁺ CD69⁺ T cells were co-incubated with IL-2 (1000 U/mL) and αCD3 antibodies (10 ng/mL) and αCD28 antibodies (10 ng/mL) for 21 days in 10 mL of DMEM complete medium (37°C, 5% CO₂) to expand cancer-specific T cells (cell viability 90%).

### (4) T cells for cancer treatment

Female C57BL/6 aged 6-8 weeks were selected as model mice to prepare melanoma-bearing mice, and each mouse was subcutaneously inoculated with 1.5×10⁵ B16F10 cells on the lower right side of the back on day 0. Before tumor inoculation, mice were subcutaneously inoculated with 100000 cancer-specific T cells obtained by different nanoparticle-assisted sorting or 100 µL PBS on day 3, day 6, day 9, day 14, and day 20, separately. The tumor growth rate of the mice and the survival of the mice were monitored.

### (4) Experimental results

As shown in FIG. 23, the tumor volume of the mice in the PBS group grew rapidly. The tumor growth rates of the mice treated by the cancer-specific T cells sorted and expanded by Nanoparticle 1, Nanoparticle 2, Nanoparticle 3, Nanoparticle, and Nanoparticle 5 were significantly slower and the survival time was significantly prolonged. The effect of Nanoparticle 1 was better than that of Nanoparticle 2, Nanoparticle 3, Nanoparticle 4, and Nanoparticle 5, indicating that the effect of isolating and purifying protein and peptide antigen components in the water-soluble components by salting-out using ammonium sulfate was better than that using ammonium carbonate, and the effect of isolating and purifying a portion of components in the water-soluble components was better than that of directly using the water-soluble components. Moreover, the precipitation effect of dissolving water-soluble components using urea for salting-out treatment was better than that using PEG.

### Example 23: Sorted and expanded T cells to kill breast cancer cells

This example used tumor tissue from multiple breast cancer patients to prepare nanoparticles.

### (1) Preparation of antigen components

The tumor tissue obtained from surgical resection of 12 patients with triple-negative breast cancer was collected. The tumor tissue of each patient was cut into pieces and then ground, and added to ultrapure water after passing through a cell strainer, and then freezing and thawing was repeated 5 times, accompanied by ultrasound to lyse cancer cells in the tumor tissue. 1 mg/mL of nuclease was added to the lysed cancer cells to degrade the nucleic acid in the lysate, then the nuclease was inactivated by heating at 95°C for 10 minutes, then the supernatant was collected by centrifugation at 5000 g for 5 minutes, which was water-soluble components, and the precipitate was dissolved by an 8 M aqueous urea (containing 0.01 M arginine) solution, which was water-insoluble components. The water-soluble components of 12 breast cancer patients were mixed at a mass ratio of 1:1 to obtain a water-soluble component mixture; and the water-insoluble components of 12 breast cancer patients were mixed at a mass ratio of 1:1 to obtain a water-insoluble component mixture. The antigen components 1 of the Nanoparticle 1 (NP1) were prepared by mixing the water-soluble component mixture and the water-insoluble component mixture at a mass ratio of 5:1.

The tumor tissue obtained from surgical resection of another patient A with triple-negative breast cancer was collected. Patient A was not included in the above 12 cancer patients. The tumor tissue was cut into pieces and then ground, and added to ultrapure water after passing through a cell strainer, and then freezing and thawing was repeated 5 times, accompanied by ultrasound to lyse cancer cells. 1 mg/mL of nuclease was added to the lysed cancer cells to degrade the nucleic acid in the lysate, then the nuclease was inactivated by heating at 95°C for 10 minutes, then the supernatant was collected by centrifugation at 5000 g for 5 minutes, which was water-soluble components, the precipitate was dissolved by an 8 M aqueous urea (containing 0.01 M arginine) solution, which was water-insoluble components, and the water-soluble components and the water-insoluble components were mixed at a mass ratio of 5:1 to obtain antigen components 2 for preparing Nanoparticle 2 (NP2).

### (2) Preparation of nanoparticles

In this example, the nanoparticles were prepared by a double emulsion method. The molecular weight of the Nanoparticle 1 backbone material PLGA was 10 KDa to 20KDa, and the immune adjuvants adopted were CpG2395 (class C), CpG1018 (class B) and Poly ICLC. During preparation, the antigen components 1 and adjuvants were first loaded inside the particles, and then 100 mg nanoparticles were centrifuged at 12000 g for 20 minutes, resuspended with 10 mL of ultrapure water containing 4% trehalose and dried for 48 h for later use. The particle size of the nanoparticles was about 280 nm, and approximately 950 µg of protein or peptide components were loaded, and 0.02 mg of CpG2395, CpG1018 and Poly(I:C) each were loaded per 1 mg of PLGA nanoparticles.

The molecular weight of the Nanoparticle 2 backbone material PLGA was 10 KDa to 20KDa, and the immune adjuvants adopted were CpG2395 (class C), CpG1018 (class B) and Poly ICLC. During preparation, the nanoparticles internally loaded with the antigen components 2 and adjuvants were prepared by the double emulsion method, and then 100 mg of the nanoparticles were centrifuged at 12000 g for 20 minutes, and resuspended using 10 mL of ultrapure water containing 4% trehalose and then dried for 48 h for later use. The particle size of the nanoparticles was about 280 nm, and approximately 950 µg of protein or peptide components were loaded, and 0.02 mg of CpG2395, CpG1018 and Poly(I:C) each were loaded per 1 mg of PLGA nanoparticles.

### (3) Isolation and expansion of cancer-specific T cells

Patient A was treated after surgical resection of the tumor tissue, and the therapeutic effect was good after treatment, and the tumor mass gradually became smaller. 10 mL of peripheral blood was drawn from the patient before or after immunotherapy, separately. PBMCs were then isolated from peripheral blood and cultured *in vitro* for 12 hours, and then subsequent experiments were performed.

Pre- or post-immunotherapy PBMCs (6 million cells), IL-7 (10 ng) and IL-15 (10 ng) were co-incubated with 20 µg nanoparticles (Nanoparticle 1 or Nanoparticle 2) for 72 hours in 2 mL AIM V serum-free medium (37°C, 5% CO₂). After incubation, the cells were harvested and CD3⁺ CD25⁺ T cells were sorted using flow cytometry (cell viability 85%), and 300000 T cells obtained from the above sorting were co-incubated with IL-2 (1000 U/mL) and αCD3 antibodies (10 ng/mL) and αCD28 antibodies (10 ng/mL) for 21 days in 10 mL of DMEM complete medium (37°C, 5%CO₂) to expand cancer-specific T cells (cell viability 85%).

Alternatively, PBMCs (6 million), IL-7 (10 ng), IL-15 (10 ng) after immunotherapy were co-incubated with 5 ng nanoparticles (Nanoparticle 1 or Nanoparticle 2, separately) for 72 hours in 2 mL AIM V serum-free medium (37°C, 5% CO₂). After incubation, the cells were harvested and CD3⁺ CD25⁺ T cells were sorted using flow cytometry (cell viability 85%), and 300000 T cells obtained from the above sorting were co-incubated with IL-2 (1000 U/mL) and αCD3 antibodies (10 ng/mL) and αCD28 antibodies (10 ng/mL) for 21 days in 10 mL of DMEM complete medium (37°C, 5%CO₂) to expand cancer-specific T cells (cell viability 85%).

Alternatively, PBMCs (6 million), IL-7 (10 ng), IL-15 (10 ng) after immunotherapy were co-incubated with 100 mg nanoparticles (Nanoparticle 1 or Nanoparticle 2, separately) for 72 hours in 2 mL AIM V serum-free medium (37°C, 5% CO₂). After incubation, the cells were harvested and CD3⁺ CD25⁺ T cells were sorted using flow cytometry (cell viability 85%), and 300000 T cells obtained from the above sorting were co-incubated with IL-2 (1000 U/mL) and αCD3 antibodies (10 ng/mL) and αCD28 antibodies (10 ng/mL) for 21 days in 10 mL of DMEM complete medium (37°C, 5%CO₂) to expand cancer-specific T cells (cell viability 85%).

### (4) Cancer-specific T cells to kill cancer cells

Nude mice aged 6-8 weeks were selected, and on day 0, each nude mouse was subcutaneously inoculated with 5×10⁵ cancer cells expanded from tumor tissue obtained by surgical resection of patient A. After tumor inoculation, the mice were subcutaneously injected with 300000 cancer-specific T cells obtained by different nanoparticle-assisted sorting and expansion or 100 µL PBS on day 3, day 6, day 9, day 14, and day 20, separately. The tumor growth rate of the mice was monitored.

### (5) Experimental results

As shown in FIG. 24, compared with the control group, the cancer-specific T cells obtained by sorting and expansion of the 2 kinds of nanoparticles can effectively control the growth of tumor tissue, and the effects of the Nanoparticle 1 and the Nanoparticle 2 were similar. Moreover, the effect of co-incubation with the appropriate concentration (10 µg/mL) of nanoparticles to assist sorting was significantly better than using too low concentration (2.5 ng/mL) or too high concentration (50 mg/mL) of nanoparticles. Moreover, the cancer-specific T cells sorted and expanded after immunotherapy had a better effect than the cancer-specific T cells sorted and expanded before immunotherapy. Moreover, it was better to use peripheral blood immune cells after immunotherapy for sorting and expansion.

### Example 24: Sorted and expanded T cells to kill esophageal cancer cells

### (1) Preparation of antigen components

Tumor tissue was collected from 5 patients with esophageal cancer. The tumor tissue of the 5 patients was mixed according to the mass ratio of 1:1:1:1:1, then cut into pieces and ground, then passed through a cell strainer and then added with an appropriate amount of an 8 M aqueous urea solution to lyse the above cells, and the 8 M aqueous urea solution was used to completely dissolve tumor tissue lysate components to obtain antigen components 1 for preparing Nanoparticle 1 and Nanoparticle 2.

The tumor tissue of another patient A with esophageal cancer was collected. Patient A was not included in the above 5 cancer patients. The tumor tissue of patient A was cut into pieces and ground, passed through a cell strainer and then added with an appropriate amount of an 8 M aqueous urea solution to lyse the above cells, and the 8 M aqueous urea solution was used to completely dissolve tumor tissue lysate components to obtain antigen components 2 for preparing Nanoparticle 3 and Nanoparticle 4.

### (2) Preparation of nanoparticles

The Nanoparticle 1 (NP1) in this example was prepared by a double emulsion method. The nanoparticle preparation material adopted was PLGA with a molecular weight of 10 KDa to 30 KDa, the loaded adjuvants were poly I:C and CpG7909, and the loaded substance which increased lysosome immune escape was KALA peptide. The preparation method was as previously described. The antigen components, adjuvants and KALA peptide were first loaded inside the nanoparticles, and then 100 mg nanoparticles were centrifuged at 12000 g for 25 min and freeze-dried for 48 h after resuspension using 10 mL of ultrapure water containing 4% trehalose. The average particle size of the nanoparticles was about 250 nm, and approximately 100 µg of protein or peptide components of the tumor tissue were loaded, 0.01 mg of poly I:C and CpG7909 each were loaded, and 0.15 mg of KALA peptide was loaded per 1 mg of PLGA nanoparticles.

The preparation materials and method of Nanoparticle 2 (NP2) were the same. The particle size of NP2 was about 250 nm, and approximately 0.01 µg of protein or peptide components of the tumor tissue were loaded, 0.01 mg of poly I:C and CpG7909 each were loaded, and 0.15 mg of KALA peptide was loaded per 1 mg of PLGA nanoparticles.

The preparation materials and method of Nanoparticle 3 (NP3) were the same. The particle size of NP3 was about 250 nm, and approximately 100 µg of protein or peptide components of the tumor tissue were loaded, 0.01 mg of poly I:C and CpG7909 each were loaded, and 0.15 mg of KALA peptide was loaded per 1 mg of PLGA nanoparticles.

The preparation materials and method of Nanoparticle 4 (NP4) were the same. The particle size of NP4 was about 250 nm, and approximately 0.01 µg of protein or peptide components of the tumor tissue were loaded, 0.01 mg of poly I:C and CpG7909 each were loaded, and 0.15 mg of KALA peptide was loaded per 1 mg of PLGA nanoparticles.

### (3) Detection of cancer-specific T cells

Patient A underwent cancer immunotherapy after surgical resection of the tumor tissue, and the therapeutic effect was good after treatment, and the tumor mass gradually became smaller. 12 mL of peripheral blood was drawn from the patient after immunotherapy. PBMCs were then isolated from the peripheral blood. PBMCs (10 million) and 100 µg nanoparticles (Nanoparticle 1, or Nanoparticle 2, or Nanoparticle 3, or Nanoparticle 4) were co-incubated for 96 hours in 10 mL AIM V serum-free medium (37°C, 5% CO₂). After incubation, the cells were harvested and labeled with CD3 and HLA-DR flow antibodies, and then CD3⁺ HLA-DR⁺ T cells (cell viability 85%), i.e. cancer-specific T cells, were sorted using flow cytometry. The T cells sorted above were co-incubated with IL-2 (1000 U/mL) and αCD3 antibodies (10 ng/mL) and αCD28 antibodies (10 ng/mL) for 21 days in 10 mL of DMEM complete medium (37°C, 5% CO₂) to expand cancer-specific T cells (cell viability 85%).

### (4) Sorted and expanded T cells to kill cancer cells

Nude mice aged 6-8 weeks were selected, and on day 0, each nude mouse was subcutaneously inoculated with5×10⁵ cancer cells expanded from tumor tissue obtained by surgical resection of patient A. After tumor inoculation, the mice were subcutaneously injected with 500000 cancer-specific T cells obtained by different nanoparticle-assisted sorting and expansion or 100 µL PBS on day 3, day 6, day 9, day 14, and day 20, separately. The tumor growth rate of the mice was monitored.

### (4) Experimental results

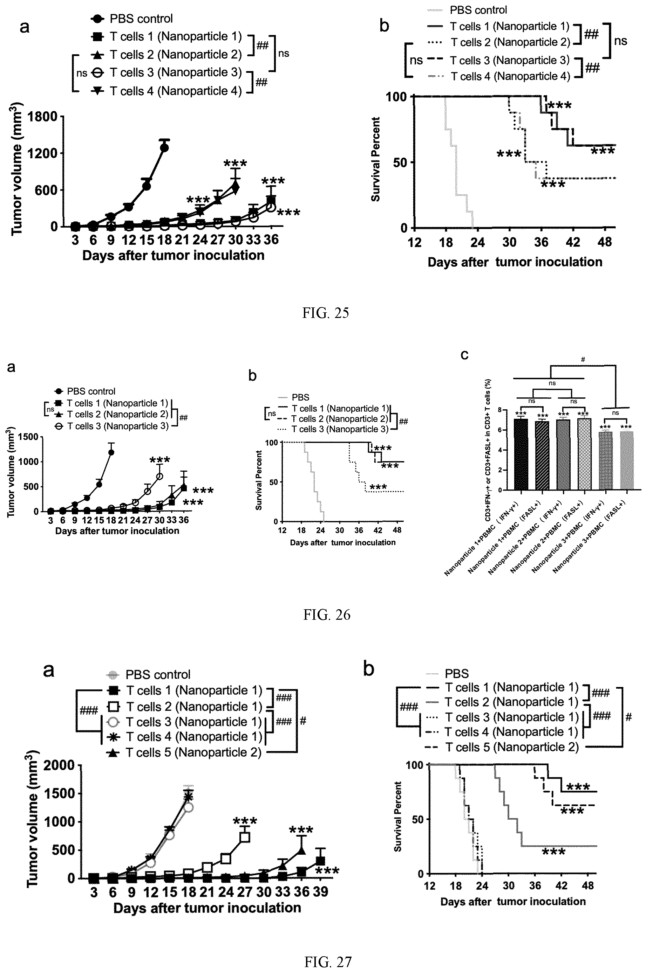

As shown in FIG. 25, the effect of cancer-specific T cells obtained by sorting and expansion of nanoparticles prepared by loading a mixture of the tumor tissue of multiple allogeneic cancer patients was not significantly different from that of nanoparticles obtained by assisted sorting and expansion using the autologous tumor tissue of the cancer patients. Moreover, nanoparticles loaded with appropriate antigen components were better than nanoparticles loaded with only low levels of antigen components.

### Example 25: Sorted and expanded T cells to kill lung cancer cells

In this example, water-soluble components and water-insoluble components in a plurality of cancer cell lines of human lung cancer were loaded onto nanoparticles, and then cancer-specific T cells in peripheral immune organs of lung cancer patients were sorted and expanded with the nanoparticles.

### (1) Preparation of antigen components

Human lung cancer cell lines A549 cells, H1299 cells, PC9 cells, H1437 cells, H226 cells, HCC1588 cells, H2170 cells, and H520 cells were cultured separately.

After the 8 kinds of cells were collected separately, A549 cells, H1299 cells, PC9 cells, H1437 cells, H226 cells, HCC1588 cells, H2170 cells, and H520 cells were mixed according to a cell number ratio of 20:20:2:2:2:1:1:1:1, then the medium was removed after centrifugation, the cell precipitate was resuspended with ultrapure water, and then repeatedly frozen and thawed 5 times, and the cancer cells were more thoroughly lysed by ultrasonication during the freezing and thawing process. After the cells were lysed, the lysate was centrifuged at a rotational speed of 5000 g for 5 minutes, and the supernatant was taken as water-soluble components that were soluble in pure water; and adding 6 M guanidine sulfate to the obtained precipitated portion to dissolve the precipitated portion can convert the water-insoluble components that were insoluble in pure water into soluble components in the 6 M aqueous solution of guanidine sulfate. After the water-soluble components in the lysate were heated for 10 minutes at 95°C, the obtained sample was centrifuged at 3000 g for 5 minutes, the precipitated protein and peptide components were dissolved in the 6 M aqueous solution of guanidine sulfate, the mRNA in the supernatant was extracted using an mRNA extraction kit, and then the mRNA component was mixed with the protein and peptide components dissolved in 6 M guanidine sulfate to obtain antigen components in the water-soluble components; and then a saturated ammonium sulfate solution was added to the water-insoluble components dissolved by 6 M guanidine sulfate to salt out the protein and peptide components, and 6 M guanidine sulfate was used for secondary dissolution of the precipitate obtained by salting-out to obtain antigen components in the water-insoluble components. The above antigen components of the water-insoluble components and the antigen components of the water-soluble components were mixed at a mass ratio of 1:1 to obtain antigen components 1 for preparing Nanoparticle 1 (NP1).

Alternatively, a tumor tissue sample surgically resected from patient A with non-small cell lung cancer was collected. The immunotherapy effect of the patient with non-small cell lung cancer was good. The tumor tissue was cut into pieces and filtered through a cell screen, then resuspended with ultrapure water and repeatedly frozen and thawed 5 times. During the freezing and thawing process, ultrasonication was used to more thoroughly lyse the cancer cells. After the cells were lysed, the lysate was centrifuged at a rotational speed of 5000 g for 5 minutes, and the supernatant was taken as water-soluble components that were soluble in pure water; and adding 6 M guanidine sulfate to the obtained precipitated portion to dissolve the precipitated portion can convert the water-insoluble components that were insoluble in pure water into soluble components in the 6 M aqueous solution of guanidine sulfate. After the water-soluble components in the lysate were heated for 10 minutes at 95°C, the obtained sample was centrifuged at 3000 g for 5 minutes, the precipitated protein and peptide components were dissolved in the 6 M aqueous solution of guanidine sulfate, the mRNA in the supernatant was extracted using an mRNA extraction kit, and then the mRNA component was mixed with the protein and peptide components dissolved in 6 M guanidine sulfate to obtain antigen components in the water-soluble components; and then a saturated ammonium sulfate solution was added to the water-insoluble components dissolved by 6 M guanidine sulfate to salt out the protein and peptide components, and 6 M guanidine sulfate was used for secondary dissolution of the precipitate obtained by salting-out to obtain antigen components in the water-insoluble components. The above antigen components of the water-insoluble components and the antigen components of the water-soluble components were mixed at a mass ratio of 1:1 to obtain antigen components 2 for preparing Nanoparticle 2 (NP2).

Alternatively, a tumor tissue sample from another patient B with non-small cell lung cancer was collected. The tumor tissue was cut into pieces and filtered through a cell screen, then resuspended with ultrapure water and repeatedly frozen and thawed 5 times. During the freezing and thawing process, ultrasonication was used to more thoroughly lyse the cancer cells. After the cells were lysed, the lysate was centrifuged at a rotational speed of 5000 g for 5 minutes, and the supernatant was taken as water-soluble components that were soluble in pure water; and adding 6 M guanidine sulfate to the obtained precipitated portion to dissolve the precipitated portion can convert the water-insoluble components that were insoluble in pure water into soluble components in the 6 M aqueous solution of guanidine sulfate. After the water-soluble components in the lysate were heated for 10 minutes at 95°C, the obtained sample was centrifuged at 3000 g for 5 minutes, the precipitated protein and peptide components were dissolved in the 6 M aqueous solution of guanidine sulfate, the mRNA in the supernatant was extracted using an mRNA extraction kit, and then the mRNA component was mixed with the protein and peptide components dissolved in 6 M guanidine sulfate to obtain antigen components in the water-soluble components; and then a saturated ammonium sulfate solution was added to the water-insoluble components dissolved by 6 M guanidine sulfate to salt out the protein and peptide components, and 6 M guanidine sulfate was used for secondary dissolution of the precipitate obtained by salting-out to obtain antigen components in the water-insoluble components. The above antigen components of the water-insoluble components and the antigen components of the water-soluble components were mixed at a mass ratio of 1:1 to obtain antigen components 3 for preparing Nanoparticle 3 (NP3).

### (2) Preparation of nanoparticles loaded with whole-cell components

The Nanoparticle 1 (NP1) in this example was prepared by a double emulsion method in a solvent evaporation method. The molecular weight of the nanoparticle preparation material PLGA adopted was 20 KDa to 40 KDa, and the adjuvants adopted were poly(I: C), CpG7909 and CpG2395. The preparation method was as previously described. The antigen components and adjuvants were first loaded inside the nanoparticles, and then 100 mg of the nanoparticles were centrifuged at 10000 g for 20 minutes, and resuspended with 10 mL of ultrapure water containing 4% trehalose and freeze-dried for 48 h. The average particle size of the Nanoparticle 1 was about 380 nm, and approximately 800 µg of protein or peptide components were loaded, and 0.02 mg of poly(I:C), CpG7909 and CpG2395 each were loaded per 1 mg of PLGA nanoparticles.

The preparation process of Nanoparticle 2 (NP2) was the same as that of the Nanoparticle 1. The average particle size of the Nanoparticle 2 was about 380 nm, and approximately 800 µg of protein or peptide components were loaded, and 0.02 mg of poly(I:C), CpG7909 and CpG2395 each were loaded per 1 mg of PLGA nanoparticles.

The preparation process of Nanoparticle 3 (NP3) was the same as that of the Nanoparticle 1. The average particle size of the Nanoparticle 3 was about 380 nm, and approximately 800 µg of protein or peptide components were loaded, and 0.02 mg of poly(I:C), CpG7909 and CpG2395 each were loaded per 1 mg of PLGA nanoparticles.

### (3) Sorting and expansion of T cells

Patient A with non-small cell lung cancer showed tumor tissue shrinkage after immunotherapy. 10 mL of peripheral blood of patient A with non-small cell lung cancer was drawn two weeks after immunotherapy of patient A with non-small cell lung cancer. Peripheral blood mononuclear cells (PBMCs) were isolated from 10 mL peripheral blood of the patient with non-small cell lung cancer using gradient centrifugation.

The Nanoparticle 1 (0.5 mg) or Nanoparticle 2 (0.5 mg) or Nanoparticle 3 (0.5 mg) were co-incubated with PBMCs (10 million cells ) for 12 hours in 1 mL of AIM V serum-free medium (37°C, 5% CO₂). The cells were then harvested and centrifuged at 400 g for 5 minutes, the cells were resuspended in PBS and then T cells were first treated with Fc block to avoid non-specific loading.

Then half of the cells were extracellularly stained with CD3 antibodies and FASL antibodies, then the cells were fixed with 4% paraformaldehyde and a membrane rupture agent was used to rupture cell membranes, and the T cells were intracellularly stained with FN-y antibodies. Then T cells in the sample were detected by flow cytometry. The proportion of CD3⁺ T cells that can secrete IFN-γ and T cells that can express FASL after activation in all CD3⁺ T cells was analyzed.

The other half of the cells were directly sorted for CD3⁺ FASL⁺ T cells (cell viability 85%), and the sorted 500000 T cells were co-incubated with IL-2 (1000 U/mL) and αCD3 antibodies (10 ng/mL) and αCD28 antibodies (10 ng/mL) for 28 days in 10 mL of DMEM complete medium (37°C, 5% CO₂) to expand cancer-specific T cells (cell viability 85%).

### (4) Sorted and expanded T cells to kill cancer cells

Nude mice aged 6-8 weeks were selected, and on day 0, each nude mouse was subcutaneously inoculated with 5×10⁵ cancer cells obtained by expansion of the cancer cells in patient A on the lower right side of the back. After tumor inoculation, the mice were subcutaneously injected with 500000 cancer-specific T cells obtained by different nanoparticle-assisted sorting and expansion or 100 µL PBS on day 3, day 6, day 9, day 14, and day 20, separately. The tumor growth rate of the mice and the survival of the mice were monitored.

### (5) Experimental results

As shown in FIG. 26, the cancer-specific T cells obtained by sorting and expansion of Nanoparticle 1 and Nanoparticle 2 had the same effect on cancer-specific T cells, and the T cells that can secrete IFN-γ and T cells that can express FASL after activation overlapped, and the cancer-specific T cells obtained by sorting and expansion according to the present disclosure can effectively kill cancer cells. T cells sorted the expanded using nanoparticles prepared from the tumor tissue of the cancer patient were better than T cells sorted the expanded using nanoparticles prepared from the tumor tissue of a single other patient; and the effect of nanoparticles prepared using antigen components of multiple cancer cell lines was the same as that of nanoparticles prepared from the patient's own tumor tissue.

### Example 26: Cancer-specific T cells for melanoma treatment

### (1) Collection of antigen components

The cultured B16-F10, B16-F1, S91, ME, K735, B16-BL6, and MEC57 cancer cell lines were collected, and the above cancer cell lines were mixed in a quantity ratio of 1:1:1:1:1:1:1, then the mixed cells were lysed using 0.2 M arginine and 0.1 M semicarbazide hydrochloride, all lysed components were dissolved using 0.2 M arginine and 0.1 M semicarbazide hydrochloride after lysis, then a saturated aqueous solution of ammonium sulfate was added dropwise thereto, the obtained sample was centrifuged at 3000 g for 5 minutes after the precipitation was complete, the precipitate was dissolved in 0.2 M arginine and a 0.1 M aqueous semicarbazide hydrochloride solution for later use, the supernatant was heated at 95°C for 10 minutes, the obtained sample was centrifuged at 3000 g for 5 minutes, and the precipitate was dissolved in 0.2 M arginine and the 0.1 M aqueous semicarbazide hydrochloride solution after the supernatant was discarded; and then the precipitate after salting-out dissolved with 0.2 M arginine and 0.1 M semicarbazide hydrochloride and the precipitate after heating were combined to obtain antigen components 1 for preparing Nanoparticle 1.

Alternatively, the cultured B16-F10 cancer cell line was collected, and the above cancer cells were lysed using 0.2 M arginine and 0.1 M semicarbazide hydrochloride, after lysis, all lysed components were dissolved using 0.2 M arginine and 0.1 M semicarbazide hydrochloride, then the saturated aqueous solution of ammonium sulfate was added dropwise thereto, the obtained sample was centrifuged at 3000 g for 5 minutes after the precipitation was complete, the precipitate was dissolved in 0.2 M arginine and a 0.1 M aqueous semicarbazide hydrochloride solution for later use, the supernatant was heated at 95 C for 10 minutes, the obtained sample was centrifuged at 3000 g for 5 minutes, and the precipitate was dissolved in 0.2 M arginine and the 0.1 M aqueous semicarbazide hydrochloride solution after the supernatant was discarded; and then the precipitate after salting-out dissolved with 0.2 M arginine and 0.1 M semicarbazide hydrochloride and the precipitate after heating were combined to obtain antigen components 2 for preparing Nanoparticle 2.

### (2) Preparation of nanoparticles

The Nanoparticle 1 in this example was prepared by a double emulsion method. The molecular weight of the particle preparation material PLGA was 10 KDa to 20 KDa, and the immune adjuvants adopted were poly(I:C), CpG7909 and CpG2395. The preparation method was as previously described. The antigen components 1 and adjuvants were first loaded inside the nanoparticles, and then 100 mg of the nanoparticles were centrifuged at 12000 g for 20 minutes, and resuspended with 10 mL of ultrapure water containing 4% trehalose and freeze-dried for 48 h. The average particle size of the Nanoparticle 1 (Nanovaccine 1) was about 280 nm, and approximately 250 µg of protein or peptide components were loaded, and 0.01 mg of poly(I:C), CpG7909 and CpG2395 each were loaded per 1 mg of PLGA particles.

The preparation materials and preparation method of the Nanoparticle 2 in this example were the same as those of the Nanoparticle 1. The antigen components 2 and adjuvants were first loaded inside the nanoparticles, and then 100 mg of the nanoparticles were centrifuged at 12000 g for 20 minutes, and resuspended with 10 mL of ultrapure water containing 4% trehalose and freeze-dried for 48 h. The average particle size of the Particle 2 was about 280 nm, and approximately 250 µg of protein or peptide components were loaded, and 0.01 mg of poly(I:C), CpG7909 and CpG2395 each were loaded per 1 mg of PLGA particles.

### (3) Preparation of cancer-specific T cells

Female C57BL/6 mice aged 6-8 weeks were selected and subcutaneously inoculated with 1.5×10⁵ B16F10 mouse melanoma cancer cells on day 0. Each mouse was intraperitoneally injected with 150 µg PD-L1 antibodies on day 6, day 8, day 10, day 12, day 14, day 16, day 18, day 20, separately. The mice were sacrificed on day 22, the peripheral blood of the mice was collected, and then mouse PBMCs were prepared to obtain mixed immune cells co-incubated with nanoparticles.

50 million of the above mixed immune cells and 4 mg Nanoparticle 1 were co-incubated for 6 hours in 10 mL RPMI 1640 complete medium, and then the incubated CD3⁺ CD69⁺ T cells, i.e., cancer-specific T cells that can recognize tumor antigens (cell viability 85%), were sorted by flow cytometry. The CD3⁺ CD69⁺ T cells obtained from the above sorting were co-incubated with IL-2 (100 U/mL), IL-7 (100 U/mL) and αCD3 antibodies (10 ng/mL) and αCD28 antibodies (10 ng/mL) for 48 days in 10 mL of DMEM complete medium (37°C, 5% CO₂) to obtain T cells 1.

Alternatively, 50 million of the above mixed immune cells and 4 mg of Nanoparticle 1 were co-incubated for 6 hours in 10 mL of RPMI 1640 complete medium, and then the incubated CD3⁺ T cells (cell viability 85%) were sorted by flow cytometry. The CD3⁺ T cells obtained from the above sorting were co-incubated with IL-2 (100 U/mL), IL-7 (100 U/mL) and αCD3 antibodies (10 ng/mL) and αCD28 antibodies (10 ng/mL) for 48 days in 10 mL of DMEM complete medium (37°C, 5% CO₂) to obtain T cells 2.

Alternatively, 50 million of the above mixed immune cells and 4 mg Nanoparticle 1 were co-incubated for 6 hours in 10 mL of RPMI 1640 complete medium, and then the incubated CD3⁺ IFN-*γ*⁺ T cells (cell viability 0%) were sorted by flow cytometry. The CD3⁺ IFN-*γ*⁺ T cells obtained from the above sorting were co-incubated with IL-2 (100 U/mL), IL-7 (100 U/mL), and αCD3 antibodies (10 ng/mL) and αCD28 antibodies (10 ng/mL) for 48 days in 10 mL of DMEM complete medium (37°C, 5% CO₂) to obtain T cells 3.

Alternatively, 50 million of the above mixed immune cells and 4 mg of Nanoparticle 1 were co-incubated for 6 hours in 10 mL of RPMI 1640 complete medium, and then the incubated CD3⁺ FOXP3⁺ (cell viability 85%) were sorted by flow cytometry. The CD3⁺ FOXP3⁺ T cells obtained from the above sorting were co-incubated with IL-2 (100 U/mL), IL-7 (100 U/mL) and αCD3 antibodies (10 ng/mL) and αCD28 antibodies (10 ng/mL) for 48 days in 10 mL of DMEM complete medium (37°C, 5% CO₂) to obtain T cells 4.

50 million of the above mixed immune cells and 4 mg Nanoparticle 2 were co-incubated for 6 hours in 10 mL RPMI 1640 complete medium, and then the incubated CD3⁺ CD69⁺ T cells, i.e., cancer-specific T cells that can recognize tumor antigens (cell viability is 85%), were sorted by flow cytometry. The CD3⁺ CD69⁺ T cells obtained from the above sorting were co-incubated with IL-2 (100 U/mL), IL-7 (100 U/mL) and αCD3 antibodies (10 ng/mL) and αCD28 antibodies (10 ng/mL) for 48 days in 10 mL of DMEM complete medium (37°C, 5% CO₂) to obtain T cells 5.

### (4) Nanovaccines for cancer treatment

Female C57BL/6 aged 6-8 weeks were selected as model mice, and each mouse was subcutaneously inoculated with 1.5×10⁵ B16F10 cells on the lower right side of the back on day 0. Before tumor inoculation, the mice were subcutaneously inoculated with 1 million T cells (T cells 1, or T cells 2, or T cells 3, or T cells 4, or T cells 5) or 100 µL PBS or 2 mg Nanoparticle 1 on day 3, day 6, day 9, day 14, and day 20, separately. The tumor growth rate of the mice and the survival of the mice were monitored.

### (4) Experimental results

As shown in FIG. 27, the tumor volume of the mice in the PBS group grew rapidly. The tumor growth rate of the mice treated with T cells and Nanoparticle 1 was significantly slower and the survival time was significantly prolonged, and some mice were cured without tumors. Moreover, the T cells 1 were better than T cells 2, T cells 3, T cells 4, T cells 5, and Nanoparticle 1. This showed that it was necessary to sort out cancer-specific T cells first and then expand them; and it was necessary to use appropriate activation markers to ensure that T cells of high viability were sorted before expansion. Due to the differences in the microenvironment of the cancer cells *in vivo* and the high heterogeneity of the cancer cells, using multiple cancer cell lines can cover a more complete antigen spectrum and more diverse antigens than a single cancer cell line, so the effect was better. The effect of T cell 1 injection was better than that of direct injection of Nanoparticle (Vaccine) 1, indicating that the effect of reinfusing cancer-specific T cells after sorting and expansion was better than that of direct injection of Nanoparticle (Vaccine) *in vivo.*

### Example 27: T cells for colon cancer treatment

### (1) Collection of antigen components

The cultured CMT93, Colon26 (C26), CT26, MC38, and MC26 cancer cell lines were collected, and the above 5 cancer cell lines were mixed in a quantity ratio of 1:1:1:4:1, then the mixed cells were lysed using 0.2 M methylguanidine hydrochloride and 0.05 M polyhexamethylene guanidine hydrochloride, and all lysed components were dissolved using 0.2 M methylguanidine hydrochloride and 0.05 M polyhexamethylene guanidine hydrochloride after lysis, then a saturated aqueous solution of ammonium sulfate was added dropwise thereto, the obtained sample was centrifuged at 3000 g for 5 minutes after the precipitation was complete, the precipitate was dissolved in a 0.2 M aqueous solution of methylguanidine hydrochloride and a 0.05 M aqueous solution of polyhexamethylene guanidine hydrochloride for later use, the supernatant was heated at 95°C for 10 minutes, and the obtained sample was centrifuged at 3000 g for 5 minutes, and the precipitate was dissolved in the 0.2 M aqueous solution of methylguanidine hydrochloride and the 0.05 M aqueous solution of polyhexamethylene guanidine hydrochloride after the supernatant was discarded; and then the precipitate after salting-out using the 0.2 M methylguanidine hydrochloride solution and the 0.05 M polyhexamethylene guanidine hydrochloride solution and the precipitate after heating were combined to obtain antigen components 1 for preparing Nanoparticle 1.

### (2) Preparation of nanoparticles

The Nanoparticle 1 or Nanovacccine 1 in this example was prepared by a double emulsion method. The molecular weight of the particle preparation material PLGA was 10 KDa to 20 KDa, and the loaded immune adjuvants were poly(I:C) and CpG7909. The preparation method was as previously described. The antigen components 1 and adjuvants were first loaded inside the nanoparticles, and then 100 mg of the nanoparticles were centrifuged at 13000 g for 20 minutes, and resuspended with 10 mL of ultrapure water containing 4% trehalose and freeze-dried for 48 h. The average particle size of the Nanoparticle 1 was about 280 nm, and approximately 25 µg of protein or peptide components were loaded per 1 mg of PLGA nanoparticles, and 0.01 mg of poly(I:C) and CpG7909 each were loaded per 1 mg of PLGA nanoparticles.

### (3) Preparation of cancer-specific T cells

Female C57BL/6 mice aged 6-8 weeks were selected and subcutaneously inoculated with 1×10⁶ MC38 mouse colon cancer cells on day 0. Each mouse was intraperitoneally injected with 150 µg PD-L1 antibodies on day 6, day 8, day 10, day 12, day 14, day 16, day 18, day 20, and day 22, separately. The mice were sacrificed on day 24, lymph nodes of the mice were collected, then CD3⁺ CD69⁻ T cells, CD19⁺ B cells and CD11c⁺ DCs were sorted from lymph node cells using flow cytometry, and then the above CD19⁺ B cells and CD11c⁺ DCs were mixed at a number ratio of 1:1 to obtained mixed antigen-presenting cells co-incubated with nanoparticles.

Five million of the above mixed antigen-presenting cells and 100 *µ*g of Nanoparticle 1 were co-incubated for 4 hours in 10 mL of RPMI 1640 complete medium (37°C, 5% CO₂), and then centrifuged at 400 g for 4 minutes to remove the supernatant (containing nanoparticles) to collect the incubated mixed antigen-presenting cells. The incubated mixed antigen-presenting cells were fixed in 4% paraformaldehyde solution for 30 minutes and then washed, then the fixed antigen-presenting cells were mixed with CD3⁺ CD69⁻ T cells in a quantity ratio of 2:1 and then co-incubated for 36 hours in 10 mL of RPMI 1640 complete medium (37°C, 5% CO₂), and then the incubated CD3⁺ CD69⁺ T cells were sorted by flow cytometry, i.e., cancer-specific T cells that can recognize tumor antigens (cell viability 90%). The CD3⁺ CD69⁺ T cells obtained from the above sorting were co-incubated with IL-2 (1000 U/mL), IL-7 (10 U/mL) and αCD3 antibodies (10 ng/mL) and αCD28 antibodies (10 ng/mL) for 42 days in 10 mL of DMEM complete medium (37°C, 5% CO₂) to obtain T cells 1.

Five million of the above mixed antigen-presenting cells and 100 *µ*g of Nanoparticle 1 were co-incubated for 4 hours in 10 mL of RPMI 1640 complete medium (37°C, 5% CO₂), and then centrifuged at 400 g for 4 minutes to remove the supernatant (containing nanoparticles) to collect the incubated mixed antigen-presenting cells. The incubated mixed antigen-presenting cells were fixed in 4% paraformaldehyde solution for 30 minutes and then washed, then the fixed antigen-presenting cells were mixed with CD3⁺ CD69⁻ T cells in a quantity ratio of 2:1 and then co-incubated for 36 hours in 10 mL of RPMI 1640 complete medium (37°C, 5% CO₂), and then the incubated CD3⁺ T cells were sorted by flow cytometry (cell viability 90%). The CD3⁺ T cells obtained from the above sorting were co-incubated with IL-2 (1000 U/mL), IL-7 (10 U/mL) and αCD3 antibodies (10 ng/mL) and αCD28 antibodies (10 ng/mL) for 42 days in 10 mL of DMEM complete medium (37°C, 5% CO₂) to obtain T cells 2.

Five million of the above mixed antigen-presenting cells and 100 *µ*g of Nanoparticle 1 were co-incubated for 4 hours in 10 mL of RPMI 1640 complete medium (37°C, 5% CO₂), and then centrifuged at 400 g for 4 minutes to remove the supernatant (containing nanoparticles) to collect the incubated mixed antigen-presenting cells. The incubated mixed antigen-presenting cells were fixed in 4% paraformaldehyde solution for 30 minutes and then washed, then the fixed antigen-presenting cells were mixed with CD3⁺ CD69⁻ T cells in a quantity ratio of 2:1 and then co-incubated for 36 hours in 10 mL of RPMI 1640 complete medium (37°C, 5% CO₂), and then the incubated CD3⁺ IFN-*γ*⁺ T cells were sorted by flow cytometry (cell viability 0%). The CD3⁺ IFN-*γ*⁺ T cells obtained from the above sorting were co-incubated with IL-2 (1000 U/mL), IL-7 (10 U/mL), and αCD3 antibodies (10 ng/mL) and αCD28 antibodies (10 ng/mL) for 42 days in 10 mL of DMEM complete medium (37°C, 5% CO₂) to obtain T cells 3.

### (4) Nanovaccines for cancer treatment

Female C57BL/6 aged 6-8 weeks were selected as model mice, and each mouse was subcutaneously inoculated with 1.0×10⁶ MC38 colon cancer cells on the lower right side of the back on day 0. Before tumor inoculation, the mice were subcutaneously inoculated with 1 million T cells (T cells 1, or T cells 2, or T cells 3, or 2 mg Nanoparticle 1) or 100 µL PBS on day 3, day 6, day 9, day 14, and day 20, separately. The tumor growth rate of the mice and the survival of the mice were monitored.

### (4) Experimental results

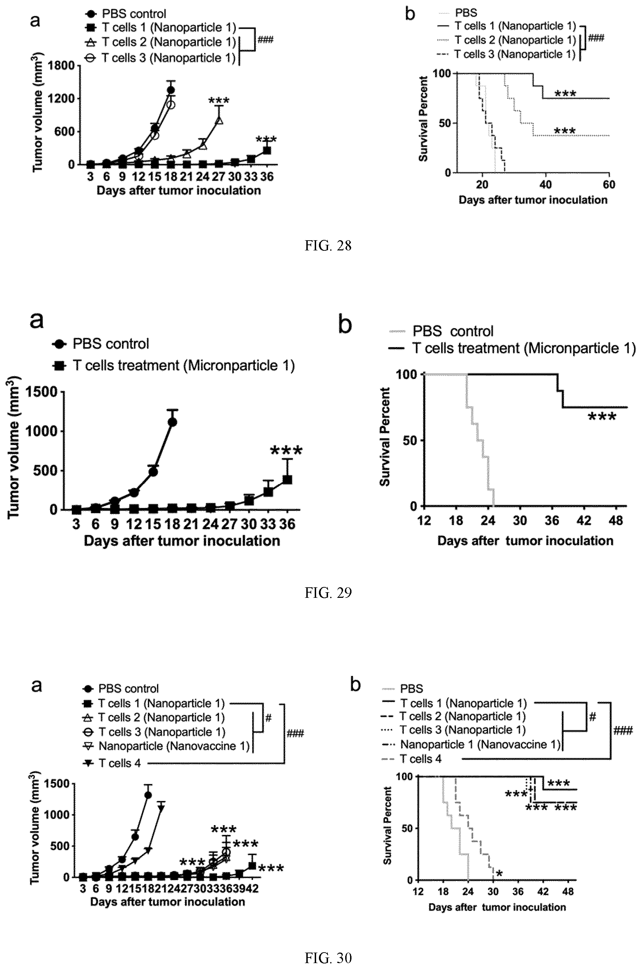

As shown in FIG. 28, the tumor volume of mice in the PBS group grew rapidly, and the mice died quickly. The tumor growth rate of the mice treated with T cells was significantly slower and the survival time was significantly prolonged, and some mice were cured without tumors. Moreover, the effect of T cells 1 was better than that of T cells 2, T cells 3 and Nanoparticle 1. This showed that it was necessary to first sort out cancer-specific T cells and then expand them, and it was necessary to use appropriate activation markers to ensure that T cells of high viability were sorted before expansion. Moreover, the therapeutic effect of T cell reinfusion after sorting and expansion was better than that of direct injection of nanoparticles.

### Example 28: Sorted and expanded T cells for lung cancer prevention

### (1) Lysis of cancer cells

The cultured LLC cells were centrifuged at 400 g for 5 minutes, and then washed twice with PBS and resuspended in ultrapure water. The obtained cancer cells were inactivated and denatured by ultraviolet and high-temperature heating respectively, then ultrapure water was added, and freezing and thawing was repeated 5 times, accompanied by ultrasound to lyse the cancer cells, the cell lysate was centrifuged at 5000 g for 10 minutes, the supernatant was water-soluble components, the precipitate was dissolved by using 10% octyl glucoside to obtain dissolved original water-insoluble components, and the water-soluble components and the water-insoluble components were mixed at a mass ratio of 2:1 to obtain antigen components 1 required for preparing the microparticles.

### (2) Preparation of microparticles

Microparticle 1 in this example was prepared by a double emulsion method, the molecular weight of the microparticle skeleton material PLGA was 38 KDa to 54 KDa, and the immune adjuvants adopted were CpG1018 and Poly ICLC. During preparation, the antigen components 1 and adjuvants were first loaded inside, and then 100 mg microparticles were centrifuged at 9000 g for 20 minutes, resuspended with 10 mL of ultrapure water containing 4% trehalose and dried for 48 h for later use. The average particle size of the microparticle system was about 5.0 µm; and approximately 410 µg of protein or protein or peptide components of the antigen components were loaded, 0.01 mg of CpG1018 and Poly ICLC each were loaded per 1 mg of PLGA microparticles.

### (3) Preparation of dendritic cells

Same as Example 16.

### (4) Activation of dendritic cells

Five million BMDCs, 2 mg microparticles and IL-15 (20 ng/mL) were co-incubated for 8 h in 5 mL RPMI 1640 (10% FBS) medium, and then the BMDCs were collected and the activated dendritic cells were irradiated with radiation to inactivate the dendritic cells, and the inactivated dendritic cells were used to activate T cells.

### (5) Preparation of cancer-specific T cells

Female C57BL/6 aged 6-8 weeks were selected and subcutaneously injected with 100 µL of Microparticle 1 containing 2 mg PLGA on day 0, day 4, day 7, day 14, day 21, and day 28, separately. The mice were sacrificed on day 32, the peripheral blood of the mice was collected, then peripheral blood mononuclear cells (PBMCs) were isolated from the peripheral blood, and CD3⁺ T cells were sorted from the PBMCs using flow cytometry. The sorted CD3⁺ T cells (2 million cells), the inactivated BMDCs prepared in step 4 (3 million cells) and IL-7 (10 ng/mL) were co-incubated for 18 hours in 10 mL of RPMI 1640 complete medium. Then CD3⁺ CD69⁺ T cells (cell viability 85%), i.e., cancer-specific T cells that can recognize tumor antigens, in the incubated T cells were sorted by flow cytometry. The 300000 CD3⁺ CD69⁺ T cells sorted above were co-incubated with IL-2 (1000 U/mL), IL-7 (1000 U/mL), IL-15 (1000 U/mL), and αCD3 antibody (10 ng/mL) in 10 mL of DMEM complete medium (37°C, 5% CO2) for 14 days to expand cancer-specific T cells (cell viability 85%).

### (6) Cancer-specific T cells for cancer prevention

Female C57BL/6 mice aged 6-8 weeks were selected as model mice. One day before adoptive transfer of cells, the recipient mice were intraperitoneally injected with a 100 mg/kg dose of cyclophosphamide to eliminate immune cells in the recipient mice. The mice were subcutaneously injected with 100 µL containing 1.2 million expanded CD3⁺ T cells on day 0. At the same time, each mouse was subcutaneously inoculated with 1×10⁶ LLC cells on day 0, and the methods of monitoring tumor volumes and survival time were the same as above.

### (7) Experimental results

As shown in FIG. 29, compared with the control group, the lung cancer tumor growth rate of the cancer-specific T cell treatment group obtained by microparticle sorting and expansion was significantly slower and the survival time of the mice was significantly prolonged.

### Example 29: Cancer-specific T cells for melanoma treatment

### (1) Collection of antigen components

The cultured B16-F10, B16-F1, S91, ME, K735, B16-BL6, and MEC57 cancer cell lines were collected, and the above cancer cell lines were mixed in a quantity ratio of 1:1:1:1:1:1:1, then the mixed cells were lysed using an 8 M aqueous urea solution, all lysed components were dissolved using the 8 M aqueous urea solution after lysis, then a saturated aqueous solution of ammonium sulfate was added dropwise thereto, the obtained sample was centrifuged at 3000 g for 5 minutes after the precipitation was complete, the precipitate was dissolved in the 8 M aqueous urea solution for later use, the supernatant was heated at 95°C for 10 minutes, then the obtained sample was centrifuged at 3000 g for 5 minutes, and the precipitate was dissolved in the 8 M aqueous urea solution after the supernatant was discarded; and then the precipitate after salting-out dissolved using the 8 M aqueous urea solution and the precipitate after heating dissolved were combined to obtain the antigen components 1 for preparing Nanoparticle 1.

### (2) Preparation of nanoparticles

The Nanoparticle 1 in this example was prepared by a double emulsion method. The molecular weight of the particle preparation material PLGA was 10 KDa to 20 KDa, and the immune adjuvants adopted were poly(I:C), CpG7909 and CpG2395. The preparation method was as previously described. The antigen components 1 and adjuvants were first loaded inside the nanoparticles, and then 100 mg of the nanoparticles were centrifuged at 12000 g for 20 minutes, and resuspended with 10 mL of ultrapure water containing 4% trehalose and freeze-dried for 48 h. The average particle size of the Nanoparticle 1 (Nanovaccine 1) was about 280 nm, and approximately 250 µg of protein or peptide components were loaded per 1 mg of PLGA particles, and 0.01 mg of poly(I:C), CpG7909 and CpG2395 each were loaded per 1 mg of PLGA particles.

### (3) Preparation of cancer-specific T cells

Female C57BL/6 mice aged 6-8 weeks were selected and subcutaneously inoculated with 1.5×10⁵ B16F10 mouse melanoma cancer cells on day 0. Each mouse was intraperitoneally injected with 150 µg PD-L1 antibodies on day 6, day 8, day 10, day 12, day 14, day 16, day 18, day 20, separately. The mice were sacrificed on day 22, the peripheral blood of the mice was collected, and then mouse PBMCs were prepared to obtain mixed immune cells co-incubated with nanoparticles.

50 million of the above mixed immune cells, 4 mg Nanoparticle 1, IL-2 (10 ng/mL), IL-7 (10 ng/mL) and IL-15 (10 ng/mL) were co-incubated for a certain period of time (1 hour, 6 hours or 168 hours) in 10 mL RPMI 1640 complete medium, and then the incubated CD3⁺ CD69⁺ T cells were sorted by flow cytometry, i.e., cancer-specific T cells that can recognize tumor antigens (cell viability 80%). The CD3⁺ CD69⁺ T cells obtained from the above sorting were co-incubated with IL-2 (100 U/mL), IL-7 (100 U/mL) and αCD3 antibodies (10 ng/mL) and αCD28 antibodies (10 ng/mL) for 48 days in 10 mL of DMEM complete medium (37°C, 5% CO₂) to obtain T cells (cell viability 80%). The nanoparticles and immune cells were co-incubated for 6 hours and sorted and expanded to obtain T cells 1; the nanoparticles and immune cells were co-incubated for 1 hour and sorted and expanded to obtain T cells 2; and the nanoparticles and immune cells were co-incubated for 168 hours and sorted and expanded to obtain T cells 3.

Alternatively, 50 million of the above mixed immune cells obtained by sorting, 4 mg Nanoparticle 1, IL-2 (10 ng/mL), IL-7 (10 ng/mL) and IL-15 (10 ng/mL) were directly co-incubated for 6 hours in 10 mL RPMI 1640 complete medium, and then without any sorting and expansion, the obtained cells were mixed cell T cells 4 (cell viability 80%).

### (4) Nanovaccines for cancer treatment

Female C57BL/6 aged 6-8 weeks were selected as model mice, and each mouse was subcutaneously inoculated with 1.5×10⁵ B16F10 cells on the lower right side of the back on day 0. After tumor inoculation, the mice were subcutaneously injected with 100000 T cells (T cells 1, or T cells 2, or T cells 3, or T cells 4) or 100 µL PBS or 2 mg Nanoparticle 1 (Nanovaccine 1) on day 3, day 6, day 9, day 14, and day 20, separately. The tumor growth rate of the mice and the survival of the mice were monitored.

### (4) Experimental results

As shown in FIG. 27, the tumor volume of the mice in the PBS group grew rapidly. The tumor growth rate of the mice treated with T cells was significantly slower. Moreover, the effect of T cells 1 was better than that of T cells 2, T cells 3 and T cells 4. This indicated that the appropriate incubation time of nanoparticles was very critical during sorting of cancer-specific T cells. Moreover, proper sorting and expansion after incubation was completed can achieve better results.

In some embodiments, nanoparticles and/or microparticles were co-incubated with antigen-presenting cells and T cells simultaneously, and in some embodiments, nanoparticles and/or microparticles were first co-incubated with antigen-presenting cells and then the incubated antigen-presenting cells were co-incubated with T cells. When nanoparticles and/or microparticles are used to first co-incubate with antigen-presenting cells and then the incubated antigen-presenting cells are co-incubated with T cells, the nanoparticles and/or the microparticles and the antigen-presenting cells may be co-incubated with the T cells directly without other treatment after co-incubation, or the incubated antigen-presenting cells may be fixed or irradiated and then co-incubated with theT cells. The antigen-presenting cells co-incubated with nanoparticles may be treated by fixation with a reagent such as paraformaldehyde, radiation irradiation treatment, or other treatments to inactivate the incubated antigen-presenting cells. When nanoparticles are first co-incubated with antigen-presenting cells, and then the co-incubated antigen-presenting cells are co-incubated with T cells, the antigen-presenting cells may be live cells or dead cells.

The researchers have found that compounds with a guanidino group or a urea structure with a structure represented by structural formula 1 (such as compounds such as urea, guanidine hydrochloride, metformin, and methylguanidine hydrochloride described in the present disclosure) can be used as a dissolving agent in a dissolving solution to dissolve water-insoluble components in cancer cells/tumor tissue or precipitated components generated by treatment such as salting-out. In practical application, other compounds with a structure set forth in structural formula 1 should theoretically also be used as dissolving agents and also fall within the scope of the present disclosure.

Due to space limitations, only a few surface markers such as CD69, CD25, HLA-DR, CD107a, and FASL are listed and used as markers of T cell activation in the embodiments of the present disclosure to sort activated cancer-specific T cells. In practical application, any other surface markers that may be used to indicate T cell activation may also be used, and molecules that may be used as surface markers include but are not limited to: any one of CD69, CD137, CD25, CD134, CD80, CD86, OX40L, OX40, CD28, FAS-L, IL-2R, HLA- DR, CD127 (IL-7R), CD150, CD107A, CD83, CD166, CD39, CD178, CD212, CD229, CD100, CD107b, CD108, CD109, CD113, CD122, CD126, CD253, CD197, PD-1, TIM3, LAG-3, TIGIT, CD62L, CD70, CTLA-4 (CD152), CD27, CD26, CD30, TNFRSF9, CD74, PD-L1 (CD274), CD258, CD261, 4-1BB, CD154, ICAM-1, LFA-1, LFA-2, VLA-4, CD160, CD71, CXCR3, TNFRSF14, TNFRSF18, TNFSF4, TNFSF9, TNFSF14, CD11a, CD101, CD48, CD244, CD49a, CD95, CD44, CXCR 1, CD103, CD45RO, ICOS (CD278), VTCN1, HLA2, LGAL59, CCR7, CD357, BCL6, TCF-1, CD38, CD27, etc., or any combination thereof. Any of the above surface markers which may indicate T cell activation or components of one or more surface markers are also within the scope of the present disclosure.

Obviously, the above embodiments are merely examples for clarity of illustration, and are not limiting the implementations. For those skilled in the art, on the basis of the above description, other changes or modifications in different forms can be made. All implementations need not be and cannot be exhaustive herein. However, obvious changes or modifications derived therefrom are still within the scope of protection of the present disclosure.

## Claims

1. A method for preparing a cancer-specific T cell derived from an autologous or allogeneic source for preventing or treating cancer, specifically comprising steps of: first, isolating an immune cell from peripheral blood or a peripheral immune organ; then, co-incubating with an antigen-presenting cell and a nanoparticle and/or a microparticle loaded with a tumor whole-cell component or containing a partial antigen component of whole-cell component for a period of time to activate a cancer-specific T cell; then, isolating the cancer-specific T cell activated by a cancer antigen; and, reinfusing the cancer-specific T cell into the body to exert an anti-cancer effect after *in vitro* expansion, wherein
preferably, the preparation method specifically comprises steps of:
(1) first, isolating an immune cell from peripheral blood or a peripheral immune organ, or sorting a T cell from the immune cell;
(2) co-incubating a microparticle and/or a nanoparticle loaded with a tumor antigen component with an antigen-presenting cell and the T cell for a certain period of time after mixing, and then sorting out a T cell activated and expressing a specific cell marker; and
(3) co-incubating the T cell expressing the specific cell marker sorted in the step (2) with a cytokine and/or an antibody to obtain an expanded specific T cell;
preferably, in the step (2), the tumor antigen component is a whole-cell lysate component of tumor tissue/a cancer cell or a portion of a whole-cell lysate component of tumor tissue/a cancer cell; and the whole-cell lysate component may be divided into a water-soluble component and a water-insoluble component solubilized in a solubilizing solution containing a solubilizing agent;
preferably, in the step (2), the tumor antigen component is obtained by whole-cell lysis of one or more cancer cells and/or tumor tissue, by treatment after whole-cell lysis of one or more cancer cells and/or tumor tissue, or by lysis after whole-cell treatment of one or more cancer cells and/or tumor tissue, and preferably, at least one of the cancer cells or tumor tissue is the same as a target disease type; or the antigen component consists of a portion of a component in one or more cancer cells and/or tumor tissue, and the portion of the component contains a protein/peptide component and/or an mRNA component in a lysate; and
preferably, the T cell sorted in the step (1) is any one of a CD3⁺ T cell, a CD3⁺ CD8⁺ T cell and a CD4⁺ T cell, or a combination thereof.

2. The method of claim 1, wherein the nanoparticle and/or the microparticle loaded with the tumor antigen component may be co-incubated together with the antigen-presenting cell and the T cell to activate the cancer-specific T cell; or the nanoparticle and/or the microparticle loaded with the tumor antigen component may be first co-incubated with the antigen-presenting cell to activate the antigen-presenting cell, and then the activated antigen-presenting cell is alone co-incubated with the T cell to activate the cancer cell-specific T cell; and after the nanoparticle and/or the microparticle loaded with the tumor antigen component is first co-incubated with the antigen-presenting cell to activate the antigen-presenting cell, the antigen-presenting cell may be co-incubated with the T cell to activate the specific T cell without special treatment, or the antigen-presenting cell may be treated with fixation, radiation, irradiation, modification, inactivation, mineralization, etc., and then co-incubated with the T cell to activate the specific T cell. During sorting of the cancer-specific T cell, one marker may be used as an activation marker for sorting, or a component of a plurality of markers may be used as an activation marker for sorting.

3. The method of any one of claims 1-2, wherein the antigen component may comprise the mRNA component in addition to the protein and peptide components.

4. The method of any one of claims 1-3, wherein in the step (1), the autologous or allogeneic source with the immune cell isolated from peripheral blood or a peripheral immune system may not be treated, or may be treated with radiotherapy, immunotherapy, chemotherapy, particle therapy, and vaccine therapy when the above cell is isolated and extracted.

5. The method of any one of claims 1-4, wherein a method of the sorting in the step (1) and the step (2) is any one of flow cytometry and a magnetic bead method, or a combination thereof.

6. The method of any one of claims 1-5, wherein the tumor antigen component is a cell lysis component of tumor tissue and/or a cancer cell, comprising one or both of a water-soluble component and a water-insoluble component generated after cell lysis of the tumor tissue and/or the cancer cell, the water-soluble component and the water-insoluble component are collected separately and the nanoparticle or the microparticle is prepared separately, and the water-insoluble component is solubilized using the solubilizing solution containing the solubilizing agent; or the cancer cell or the tumor tissue may be lysed directly and the whole-cell component may be solubilized by directly adopting the solubilizing solution containing the solubilizing agent, and the nanoparticle or the microparticle is prepared, and the water-insoluble component is solubilized by the solubilizing solution containing the solubilizing agent; and
preferably, when the antigen component is the whole-cell lysate component of the tumor tissue/the cancer cell, a preparation method thereof is: (1) first, lysing the cancer cell/the tumor tissue; then, preparing a water-soluble component and a water-insoluble component separately; and then, solubilizing the water-insoluble component using a specific solubilizing agent containing the solubilizing solution for use; or (2) lysing the cell using the solubilizing solution containing the solubilizing agent, and then solubilizing a lysed whole-cell component using the solubilizing solution containing the solubilizing agent.

7. The method of any one of claims 1-6, wherein when the tumor antigen component is the portion of the component of the whole-cell lysate component of the tumor tissue/the cancer cell, a preparation method thereof is: (1) first, preparing a lysate of the tumor tissue/the cancer cell; then, preparing the water-soluble component and the water-insoluble component separately; then, solubilizing the water-insoluble component using the specific solubilizing agent containing the solubilizing solution for use; then, isolating and extracting the protein and peptide components in the water-soluble component from the water-soluble component using an appropriate method; and then, using the protein and peptide components isolated and extracted from the water-soluble component together with all the water-insoluble components as the antigen component;
(2) first, preparing a lysate of the tumor tissue/the cancer cell; then, preparing the water-soluble component and the water-insoluble component separately; then, solubilizing the water-insoluble component using the specific solubilizing agent containing the solubilizing solution for use; then, isolating and extracting the protein and peptide components in the water-soluble component from the water-insoluble component using an appropriate method; and then, using the protein and peptide components isolated and extracted from the water-insoluble component together with all the water-soluble component as the antigen component;
(3) first, preparing a lysate of the tumor tissue/the cancer cell; then, preparing the water-soluble component and the water-insoluble component separately; then, solubilizing the water-insoluble component using the specific solubilizing agent containing the solubilizing solution; then, isolating and extracting the protein and peptide components in the water-soluble component from the water-soluble component and the water-insoluble component using an appropriate method, respectively; and then, using the protein and peptide components isolated and extracted from the water-soluble component and the water-insoluble component together as the antigen component;
or (4) lysing the cell or the tissue directly and solubilizing the whole-cell component directly by using the solubilizing solution containing the solubilizing agent; then, preparing the protein/peptide component therein by an appropriate treatment method; and, using the protein/peptide component as the antigen component;
in the above preparation treatment method, a step of isolating and extracting whole-cell mRNA may be added, and the whole-cell mRNA may be used as a portion of the antigen component;
the appropriate treatment method comprises but is not limited to treatment methods such as salting-out, heating and enzymatic hydrolysis; and the water-insoluble component or a precipitate generated after treatments such as salting-out, heating and enzymatic hydrolysis is solubilized using the solubilizing solution containing the solubilizing agent.

8. The method of any one of claims 1-7, wherein a concentration of the nanoparticle and/or the microparticle is 2.5 ng/mL to 50 mg/mL when the nanoparticle and/or the microparticle is co-incubated with the antigen-presenting cell alone or when the nanoparticle and/or the microparticle is co-incubated with the antigen-presenting cell and the T cell together; and time of the co-incubation is 1 hour to 168 hours.

9. The method of any one of claims 1-8, wherein the whole-cell component may be treated by inactivation or (and) denaturation, solidification, biomineralization, ionization, chemical modification, nuclease treatment, etc. before or (and) after lysis before preparing the nanoparticle or the microparticle; or
the nanoparticle or the microparticle may also be prepared directly without any inactivation or (and) denaturation, solidification, biomineralization, ionization, chemical modification, and nuclease treatment before or (and) after the cell lysis.

10. The method of any one of claims 1-9, wherein the tumor tissue cell is treated by inactivation or (and) denaturation before the lysis, the tumor tissue cell may also be treated by inactivation or (and) denaturation after the cell lysis, or the tumor tissue cell may also be treated by inactivation or (and) denaturation both before and after the cell lysis.

11. The method of any one of claims 1-10, wherein a method of the inactivation or (and) denaturation treatment before or (and) after the cell lysis comprises any one of ultraviolet irradiation, high-temperature heating, radiation irradiation, high pressure, curing, biomineralization, ionization, chemical modification, nuclease treatment, collagenase treatment, and freeze-drying, or a combination thereof.

12. The method of any one of claims 1-11, wherein in the step (2), a number ratio of the antigen-presenting cell to the T cell used is greater than 1:1; and a pre-existing cancer-specific T cell in a peripheral immune cell is activated *in vitro* after presentation by the antigen-presenting cell using the nanoparticle or the microparticle, and the nanoparticle or the microparticle is selected from a nanoparticle having a particle size of 1 nm to 1000 nm or a microparticle having a particle size of 1 µm to 1000 µm.

13. The method of any one of claims 1-12, wherein the antigen-presenting cell co-incubated with the T cell and the nanoparticle and/or the microparticle is derived from an autologous source, an allogeneic source, a cell line, a stem cell, or any mixture thereof; and the co-incubated antigen-presenting cell is a B cell, a dendritic cell, a macrophage, or any mixture of the three.

14. The method of any one of claims 1-13, wherein the antigen-presenting cell is derived from an autologous antigen-presenting cell, an allogeneic antigen-presenting cell, an antigen-presenting cell line, or an antigen-presenting cell differentiated from the stem cell, preferably, any one of the dendritic cell (DC), the B cell and the macrophage, or a combination thereof; and more preferably, a combination of more than one antigen-presenting cell is used.

15. The method of any one of claims 1-14, wherein the mixing and co-incubating is selected from any one of three ways of:
(a) mixing and co-incubating the three directly for a certain period of time;
(b) co-incubating the microparticle and/or the nanoparticle with the antigen-presenting cell for a period of time, and then adding the T cell for co-incubating; and
(c) first co-incubating the microparticle and/or the nanoparticle with the antigen-presenting cell for a period of time, and sorting an incubated antigen-presenting cell and then co-incubating both the antigen-presenting cell and the T cell.

16. The method of any one of claims 1-15, wherein a culture condition of the mixing and co-incubating is co-incubating for 1 h to 168 h under conditions of 30-38°C and 1-10% CO₂.

17. The method of any one of claims 1-16, wherein a cytokine may be added in the mixing and co-incubating; and the cytokine added comprises but is not limited to an interleukin, a tumor necrosis factor, an interferon, and a growth factor; and preferably, the cytokine added comprises interleukin 7 (IL-7) and interleukin 15 (IL-15).

18. The method of any one of claims 1-17, wherein in the step (2), the method of the sorting is isolating a cell expressing the specific cell marker from a cell population by flow cytometry or the magnetic bead method, etc. after binding an antibody with fluorescence or magnetism or a specific ligand to the specific cell marker on a surface of the T cell.

19. The method of any one of claims 1-18, wherein in the step (3), the sorted T cell expressing the specific cell marker is any one of CD69, CD137, CD25, CD134, CD80, CD86, OX40L, OX40, CD28, FAS-L, IL-2R, HLA- DR, CD127 (IL-7R), CD150, CD107A, CD83, CD166, CD39, CD178, CD212, CD229, CD100, CD107b, CD108, CD109, CD113, CD122, CD126, CD253, CD197, PD-1, TIM3, LAG-3, TIGIT, CD62L, CD70, CTLA-4 (CD152), CD27, CD26, CD30, TNFRSF9, CD74, PD-L1 (CD274), CD258, CD261, 4-1BB, CD154, ICAM-1, LFA-1, LFA-2, VLA-4, CD160, CD71, CXCR3, TNFRSF14, TNFRSF18, TNFSF4, TNFSF9, TNFSF14, CD11a, CD101, CD48, CD244, CD49a, CD95, CD44, CXCR 1, CD103, CD45RO, ICOS (CD278), VTCN1, HLA2, LGAL59, CCR7, CD357, BCL6, TCF-1, CD38, and CD27, or a combination thereof.

20. The method of any one of claims 1-19, wherein in the step (3), a concentration of the cytokine is 1 ng/mL to 6000 ng/mL, preferably 5 ng/mL to 200 ng/mL, and more preferably 10 ng/mL to 30 ng/mL.

21. The method of any one of claims 1-20, wherein in the step (3), the cytokine comprises but is not limited to an interleukin, an interferon and a tumor necrosis factor.

22. The method of any one of claims 1-21, wherein the interleukin comprises but is not limited to interleukin 2 (IL-2), interleukin 7 (IL-7), interleukin 12 (IL-12), interleukin 15 (IL-15), interleukin 17 (IL-17), and interleukin 21 (IL-21).

23. The method of any one of claims 1-22, wherein a concentration of the antibody is 1 ng/mL to 6000 ng/mL, preferably 5 ng/mL to 100 ng/mL, and more preferably 10 ng/mL to 30 ng/mL.

24. The method of any one of claims 1-23, wherein the antibody comprises but is not limited to any one of an αCD3 antibody, an αCD28 antibody, an αCD80 antibody, an αCD86 antibody, and an αOX40 antibody, or a combination thereof.

25. The method of any one of claims 1-24, wherein time of the co-incubation of the nanoparticle and/or the microparticle with a mixture of the antigen-presenting cell and the T cell is at least 1 hour, and preferably 6 hours to 96 hours.

26. The method of any one of claims 1-25, wherein time of the co-incubation of the nanoparticle and/or the microparticle with the antigen-presenting cell alone is at least 1 hour, and preferably 6 hours to 96 hours.

27. The method of any one of claims 1-26, wherein time of the co-incubation of a mixture of the activated antigen-presenting cell and the T cell is at least 1 hour, and preferably 6 hours to 96 hours.

28. The method of any one of claims 1-27, wherein time of the expansion culture is at least 1 day, and preferably 4 days to 72 days.

29. The method of any one of claims 1-28, wherein a concentration of the nanoparticle and/or the microparticle is 2.5 ng/mL to 50 mg/mL when the nanoparticle and/or the microparticle is co-incubated with the antigen-presenting cell alone or when the nanoparticle and/or the microparticle is co-incubated with the antigen-presenting cell and the T cell.

30. The method of any one of claims 1-29, wherein a content of the protein and peptide components in the antigen component loaded by the nanoparticle and/or the microparticle is higher than 10 ng/mL.

31. The method of any one of claims 1-30, wherein the specific T cell obtained after *in vitro* expansion obtained in the step (3) is reinfused into the body to exert an anticancer effect.

32. The method of any one of claims 1-31, wherein the cancer antigen loaded by the nanoparticle and/or the microparticle is the whole-cell component of the tumor tissue and/or the cancer cell containing the water-soluble component and/or the water-insoluble component of the tumor tissue and/or the cancer cell.

33. The method of any one of claims 1-32, wherein the nanoparticle and/or the microparticle for activating the cancer-specific T cell is loaded with a component of one and/or more tumor tissue and/or cancer cells in such a way that the water-soluble component and the water-insoluble component of a whole-cell are separately or together encapsulated in an interior of the particle and/or separately or together loaded onto a surface of the particle.

34. The method of any one of claims 1-33, wherein an original water-insoluble portion of the whole-cell component derived from the tumor tissue or the cancer cell loaded by the nanoparticle and/or the microparticle for activating the cancer-specific T cell is changed from insoluble in pure water to soluble in an aqueous solution containing a solubilizing agent/a dissolving agent or in an organic solvent by an appropriate solubilizing/dissolving method; and the dissolving agent/solubilizing agent adopted is selected from one or more of a compound with a structure of structural formula 1, a deoxycholate, a lauryl sulfate, glycerol, a protein-degrading enzyme, albumin, lecithin, a peptide, an amino acid, a glycoside, and a choline,
wherein structural formula 1 is as follows: the structure of structural formula 1 is as follows: 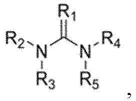
R₁ is C, N, S or O, and R₂-R₅ are independently selected from at least one of hydrogen, alkyl, amino, carboxyl, and substituted or unsubstituted guanidino; and
the compound with the structural formula 1 comprises but is not limited to metformin hydrochloride, metformin sulfate, metformin sulfonate, a metformin salt, metformin, polyhexamethyleneguanidine hydrochloride, agmatine sulfate, methylguanidine hydrochloride, tetramethylguanidine hydrochloride, urea, guanidine hydrochloride, guanidine sulfate, guanidine sulfonate, a guanidine salt, other compounds containing guanidino or urea, guanidine carbonate, arginine, guanidinoacetic acid, guanidinophosphoric acid, guanidine sulfamate, guanidinosuccinic acid, semicarbazide hydrochloride, carbamoylurea, acetylurea, a sulfonylurea compound (glibenclamide, gliclazide, gliquidone, glimepiride, etc.), a thiourea compound (thiouracil, imidazole, etc.), nitrosoureas, and other compounds with the structure of structural formula 1.

35. The method of any one of claims 1-34, wherein the surface of the nanoparticle and/or the microparticle for activating the cancer-specific T cell is linked with a target head actively targeting the antigen-presenting cell.

36. The method of any one of claims 1-35, wherein the way in which the water-soluble component and/or the water-insoluble component is loaded onto the surface of the cancer vaccine comprises at least one of adsorption, covalent linkage, charge interaction, hydrophobic interaction, one-step or multi-step solidification, mineralization, and encapsulation.

37. The method of any one of claims 1-36, wherein the nanoparticle has a particle size of 1 nm to 1000 nm; and the microparticle has a particle size of 1 µm to 1000 µm.

38. The method of any one of claims 1-37, wherein the surface of the nanoparticle or the microparticle is electrically neutral, negatively charged or positively charged.

39. The method of any one of claims 1-38, wherein a preparation material of the nanovaccineand/or the microvaccine is an organic synthetic polymer material, a natural polymer material or an inorganic material.

40. The method of any one of claims 1-39, wherein the organic synthetic polymer material is PLGA, PLA, PGA, PEG, PCL, Poloxamer, PVA, PVP, PEI, PTMC, polyanhydride, PDON, PPDO, PMMA, a poly(amino acid), or a synthetic peptide; the natural polymer material is lecithin, cholesterol, sodium alginate, albumin, collagen, gelatin, a cell membrane component, starch, saccharides, or a peptide; and the inorganic material is ferric oxide, ferroferric oxide, calcium carbonate, or calcium phosphate.

41. The method of any one of claims 1-40, wherein the component of one and/or more tumor tissue and/or cancer cells may be co-loaded onto the nanoparticle and/or the microparticle for activating the cancer-specific T cell together with an immune adjuvant.

42. The method of any one of claims 1-41, wherein both the water-soluble portion and the water-insoluble portion may be solubilized by an aqueous solubilizing solution containing the dissolving agent/the solubilizing agent or the organic solvent. The dissolving agent/the solubilizing agent is at least one of dissolving agents/solubilizing agents that may increase solubility of the protein or the peptide in the aqueous solution; and the organic solvent is an organic solvent that may dissolve the protein or the peptide.

43. The method of any one of claims 1-42, wherein the original water-insoluble portion is changed from being insoluble in pure water to being soluble in the aqueous solution containing the dissolving agent/the solubilizing agent or in the organic solvent by the appropriate solubilizing/dissolving method; and the dissolving agent/solubilizing agent adopted is selected from one or more of a compound with a structure of structural formula 1, a deoxycholate, a lauryl sulfate, glycerol, a protein-degrading enzyme, albumin, lecithin, a peptide, an amino acid, a glycoside, and a choline,
wherein structural formula 1 is as follows: the structure of structural formula 1 is as follows: 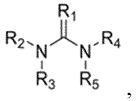
R1 is C, N, S or O, and R2-R5 are independently selected from at least one of hydrogen, alkyl, amino, carboxyl, and substituted or unsubstituted guanidino.
The compound with the structural formula 1 comprises but is not limited to metformin hydrochloride, metformin sulfate, metformin sulfonate, a metformin salt, metformin, polyhexamethyleneguanidine hydrochloride, agmatine sulfate, methylguanidine hydrochloride, tetramethylguanidine hydrochloride, urea, guanidine hydrochloride, guanidine sulfate, guanidine sulfonate, a guanidine salt, other compounds containing guanidino or urea, guanidine carbonate, arginine, guanidinoacetic acid, guanidinophosphoric acid, guanidine sulfamate, guanidinosuccinic acid, semicarbazide hydrochloride, carbamoylurea, acetylurea, a sulfonylurea compound (glibenclamide, gliclazide, gliquidone, glimepiride, etc.), a thiourea compound (thiouracil, imidazole, etc.), nitrosoureas, and other compounds with the structure of structural formula 1.

44. The method of any one of claims 1-43, wherein a cell component loaded by the nanoparticle or the microparticle for activating the cancer-specific T cell is derived from a component obtained from one or more cancer cells and/or one or more whole-cells of tumor tissue, the water-insoluble component is loaded onto a delivery particle, so that the nano-system or micron-system contains more antigens, and more preferably, the water-soluble component and the water-insoluble component are loaded onto the delivery particle together, so that the delivery particle is loaded with a whole-cell antigen component.

45. The method of any one of claims 1-44, wherein for the cell component loaded by the nanoparticle and/or the microparticle for activating the cancer-specific T cell or a mixture thereof, the mixture comprises but is not limited to water-soluble components mixed with each other, or water-insoluble components mixed with each other, or all or a portion of water-soluble components and all or a portion of water-soluble components mixed with each other.

46. The method of any one of claims 1-45, wherein in the nanoparticle and/or the microparticle, the cell component or the mixture thereof is loaded into an interior and/or onto the surface of the nanoparticle and/or the microparticle, specificially, a way of the loading is that the water-soluble component and the water-insoluble component of the cell are separately or together encapsulated in the interior of the particle and/or separately or together loaded onto the surface of the particle, comprising but not limited to the water-soluble component being loaded into the particle and loaded onto the surface of the particle, the water-insoluble component being together loaded into the particle and loaded onto the surface of the particle, the water-soluble component being loaded into the particle but the water-insoluble component being loaded onto the surface of the particle, the water-insoluble component being loaded into the particle and the water-soluble component being loaded onto the surface of the particle, the water-soluble component and the water-insoluble component being loaded into the particle but only the water-insoluble component being loaded onto the surface of the particle, the water-soluble component and the water-insoluble component being loaded into the particle but only the water-soluble component being loaded onto the surface of the particle, the water-soluble component being loaded into the particle but the water-soluble component and the water-insoluble component being together loaded onto the surface of the particle, the water-insoluble component being loaded into the particle but the water-soluble component and the water-insoluble component being together loaded onto the surface of the particle, and the water-soluble component and the water-insoluble component being together loaded into the particle and the water-soluble component and the water-insoluble component being together loaded onto the surface of the particle.

47. The method of any one of claims 1-46, wherein the interior and/or the surface of the nanoparticle or the microparticle for activating the cancer-specific T cell may further comprise an immune-enhancing adjuvant, and the immune-enhancing adjuvant comprises but is not limited to at least one of a microorganism-derived immune enhancer, a product of the human or animal immune system, an intrinsic immune agonist, an adaptive immune agonist, a chemically synthesized drug, fungal polysaccharides, traditional Chinese medicine, and others; and the immune-enhancing adjuvant comprises but is not limited to at least one of the active ingredients of a pattern recognition receptor agonist, Bacillus Calmette-Guerin (BCG), a manganese-related adjuvant, a BCG cell wall backbone, a BCG methanol extract residue, BCG muramyl dipeptide, mycobacterium phlei, polyactin, mineral oil, a virus-like particle, an immune-enhanced reconstituted influenza virosome, a cholera enterotoxin, a saponin and a derivative thereof, resiquimod, thymosin, a neonatal bovine liver active peptide, miquimod, a polysaccharide, curcumin, immune adjuvant CpG, immune adjuvant poly(I:C), immune adjuvant poly ICLC, corynebacterium pumilus vaccine, a hemolytic streptococcus preparation, coenzyme Q10, levamisole, polycytidylic acid, a manganese adjuvant, an aluminum adjuvant, a calcium adjuvant, various cytokines, an interleukin, an interferon, polyinosinic acid, polyadenylic acid, alum, aluminum phosphate, lanolin, squalene, a cytokine, vegetable oil, endotoxin, a liposome adjuvant, MF59, double-stranded RNA, double-stranded DNA, an aluminum-related adjuvant, CAF01, ginseng, and astragalus membranaceus. It may be understood by those skilled in the art that the enumerated herein is not exhaustive, and other substances that may enhance the immune response may also be adopted as the immune-enhancing adjuvant.

48. The method of any one of claims 1-47, wherein the immune-enhancing adjuvant is preferably a Toll-like receptor agonist.

49. The method of any one of claims 1-48, wherein the immune-enhancing adjuvant is preferably two or more Toll-like receptor agonists used in combination.

50. The method of any one of claims 1-49, wherein the immune adjuvant is co-loaded with the cell component in the nanoparticle or the microparticle, and may better the cancer-specific T cell after the nanoparticle or the microparticle is phagocytosed by the antigen-presenting cell.

51. The method of any one of claims 1-50, wherein the microparticle or the nanoparticle encapsulate a substance that increases escape of the nanoparticle and/or the microparticle or loaded antigens thereof from a lysosome to cytoplasm; the substance that increases lysosome escape comprises an amino acid, a peptide, saccharides, lipids, and an inorganic salt that may generate a proton sponge effect, preferably, the amino acid in the substance that increases lysosome escape comprise a positively charged amino acid, and preferably, the peptide species that increases lysosome escape comprises a positively charged amino acid.

52. The method of any one of claims 1-51, wherein the surface of the nanoparticle or the microparticle may not be linked to a target head having an active targeting function, or may be linked to a target head having an active targeting function, and
preferably, the active targeting target head may be a commonly used target head such as mannose, a CD19 antibody, a CD20 antibody, a BCMA antibody, a CD32 antibody, a CD11c antibody, a CD103 antibody, and a CD44 antibody, and leads the particle system to the antigen-presenting cell *via* targeting delivery.

53. The method of any one of claims 1-52, wherein the surface of the nanoparticle and/or the microparticle is linked with a target head actively targeting the antigen-presenting cell.

54. The method of any one of claims 1-53, wherein the nanoparticle or the microparticle may not be modified during a preparation process, or an appropriate modification technique may be adopted to increase an antigen-loading capacity of the nanoparticle or the microparticle. The modification technique comprises but is not limited to biomineralization (e.g., silicification, calcification or magnesiation), gelation, crosslinking, chemical modification, and addition of charged species.

55. The method of any one of claims 1-54, wherein the form in which the cell component or the mixture thereof is loaded into the interior of the nanoparticle or the microparticle may be any way in which the cell component or the mixture thereof may be loaded into the interior of the nanoparticle or the microparticle.

56. The method of any one of claims 1-55, wherein the way in which the cell component or the mixture thereof is loaded onto the surface of the nanoparticle or the microparticle comprises but is not limited to adsorption, covalent linkage, charge interaction (e.g., addition of a positively charged species and addition of a negatively charged species), hydrophobic interaction, one-step or multi-step solidification, mineralization, encapsulation, etc.

57. The method of any one of claims 1-56, wherein the water-soluble component and/or the water-insoluble component loaded onto the surface of the nanoparticle or the microparticle is one or more layers after being loaded, and when the vaccine surface is loaded with multiple layers of the water-soluble component and/or the water-insoluble component, there is a modifier between the layers.

58. The method of any one of claims 1-57, wherein the nanoparticle has a particle size of 1 nm to 1000 nm, more preferably a particle size of 30 nm to 1000 nm, and most preferably a particle size of 100 nm to 600 nm.

59. The method of any one of claims 1-58, wherein the microparticle has a particle size of 1 µm to 1000 µm, more preferably a particle size of 1 µm to 100 µm, more preferably a particle size of 1 µm to 10 µm, and most preferably a particle size of 1 µm to 5 µm.

60. The method of any one of claims 1-59, wherein a shape of the nanoparticle or the microparticle comprises any one of a sphere, an ellipsoidal shape, a barrel shape, a polygon, a rod shape, a sheet shape, a linear shape, a worm shape, a square, a triangle, a butterfly shape, or a disc shape.

61. The method of any one of claims 1-60, wherein the way in which the water-soluble component and/or the water-insoluble component is loaded onto the surface of the cancer vaccine comprises at least one of adsorption, covalent linkage, charge interaction, hydrophobic interaction, one-step or multi-step solidification, mineralization, and encapsulation.

62. The method of any one of claims 1 to 61, wherein the preparation material of the nanovaccine and/or the microvaccine is the organic synthetic polymer material, the natural polymer material or the inorganic material.

63. The method of any one of claims 1-62, wherein the organic synthetic polymeric material is a biocompatible or degradable polymeric material, comprising any one of PLGA, PLA, PGA, PLGA-PEG, PLA-PEG, PGA-PEG, PEG, PCL, Poloxamer, PVA, PVP, PEI, PTMC, a polyanhydride, PDON, PPDO, PMMA, a polyamino acid, a synthetic peptide, and a synthetic lipid, or a combination thereof.

64. The method of any one of claims 1-63, wherein the natural polymer material is a biocompatible or degradable polymer material, comprising any one of lecithin, cholesterol, sodium alginate, albumin, collagen, gelatin, a cell membrane component, starch, saccharides, and a peptide, or a combination thereof.

65. The method of any one of claims 1-64, wherein the inorganic material is a material without significant biological toxicity, comprising but not limited to ferric oxide, ferroferric oxide, calcium carbonate, calcium phosphate, etc.

66. The method for preparing a cancer-specific T cell derived from an autologous or allogeneic source for preventing or treating cancer of claim 1, specifically comprising steps of:
(1) isolating a mononuclear cell PBMC from peripheral blood or the immune cell of the peripheral immune organ; preferably, the PBMC is subjected to a first step of sorting to obtain at least one of the following effector T cells: a CD3⁺ CD8⁺ T cell, a CD19⁺ B cell, a CD3⁺ T cell, a CD8⁺ T cell, a CD4⁺ T cell, a B220⁺ B cell, a CD69⁻ PBMC cell, a CD25⁻ PBMC cell, a CD3⁺ CD69⁺ T cell, and a CD11c⁺ DC cell;
(2) preparing the nanoparticle and/or the microparticle loaded with a tumor antigen component,
wherein preferably, the tumor antigen component is prepared by first isolating a tumor cell or tissue, and lysing the tumor cell or the tissue to obtain any one of a water-soluble component, a water-insoluble component and a whole component, or a combination thereof; and
preferably, the nanoparticle and/or the microparticle loaded with the tumor antigen component is prepared by a double emulsion method;
(3) adding the nanoparticle and/or the microparticle loaded with the tumor antigen component into a medium, co-incubating with the PBMC cell or the T cell in the step (1) to obtain a cell culture, and further sorting a specific T cell from the cell culture, wherein the specific T cell comprises but is not limited to any one of T cells such as CD3⁺ CD69⁺, CD3⁺ CD8⁺ CD69⁺, CD3⁺ CD4⁺ CD69+, CD3⁺ CD137⁺, CD3⁺ CD4⁺ CD137⁺, CD3⁺ CD8⁺ CD137⁺, CD3⁺ CD25⁺, CD3⁺ CD8⁺ CD25⁺, CD3⁺ CD4⁺ CD25⁺, CD3⁺ CD134⁺, CD3⁺ CD8⁺ CD134⁺, CD3⁺ CD4⁺ CD134⁺, CD3⁺ IL-2R⁺, CD3⁺ CD8⁺ IL-2R⁺, CD3⁺ CD4⁺ IL-2R⁺, CD3⁺ HLA-DR⁺, CD3⁺ CD8⁺ HLA-DR⁺, CD3⁺ CD4⁺ HLA-DR⁺, CD3⁺ FASL⁺ CD3⁺ CD8⁺ FASL⁺, CD3⁺ CD4⁺ FASL⁺, CD3⁺ OX40⁺, CD3⁺ CD8⁺ OX40⁺, CD3⁺ CD4⁺ OX40⁺, CD3⁺ TCF-1⁺, CD3⁺ CD8⁺ TCF-1⁺, CD3⁺ CD4⁺ TCF-1⁺, CD3⁺ PD-1⁺, CD3⁺ CD8⁺ PD-1⁺, CD3⁺ CD4⁺ PD-1⁺, CD3⁺ CD39⁺, CD3⁺ CD8⁺ CD39⁺, CD3⁺ CD4⁺ CD39⁺, CD3⁺ CD38⁺, CD3⁺ CD8⁺ CD38⁺, CD3⁺ CD4⁺ CD38⁺, CD3⁺ CD28⁺, CD3⁺ CD8⁺ CD28⁺, CD3⁺ CD4⁺ CD28⁺, CD3⁺ CD71⁺, CD3⁺ CD8⁺ CD71⁺, CD3⁺ CD4⁺ CD71⁺, CD3⁺ CD44⁺, CD3⁺ CD8⁺ CD44⁺, CD3⁺ CD4⁺ CD44⁺, CD3⁺ CXCR3⁺, CD3⁺ CD8⁺ CXCR3⁺, CD3⁺ CD4⁺ CXCR3⁺, CD3⁺ CXCR1⁺, CD3⁺ CD8⁺ CXCR1⁺, CD3⁺ CD4⁺ CXCR1⁺, CD3⁺ ICAM-1⁺, CD3⁺ CD8⁺ ICAM-1⁺, CD3⁺ CD4⁺ ICAM-1⁺, CD3⁺ CD70⁺, CD3⁺ CD8⁺ CD70⁺, CD3⁺ CD4⁺ CD70⁺, CD3⁺ CD154⁺, CD3⁺ CD8⁺ CD154⁺, CD3⁺ CD4⁺ CD154⁺, CD3⁺ CD62L⁺, CD3⁺ CD8⁺ CD62L⁺, CD3⁺ CD4⁺ CD62L⁺, CD3⁺ CD154⁺, CD3⁺ CD8⁺ CD154⁺, CD3⁺ CD4⁺ CD154⁺, CD3⁺ CD160⁺, CD3⁺CD8⁺ CD160⁺, CD3⁺ CD4⁺ CD160⁺, CD3⁺ CD160⁺, CD3⁺ CD8⁺ CD160⁺, CD3⁺ CD4⁺ CD160⁺, CD3⁺ ICOS⁺, CD3⁺ CD8⁺ ICOS ⁺, CD3⁺ CD4⁺ ICOS⁺, CD3⁺ CD27⁺, CD3⁺ CD8⁺ CD27⁺, CD3⁺ CD4⁺ CD27⁺, CD3⁺ CD107A⁺, CD3⁺ CD8⁺ CD107A⁺, CD3⁺ CD4⁺ CD107A⁺, etc., or a combination thereof, wherein
preferably, in the co-incubation process, 0.05million/mL-50 million/mL of antigen-presenting cells are further added, and the antigen-presenting cells are any one of B cells, DC cells and macrophages, or a combination thereof;
preferably, the nanoparticle and/or the microparticle loaded with the tumor antigen component may be co-incubated together with the antigen-presenting cell and the T cell to activate the cancer-specific T cell; or the nanoparticle and/or the microparticle loaded with the tumor antigen component may be first co-incubated with the antigen-presenting cell to activate the antigen-presenting cell, and then the activated antigen-presenting cell is alone co-incubated with the T cell to activate the cancer -specific T cell; and after the nanoparticle and/or the microparticle loaded with the tumor antigen component is first co-incubated with the antigen-presenting cell to activate the antigen-presenting cell, the antigen-presenting cell may be co-incubated with the T cell to activate the specific T cell without special treatment, or the antigen-presenting cell may be treated with fixation, radiation, irradiation, modification, inactivation, mineralization, etc., and then co-incubated with the T cell to activate the specific T cell;
preferably, 2.5 ng/mL-50 mg/mL of the nanoparticle and/or the microparticle loaded with the tumor antigen component is added into the medium, and incubated with 0.01million/mL-50 million/mL of the PBMC cells or the sorted cells for 4 h to 96 h under conditions of 30-38°C and 1-5% CO2 to obtain a cell culture;
preferably, in the co-incubation process, 10 ng/mL to 500 ng/mL of an interleukin is further added, and the interleukin is any one of IL-2, IL-7, IL-12, IL-15, IL-17, and IL-21, or a combination thereof;
the medium is any one of DMEM high glucose complete medium, RPM 1640 medium and AIMV serum-free medium;
the co-incubation is performed under conditions of 30-38°C for 1 h to 168 h, preferably 4 h to 96 h, and more preferably 6 h to 72 h; and
viability of the specific T cell is greater than 60%, preferably greater than 70%, and more preferably greater than 80%; and
(4) expanding the specific T cell obtained in the step (3).

67. The method of claim 66, wherein steps of the expanding is adding 0.1million cells/mL-0.5 million cells/mL of the specific T cell sorted in the step (2) to an expansion medium, co-incubating under conditions of 30-38°C and 1-5% CO2, changing the medium using an expansion medium every 2 days to 3 days, and co-incubating for 4 days to 72 days to obtain an expanded specific T cell.

68. The method of any one of claims 66-67, wherein preferably, the expansion medium is performed in the expansion medium, and the expansion medium is any one of DMEM high glucose complete medium and RPM 1640 medium;
preferably, a condition of expansion culture is culturing for 5 days to 30 days at 30-38°C;
preferably, the expansion medium further comprises 200 U/mL to 1000 U/mL of an interleukin, 10 ng/mL to 200 ng/mL of an antibody and/or 1 ng/mL to 100 ng/mL of a granulocyte-macrophage colony-stimulating factor (GM-CSF);
preferably, the interleukin is any one of IL-2, IL-7, IL-12, IL-15, IL-17, TNF-α, and IL-21, or a combination thereof; and
preferably, the antibody is any one of an αCD3 antibody and an αCD28 antibody, or a combination thereof.

69. The method of any one of claims 66-68, wherein in the step (1), the cell is selected from any part of an individual suffering from a neoplastic disease, and preferably a splenocyte and a lymphocyte.

70. The method of any one of claims 66-69, wherein viability of the expanded specific T cell is greater than 60%, preferably greater than 75%, and more preferably greater than 80%.

71. The method of any one of claims 66-70, wherein in the step (2), the method of the sorting is isolating a cell expressing the specific cell marker from a cell population by flow cytometry or the magnetic bead method, etc. after binding an antibody with fluorescence or magnetism or a specific ligand to the specific cell marker on a surface of the T cell.

72. The method of any one of claims 66-71, wherein a cytokine may be added in the mixing and co-incubating; the cytokine added comprises but is not limited to an interleukin, a tumor necrosis factor, an interferon, and a growth factor; and preferably, the cytokine added comprises interleukin 7 (IL-7) and interleukin 15 (IL-15).

73. The method of any one of claims 66-72, wherein in the step (3), a concentration of the cytokine is 1 ng/mL to 6000 ng/mL, preferably 5 ng/mL to 100 ng/mL, and more preferably 10 ng/mL to 30 ng/mL.

74. The method of any one of claims 66-73, wherein in the step (3), the cytokine comprises but is not limited to an interleukin, an interferon and a tumor necrosis factor.

75. The method of any one of claims 66-74, wherein the interleukin comprises but is not limited to interleukin 2 (IL-2), interleukin 7 (IL-7), interleukin 12 (IL-12), interleukin 15 (IL-15), interleukin 17 (IL-17), and interleukin 21 (IL-21).

76. The method of any one of claims 66-75, wherein a concentration of the antibody is 1 ng/mL to 6000 ng/mL, preferably 5 ng/mL to 100 ng/mL, and more preferably 10 ng/mL to 30 ng/mL.

77. The method of any one of claims 66-76, wherein the antibody comprises but is not limited to any one of an αCD3 antibody, an αCD28 antibody, an αCD80 antibody, an αCD86 antibody, and an αOX40 antibody, or a combination thereof.

78. The method of any one of claims 66-77, wherein time of the co-incubation of the nanoparticle and/or the microparticle with a mixture of the antigen-presenting cell and the T cell is at least 1 hour, and preferably 6 hours to 96 hours.

79. The method of any one of claims 66-78, wherein time of the co-incubation of the nanoparticle and/or the microparticle with the antigen-presenting cell alone is at least 1 hour, and preferably 6 hours to 96 hours.

80. The method of any one of claims 66-79, wherein time of the co-incubation of a mixture of the activated antigen-presenting cell and the T cell is at least 1 hour, and preferably 6 hours to 96 hours.

81. The method of any one of claims 66-80, wherein time of the expansion culture is at least 1 day, and preferably 4 days to 36 days.

82. The method of any one of claims 66-81, wherein a concentration of the nanoparticle and/or the microparticle is 2.5 ng/mL to 50 mg/mL when the nanoparticle and/or the microparticle is co-incubated with the antigen-presenting cell alone or when the nanoparticle and/or the microparticle is co-incubated with the antigen-presenting cell and the T cell.

83. The method of any one of claims 66-82, wherein a content of the protein and peptide components in the antigen component loaded by the nanoparticle and/or the microparticle is higher than 10 ng/mL.

84. The method of any one of claims 66- 83, wherein when the tumor antigen component is a whole-cell component, a preparation method thereof is: (1) first, lysing the cancer cell/the tumor tissue; then, preparing a water-soluble component and a water-insoluble component separately; and then, solubilizing the water-insoluble component using a specific solubilizing agent containing the solubilizing solution for use; or (2) lysing the cell using the solubilizing solution containing the solubilizing agent, and then solubilizing a lysed whole-cell component using the solubilizing solution containing the solubilizing agent.

85. The method of any one of claims 66-84, wherein when the tumor antigen component is a portion of a component of the whole-cell component, a preparation method thereof is:
(1) first, preparing a lysate of the tumor tissue/the cancer cell; then, preparing the water-soluble component and the water-insoluble component separately; then, solubilizing the water-insoluble component using the specific solubilizing agent containing the solubilizing solution for use; then, isolating and extracting the protein and peptide components in the water-soluble component from the water-soluble component using an appropriate method; and then, using the protein and peptide components isolated and extracted from the water-soluble component together with all the water-insoluble component as the antigen component;
(2) first, preparing a lysate of the tumor tissue/the cancer cell; then, preparing the water-soluble component and the water-insoluble component separately; then, solubilizing the water-insoluble component using the specific solubilizing agent containing the solubilizing solution for use; then, isolating and extracting the protein and peptide components in the water-soluble component from the water-insoluble component using an appropriate method; and then, using the protein and peptide components isolated and extracted from the water-insoluble component together with all the water-soluble component as the antigen component;
(3) first, preparing a lysate of the tumor tissue/the cancer cell; then, preparing the water-soluble component and the water-insoluble component separately; then, solubilizing the water-insoluble component using the specific solubilizing agent containing the solubilizing solution; then, isolating and extracting the protein and peptide components in the water-soluble component from the water-soluble component and the water-insoluble component using an appropriate method, respectively; and then, using the protein and peptide components isolated and extracted from the water-soluble component and the water-insoluble component together as the antigen component;
or (4) lysing the cell or the tissue directly and solubilizing the whole-cell component directly by using the solubilizing solution containing the solubilizing agent; then, preparing the protein/peptide component therein by an appropriate method; and, using the protein/peptide component as the antigen component.
preferably, the appropriate method comprises but is not limited to treatment methods such as salting-out, heating and enzymatic hydrolysis; and the water-insoluble component or a precipitate generated after treatments such as salting-out, heating and enzymatic hydrolysis is solubilized using the solubilizing solution containing the solubilizing agent; and
preferably, in the above preparation method, a step of isolating and extracting whole-cell mRNA may be added, and the whole-cell mRNA may be used as a portion of the antigen component.

86. The method of any one of claims 66-85, wherein in the step (2), the tumor antigen component is prepared by first isolating a tumor cell or tissue, and lysing the tumor cell or the tissue to obtain any one of a water-soluble component, a water-insoluble component and a whole component, or a combination thereof.

87. The method of any one of claims 66-86, wherein in the step (2), the tumor antigen component is treated by salting-out, heating and enzymatic hydrolysis.

88. The method of any one of claims 66-87, wherein both the water-soluble portion and the water-insoluble portion may be solubilized by an aqueous solubilizing solution containing the solubilizing agent or the organic solvent.

89. The method of any one of claims 66-88, wherein the dissolving agent/the solubilizing agent is at least one of dissolving agents/solubilizing agents that may increase solubility of the protein or the peptide in the aqueous solution; and the organic solvent is an organic solvent that may dissolve the protein or the peptide.

90. The method of any one of claims 66-89, wherein the water-insoluble portion is changed from insoluble in pure water to soluble in the aqueous solution containing the solubilizing agent or in the organic solvent by an appropriate solubilizing method; and the solubilizing agent adopted is selected from one or more of a compound with a structure of structural formula 1, a deoxycholate, a lauryl sulfate, glycerol, a protein-degrading enzyme, albumin, lecithin, a peptide, an amino acid, a glycoside, and a choline,
wherein structural formula 1 is as follows: the structure of structural formula 1 is as follows: 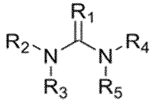
R₁ is C, N, S or O, and R₂-R₅ are independently selected from at least one of hydrogen, alkyl, amino, carboxyl, and substituted or unsubstituted guanidino.
The compound with the structural formula 1 comprises but is not limited to metformin hydrochloride, metformin sulfate, metformin sulfonate, a metformin salt, metformin, urea, guanidine hydrochloride, guanidine sulfate, guanidine sulfonate, a guanidine salt, other compounds containing guanidino, guanidine carbonate, arginine, guanidinoacetic acid, guanidinophosphoric acid, guanidine sulfamate, guanidinosuccinic acid, semicarbazide hydrochloride, carbamoylurea, acetylurea, a sulfonylurea compound (glibenclamide, gliclazide, gliquidone, glimepiride, etc.), a thiourea compound (thiouracil, imidazole, etc.), nitrosoureas, etc.

91. The method of any one of claims 66-90, wherein the immune adjuvant is co-loaded with the cell component in the nanoparticle or the microparticle, and may better the cancer-specific T cell after the nanoparticle or the microparticle is phagocytosed by the antigen-presenting cell.

92. The method of any one of claims 66-91, wherein a method for preparing the water-soluble component is: cutting the tumor tissue or the cancer cell into pieces and then grinding, filtering to obtain a single-cell suspension, adding water to freeze and thaw repeatedly for 1-5 times, ultrasonically lysing, centrifuging a lysate at a rotational speed of 5000-10000 g for 5-10 minutes, and taking a supernatant as the water-soluble component and a precipitated portion as the water-insoluble component.

93. The method of any one of claims 66-92, wherein the antigen component is a soluble component obtained after solubilizing the water-insoluble component by adding the solubilizing agent, and the solubilizing agent is one or more of a compound with the structure of structural formula 1, a deoxycholate, a lauryl sulfate, glycerol, a protein-degrading enzyme, albumin, lecithin, a peptide, an amino acid, a glycoside, and a choline,
wherein structural formula 1 is as follows: the structure of structural formula 1 is as follows: 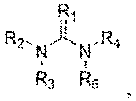
R₁ is C, N, S or O, and R₂-R₅ are independently selected from at least one of hydrogen, alkyl, amino, carboxyl, and substituted or unsubstituted guanidino. The compound with the structural formula 1 comprises but is not limited to metformin hydrochloride, metformin sulfate, metformin sulfonate, a metformin salt, metformin, urea, guanidine hydrochloride, guanidine sulfate, guanidine sulfonate, a guanidine salt, other compounds containing guanidino, guanidine carbonate, arginine, guanidinoacetic acid, guanidinophosphoric acid, guanidine sulfamate, guanidinosuccinic acid, semicarbazide hydrochloride, carbamoylurea, acetylurea, a sulfonylurea compound (glibenclamide, gliclazide, gliquidone, glimepiride, etc.), a thiourea compound (thiouracil, imidazole, etc.), and nitrosoureas.

94. The method of any one of claims 66-93, wherein the tumor tissue or the cancer cell is subjected to any one of inactivation and denaturation treatment by ultraviolet high-temperature heating and inactivation treatment by adding nuclease, or a combination thereof in advance.

95. The method of any one of claims 66-94, wherein the antigen component is a component obtained after the water-soluble component is subjected to salting-out and precipitation by heating.

96. The method of any one of claims 66-95, wherein the antigen component is a soluble component obtained by adding the solubilizing agent into the precipitated portion after lysis and centrifugation of the tumor tissue for dissolution to obtain a precipitate, and then adding a solubilizing agent to the precipitate for secondary dissolution; and
the solubilizing agent is any one of Tween 80 and guanidine sulfate, or a combination thereof.

97. A specific T cell prepared by the method for preparing a cancer-specific T cell for preventing or treating cancer of claims 1-96.

98. A cancer-specific T cell derived from an autologous or allogeneic source for preventing or treating cancer, the specific T cell comprising but not limited to any one of T cells such as CD3⁺ CD69⁺, CD3⁺ CD8⁺ CD69⁺, CD3⁺ CD4⁺ CD69+, CD3⁺ CD137⁺, CD3⁺ CD4⁺ CD137⁺, CD3⁺ CD8⁺ CD137⁺, CD3⁺ CD25⁺, CD3⁺ CD8⁺ CD25⁺, CD3⁺ CD4⁺ CD25⁺, CD3⁺ CD134⁺, CD3⁺ CD8⁺ CD134⁺, CD3⁺ CD4⁺ CD134⁺, CD3⁺ IL-2R⁺, CD3⁺ CD8⁺ IL-2R⁺, CD3⁺ CD4⁺ IL-2R⁺, CD3⁺ HLA-DR⁺, CD3⁺ CD8⁺ HLA-DR⁺, CD3⁺ CD4⁺ HLA-DR⁺, CD3⁺ FASL⁺ CD3⁺ CD8⁺ FASL⁺, CD3⁺ CD4⁺ FASL⁺, CD3⁺ OX40⁺, CD3⁺ CD8⁺ OX40⁺, CD3⁺ CD4⁺ OX40⁺, CD3⁺ TCF-1⁺, CD3⁺ CD8⁺ TCF-1⁺, CD3⁺ CD4⁺ TCF-1⁺, CD3⁺ PD-1⁺, CD3⁺ CD8⁺ PD-1⁺, CD3⁺ CD4⁺ PD-1⁺, CD3⁺ CD39⁺, CD3⁺ CD8⁺ CD39⁺, CD3⁺ CD4⁺ CD39⁺, CD3⁺ CD38⁺, CD3⁺ CD8⁺ CD38⁺, CD3⁺ CD4⁺ CD38⁺, CD3⁺ CD28⁺, CD3⁺ CD8⁺ CD28⁺, CD3⁺ CD4⁺ CD28⁺, CD3⁺ CD71⁺, CD3⁺ CD8⁺ CD71⁺, CD3⁺ CD4⁺ CD71⁺, CD3⁺ CD44⁺, CD3⁺ CD8⁺ CD44⁺, CD3⁺ CD4⁺ CD44⁺, CD3⁺ CXCR3⁺, CD3⁺ CD8⁺ CXCR3⁺, CD3⁺ CD4⁺ CXCR3⁺, CD3⁺ CXCR1⁺, CD3⁺ CD8⁺ CXCR1⁺, CD3⁺ CD4⁺ CXCR1⁺, CD3⁺ ICAM-1⁺, CD3⁺ CD8⁺ ICAM-1⁺, CD3⁺ CD4⁺ ICAM-1⁺, CD3⁺ CD70⁺, CD3⁺ CD8⁺ CD70⁺, CD3⁺ CD4⁺ CD70⁺, CD3⁺ CD154⁺, CD3⁺ CD8⁺ CD154⁺, CD3⁺ CD4⁺ CD154⁺, CD3⁺ CD62L⁺, CD3⁺ CD8⁺ CD62L⁺, CD3⁺ CD4⁺ CD62L⁺, CD3⁺ CD154⁺, CD3⁺ CD8⁺ CD154⁺, CD3⁺ CD4⁺ CD154⁺, CD3⁺ CD160⁺, CD3⁺ CD8⁺ CD160⁺, CD3⁺ CD4⁺ CD160⁺, CD3⁺ CD160⁺, CD3⁺ CD8⁺ CD160⁺, CD3⁺ CD4⁺ CD160⁺, CD3⁺ ICOS⁺, CD3⁺ CD8⁺ ICOS ⁺, CD3⁺ CD4⁺ ICOS⁺, CD3⁺ CD27⁺, CD3⁺ CD8⁺ CD27⁺, CD3⁺ CD4⁺ CD27⁺, CD3⁺ CD107A⁺, CD3⁺ CD8⁺ CD107A⁺, CD3⁺ CD4⁺ CD107A⁺, etc., or a combination thereof, wherein viability of the specific T cell is greater than 60%, preferably greater than 70%, and more preferably greater than 80%; and
preferably, the specific T cell is obtained by co-incubating a nanoparticle and/or a microparticle loaded with a tumor antigen component with a mononuclear cell (PBMC) isolated from an immune cell of peripheral blood or a peripheral immune organ.

99. The specific T cell of claim 98, wherein the PBMC cell is sorted and then co-incubated with the nanoparticle and/or the microparticle loaded with the tumor antigen component, and
preferably, the cell co-incubated with the nanoparticle and/or the microparticle loaded with the tumor antigen component is sorted again to obtain the specific T cell.
Preferably, the specific T cell may also be subjected to a step of *in vitro* expansion, the step may be performed before the above sorting step or after the above sorting step, and preferably after the sorting step.
Preferably, when the PBMC cell is co-incubated with the nanoparticle and/or the microparticle, an antigen-presenting cell (APC) is also present, and
preferably, the various types of T cells may be used alone or in combination according to a need of a patient.

100. A method for preparing a pharmaceutical composition comprising the specific T cell of any one of claims 97-99, comprising a step of adding a substance enhancing an innate immune system, such as albumin, an NK cell, a neutrophil, a γδ T cell, and an NK T cell, to the cancer-specific T cell before reinfusing the cancer-specific T cell into the patient, wherein
preferably, a cell concentration of the specific T cell is (0.01-100) × 10⁷ cells/mL, and preferably (0.1-8) × 10⁷ cells/mL, and
preferably, the pharmaceutical composition further comprises any one of hydroxyethyl starch, sugar and salt, or a combination thereof.

101. The method for preparing a pharmaceutical composition of claim 100, comprising a step of adding the substance enhancing the innate immune system, such as albumin, an NK cell, a neutrophil, a γδ T cell, and an NK T cell, to the cancer-specific T cell before reinfusing the cancer-specific T cell into the patient, wherein
preferably, the cell concentration of the specific T cell is (0.01-100) × 10⁷ cells/mL, and preferably (0.1-8) × 10⁷ cells/mL, and
preferably, the pharmaceutical composition further comprises any one of hydroxyethyl starch, sugar and salt, or a combination thereof.

102. An application of the specific T cell of any one of claims 97-99 in preparation of a medicament for treating or preventing cancer.

103. The application of claim 102, wherein the specific T cell is administered multiple times before cancer occurrence, after cancer occurrence or after surgical excision of tumor tissue.

104. An application of the specific T cell of any one of claims 97-99 in preparation of a medicament for preventing cancer recurrence or preventing cancer metastasis.

105. An application of the specific T cell of any one of claims 97-99 in preparation of a product for immunotherapy against a tumor.

106. The application of claim 105, wherein the tumor is selected from a solid tumor, a hematoma and a lymphoma. The tumor comprises but is not limited to any one of lung cancer, ovarian cancer, colon cancer, rectal cancer, melanoma, kidney cancer, bladder cancer, breast cancer, liver cancer, lymphoma, a malignant hematoma such as leukemia, brain tumor, head and neck cancer, glioma, gastric cancer, nasopharyngeal cancer, laryngeal cancer, cervical cancer, a uterine body tumor, osteosarcoma, bone cancer, pancreatic cancer, skin cancer, prostate cancer, uterine cancer, anal region cancer, testicular cancer, oviduct cancer, endometrial cancer, vaginal cancer, vulval cancer, Hodgkin's disease, non-Hodgkin's lymphoma, esophageal cancer, small intestine cancer, endocrine system cancer, thyroid cancer, parathyroid cancer, adrenal cancer, soft tissue sarcoma, urethra cancer, penile cancer, chronic or acute leukemia, a pediatric solid tumor, lymphocytic lymphoma, bladder cancer, kidney or ureter cancer, renal pelvis cancer, a central nervous system (CNS) tumor, primary CNS lymphoma, tumor angiogenesis, spinal tumor, brain stem glioma, pituitary adenoma, Kaposi's sarcoma, epidermoid carcinoma, squamous cell carcinoma, T-cell lymphoma, environmentally induced cancer, metastatic cancer, and a circulating tumor cell, or a combination thereof.
Preferably, the tumor is selected from any one of melanoma, colon cancer, triple-negative breast cancer, pancreatic cancer, metastatic cancer, liver cancer, colon cancer, lymphoma, esophageal cancer, and non-small cell lung cancer, or a combination thereof.

107. The application of claim 105, wherein the immunotherapy is selected from any one of immunotherapy in anti-tumor therapy or immunotherapy after radical surgery, or a combination thereof;
preferably, the immunotherapy is selected from immunotherapy after radical surgery for primary hepatocellular carcinoma.

108. The application of any one of claims 102-107, wherein the medicament is used for an adult patient or a pediatric patient.

109. An application of the specific T cell of any one of claims 97-99 for immunotherapy.

110. The application of claim 109, wherein an administration method of the immunotherapy is any one of intravenous injection, subcutaneous injection, intratumoral injection, intraperitoneal injection, intramuscular injection, and intradermal injection, or a combination thereof.

111. An application of the specific T cell of any one of claims 97-99 in combination with any one of radiotherapy, chemotherapy, targeted therapy, surgical therapy, or immunotherapy for anti-tumor or tumor immunotherapy.

112. An application of the specific T cell of any one of claims 97-99 for preparation of a medicament for enhancing an antiviral capacity.

113. An application of the specific T cell of any one of claims 97-99 for preparation of a medicament for enhancing treatment of an autoimmune disease.

114. The application of claim 113, wherein the autoimmune disease is selected from any one of systemic lupus erythematosus, rheumatoid arthritis, scleroderma, hyperthyroidism, juvenile diabetes, primary platelet purpura, autoimmune hemolytic anemia, ulcerative colitis, and a skin disease, or a complication thereof.
